# EUROPEAN PATENT APPLICATION

(11) **EP 4 667 576 A2**
(43) Date of publication of application: **24.12.2025**
(21) Application number: 25192266.2
(22) Date of filing: 18.10.2019
(51) Int. Cl.: C12N 15/86

(54) **NUCLEIC ACID CONSTRUCTS AND METHODS OF USE**

(30) Priority: 18.10.2018 US 201862747393 P; 29.04.2019 US 201962840343 P
(62) Divisional of application: 19801449.0
(71) Applicant: Intellia Therapeutics, Inc., Cambridge, MA 02139 (US); Regeneron Pharmaceuticals, Inc., Tarrytown, NY 10591 (US)
(72) Inventor: FINN, Jonathan Douglas, Cambridge MA, 02139 (US); HUANG, Hon-Ren, Cambridge, MA, 02139 (US)
(74) Representative: Schlich

(57) **Abstract**

Described herein is a bidirectional nucleic acid construct comprising: a) a first segment comprising a first coding sequence for a polypeptide; and b) a second segment comprising a reverse complement of a second coding sequence for the polypeptide, wherein the second coding sequence adopts a different codon usage from that of the first coding sequence, wherein the bidirectional nucleic acid construct does not comprise a promoter that drives the expression of the first or second coding sequence; wherein the construct does not comprise a homology arm, and wherein the construct does not comprise a first and second rare-cutting endonuclease target site. Accordingly, vectors, lipid nanoparticles and host cells comprising the construct are described. Also described are methods for use of a construct, vector, and/or lipid nanoparticle in a method of expressing the polypeptide or therapeutic protein in a subject.

## Description

This application claims the benefit of priority from U.S. Provisional Application No. 62/747,393, filed on October 18, 2018 and U.S. Provisional Application No. 62/840,343, filed on April 29, 2019. The specifications of each of the foreigoing applications are incorporated herein by reference in their entirety.

Genome editing in gene therapy approaches arises from the idea that the exogenous introduction of the missing or otherwise compromised genetic material can correct a genetic disease. Gene therapy has long been recognized for its enormous potential in how practitioners approach and treat human diseases. Instead of relying on drugs or surgery, patients with underlying genetic factors can be treated by directly targeting the underlying cause. Furthermore, by targeting the underlying genetic cause, gene therapy can have the potential to effectively cure patients. Yet, clinical applications of existing approaches still require improvement in several aspects.

The present disclosure provides bidirectional nucleic acid constructs that allow enhanced insertion and expression of a nucleic acid sequence of interest, e.g. encoding a therapeutic agent such as a polypeptide. As described herein, the bidirectional constructs comprise at least two nucleic acid segments, wherein one segment (the first segment) comprises a coding sequence that encodes an agent of interest (the coding sequence may be referred to herein as "transgene" or a first transgene), while the other segment (the second segment) comprises a sequence wherein the complement of the sequence encodes an agent of interest, or a second transgene. In some embodiments, the constructs comprise at least two nucleic acid segments, wherein one segment (the first segment) comprises a coding sequence that encodes a polypeptide of interest, while the other segment (the second segment) comprises a sequence wherein the complement of the sequence encodes a polypeptide of interest. When used in combination with a gene editing system, the bidirectionality of the nucleic acid constructs allows the construct to be inserted in either direction (is not limited to insertion in one direction) within a target insertion site, allowing the expression of the polypeptide of interest from either a) a coding sequence of one segment, or 2) a complement of the other (second) segment, thereby enhancing insertion and expression efficiency, as exemplified herein.

### BRIEF DESCRIPTION OF THE DRAWINGS

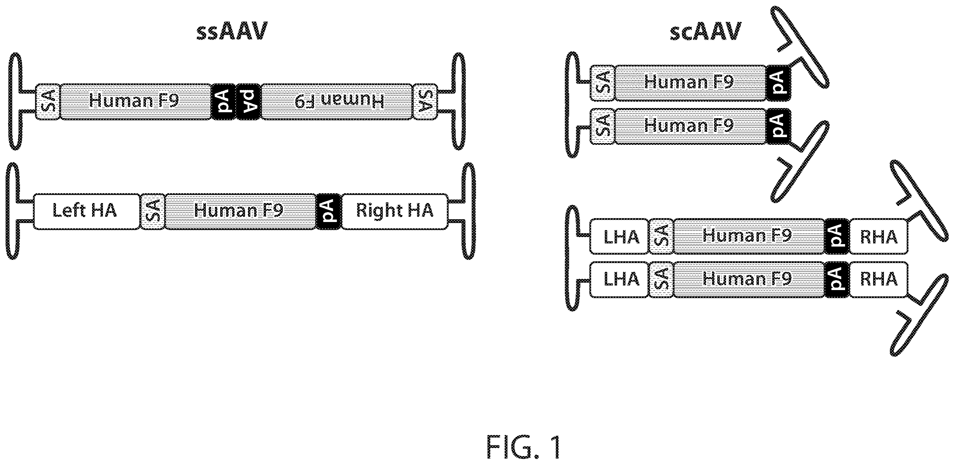

Fig. 1 shows construct formats as represented in AAV genomes. SA= splice acceptor; pA= polyA signal sequence; HA= homology arm; LHA= left homology arm; RHA= right homology arm.

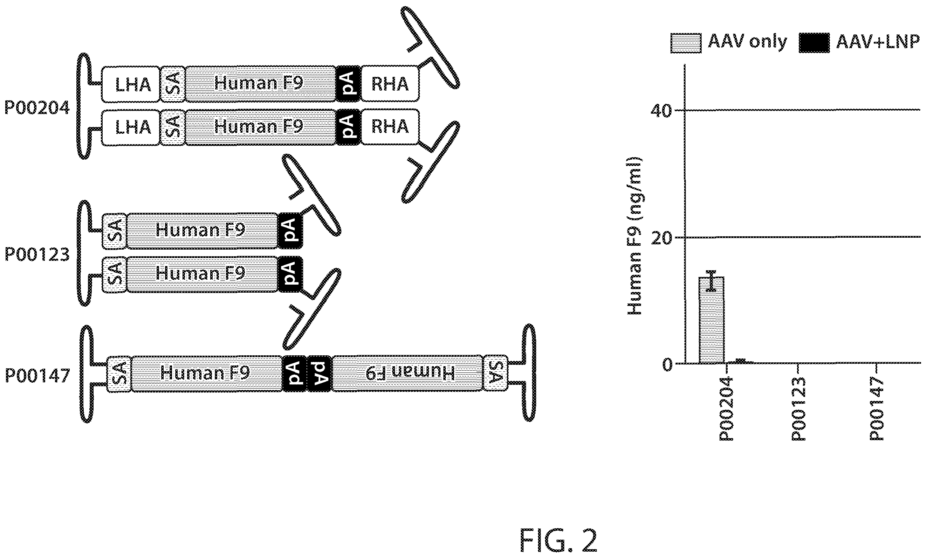

Fig. 2 shows vectors without homology arms are not effective in an immortalized liver cell line (Hepa1-6). An scAAV derived from plasmid P00204 comprising 200 bp homology arms resulted in detectable expression of hFIX in this cell line. Use of the AAV vectors derived from P00123 (scAAV lacking homology arms) and P00147 (ssAAV bidirectional construct lacking homology arms) did not result in detectable expression of hFIX.

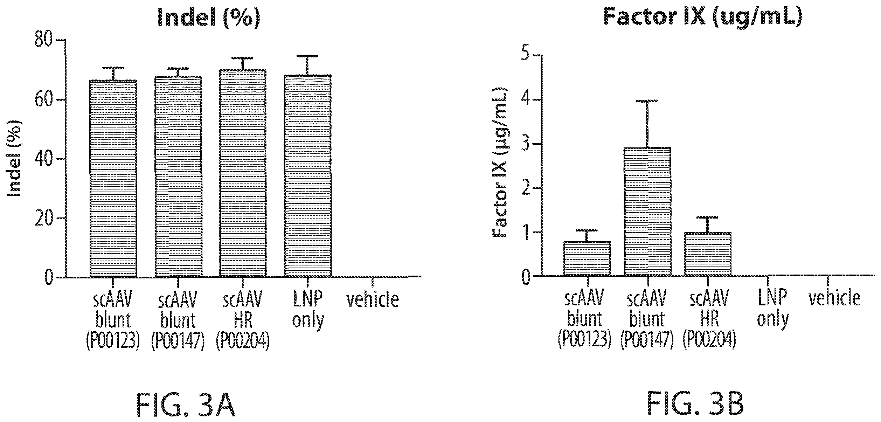

Figs. 3A and 3B show results from *in vivo* testing of insertion templates with and without homology arms using vectors derived from P00123, P00147, or P00204. Fig. 3A shows liver editing levels as measured by indel formation of ~60% were detected in each group of animals treated with LNPs comprising CRISPR/Cas9 system components. Fig. 3B shows animals receiving the ssAAV vectors without homology arms (derived from P00147) in combination with LNP treatment resulted in the highest level of hFIX expression in serum.

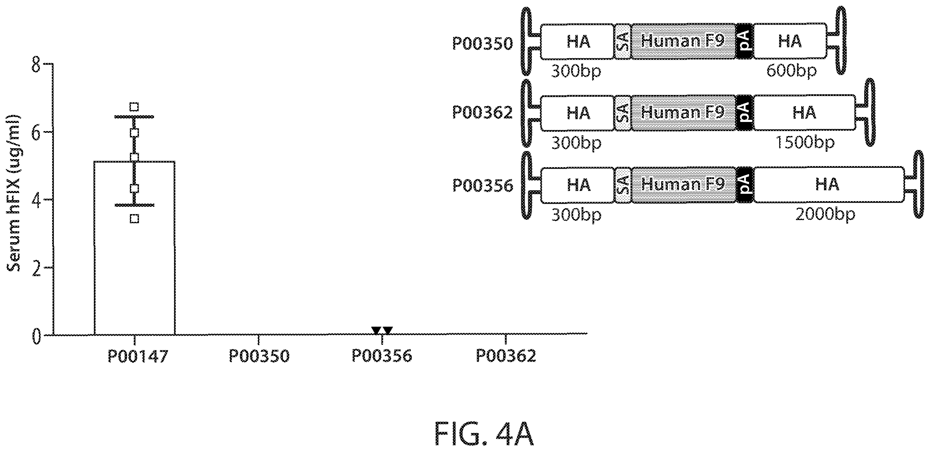

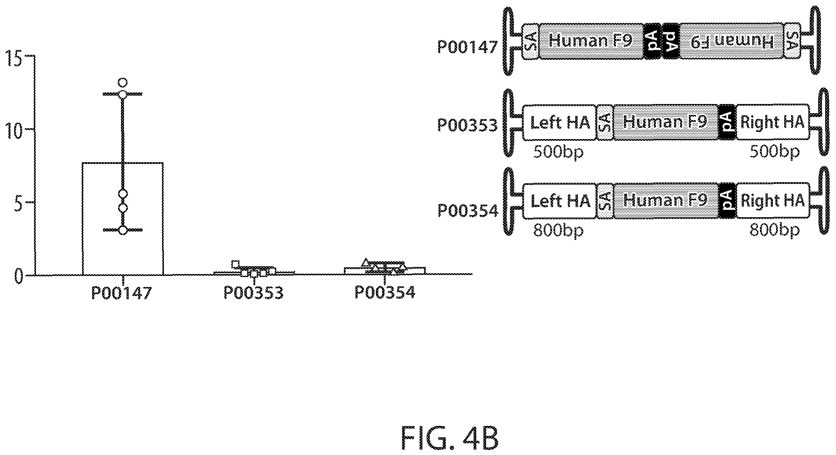

Figs. 4A and 4B show results from *in vivo* testing of ssAAV insertion templates with and without homology arms. Fig. 4A compares targeted insertion with vectors derived from plasmids P00350, P00356, P00362 (having asymmetrical homology arms as shown), and P00147 (bidirectional construct as shown in Fig. 4B). Fig. 4B compares insertion into a second site targeted with vectors derived from plasmids P00353, P00354 (having symmetrical homology arms as shown), and P00147.

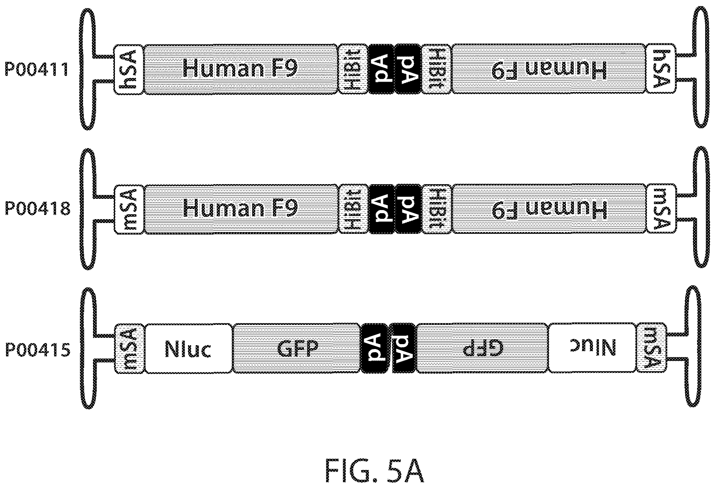

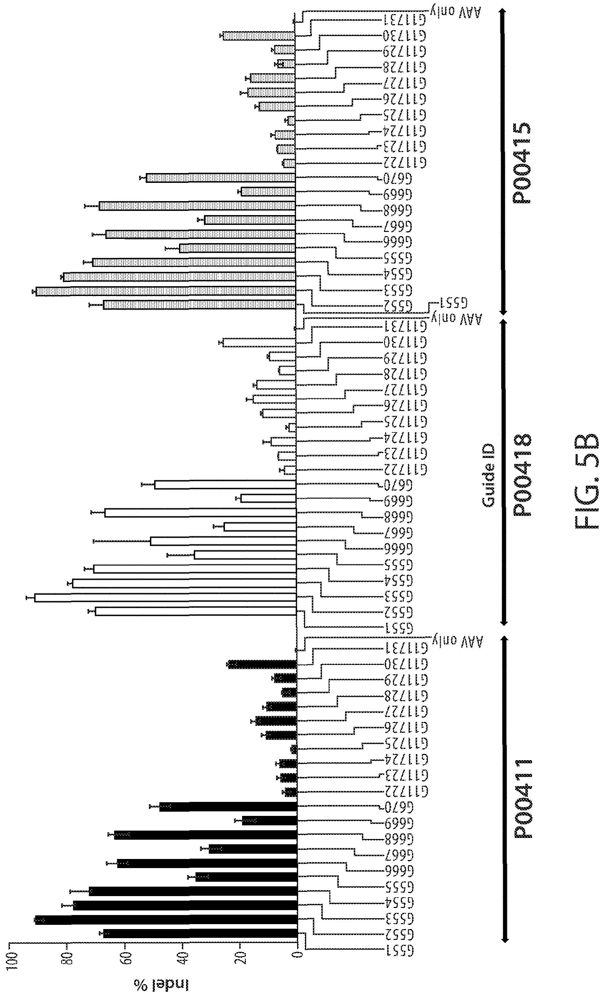

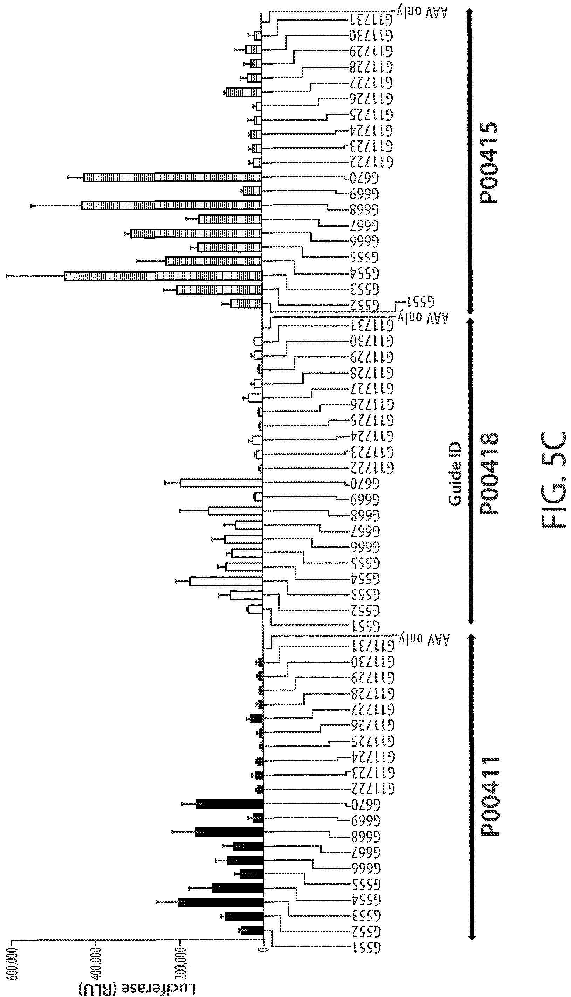

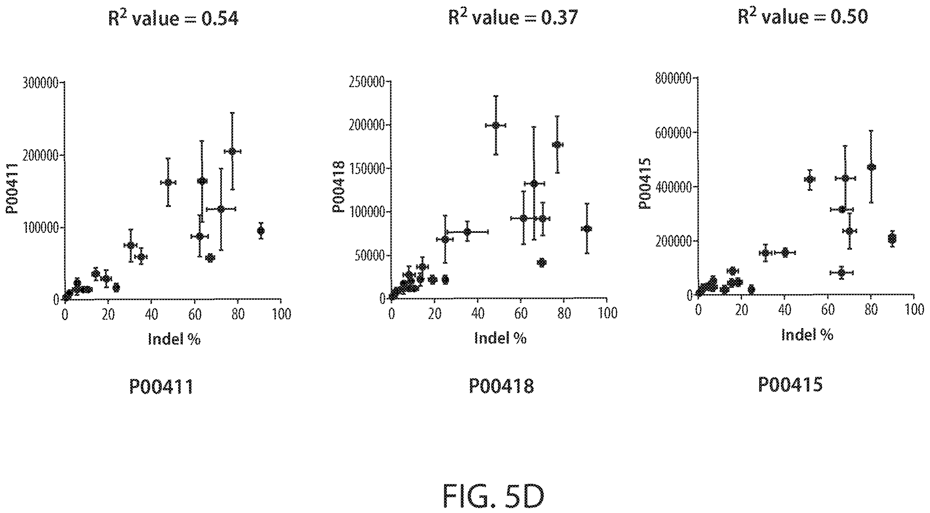

Figs. 5A-5D show results of targeted insertion by three bidirectional constructs across 20 target sites in primary mouse hepatocytes. Fig. 5A shows the schematics of each of the vectors tested. Fig. 5B shows editing as measured by indel formation for each of the treatment groups across each combination tested. Fig. 5C and Fig. 5D show that significant levels of editing (at a specific target site) did not necessarily result in more efficient insertion or expression of the transgenes. The tested constructs effectively resulted in transgene expression in this targeted insertion study. hSA= human F9 splice acceptor; mSA= mouse albumin splice acceptor; HiBit= tag for luciferase based detection; pA= polyA signal sequence; Nluc= nanoluciferase reporter; GFP= green fluorescent reporter.

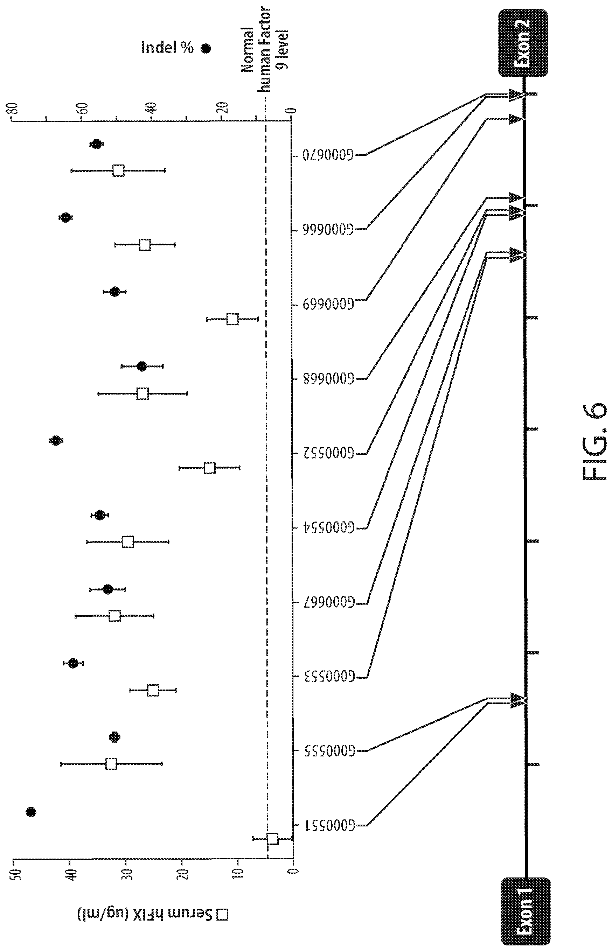

Fig. 6 shows results from *in vivo* screening of targeted insertion with bidirectional constructs across 10 target sites using with ssAAV derived from P00147. As shown, significant levels of editing do not necessarily result in high levels of transgene expression.

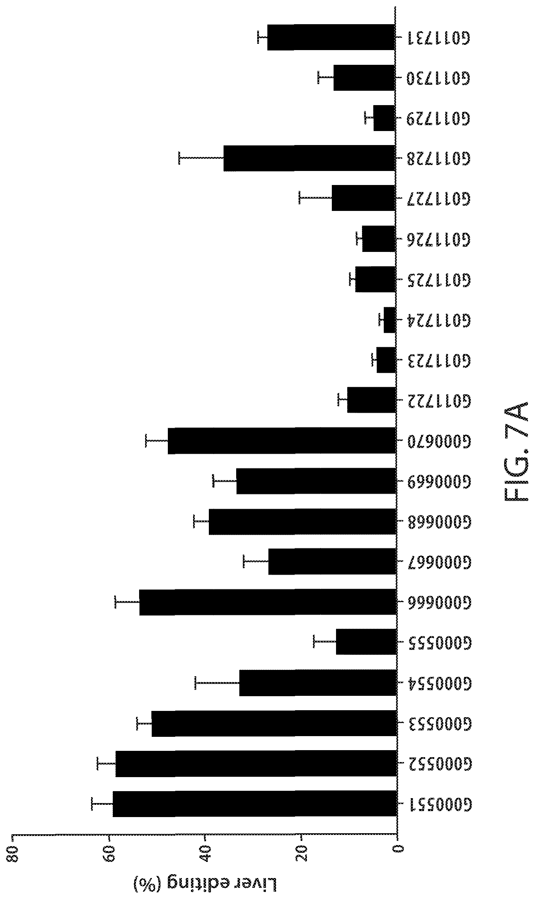

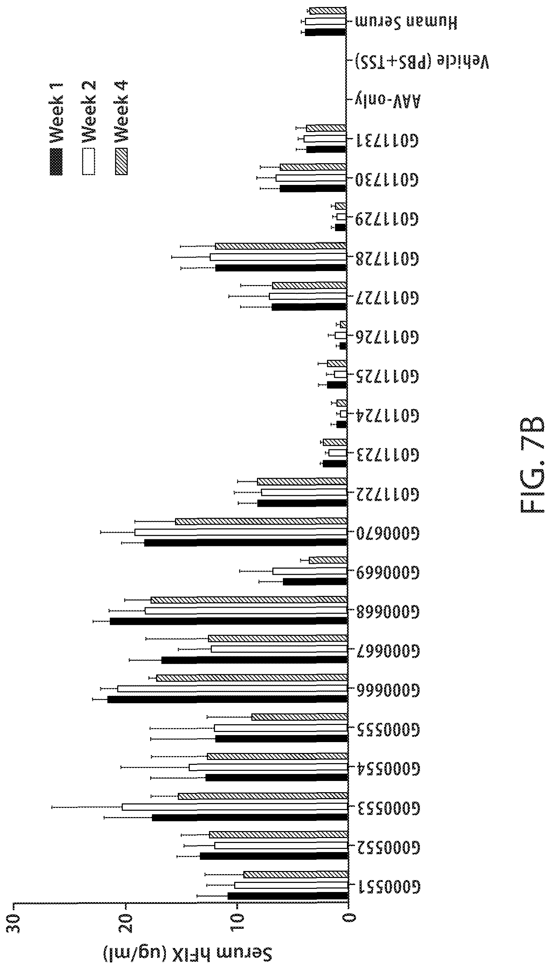

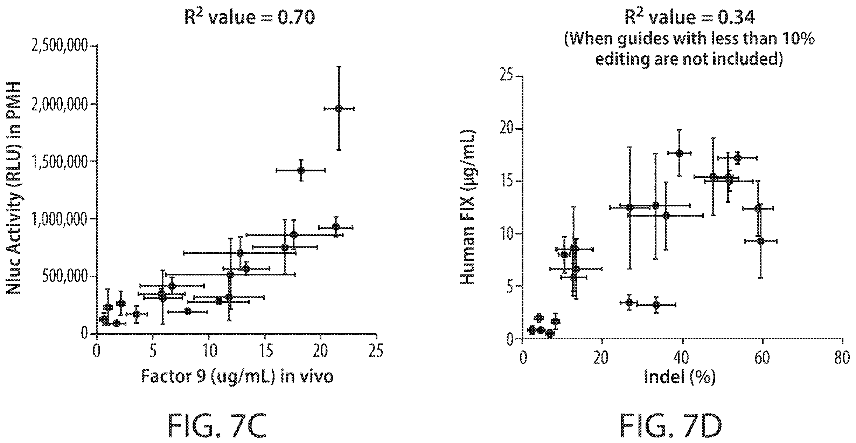

Figs. 7A-7D show results from *in vivo* screening of bidirectional constructs across 20 target sites using ssAAV derived from P00147. Fig. 7A shows editing detected for each of the treatment groups for each LNP/vector combination tested. Fig. 7B provides corresponding targeted insertion data. The results show poor correlation between editing and insertion/expression of the bidirectional constructs (Fig. 7B and Fig. 7D), and a positive correlation between in vitro and in vivo results (Fig. 7C).

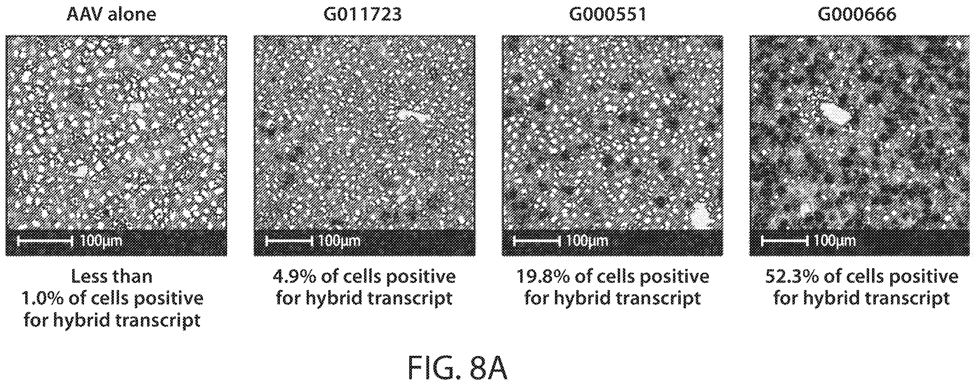

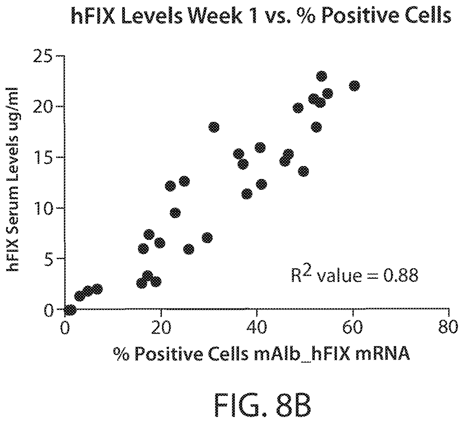

Figs. 8A and 8B show insertion of the bidirectional construct at the cellular level using *in situ* hybridization method using probes that can detect the junctions between the hFIX transgene and the mouse albumin exon 1 sequence (Fig. 8A). Circulating hFIX levels correlated with the number of cells that were positive for the hybrid transcript (Fig. 8B).

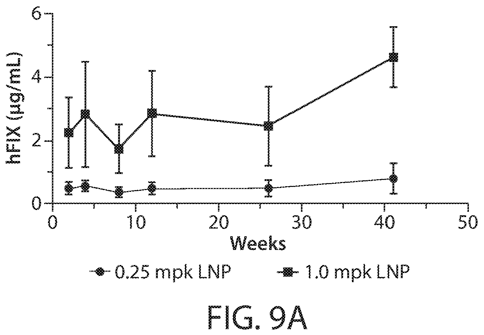

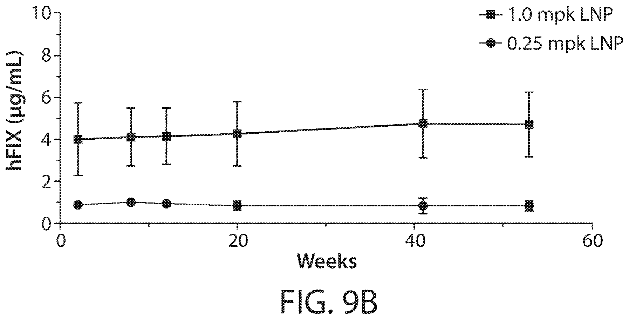

Fig. 9a shows the durability of hFIX expression *in vivo.* Fig. 9b demonstrates expression from intron 1 of albumin was sustained.

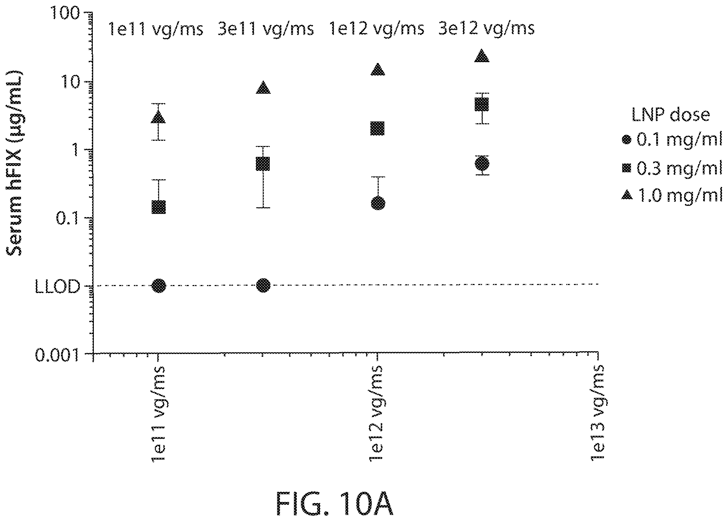

Figs. 10A-10B show that varying AAV or LNP dose can modulate the amount of expression of hFIX from intron 1 of the albumin gene *in vivo.*

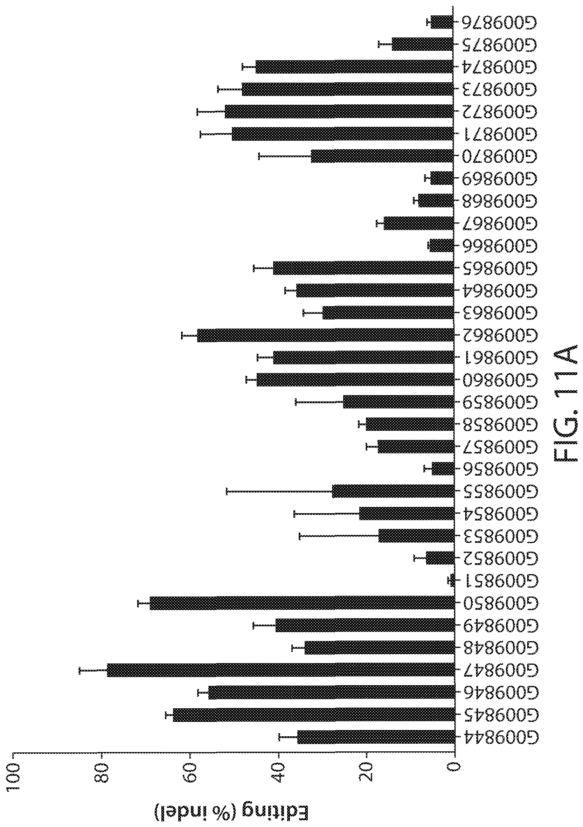

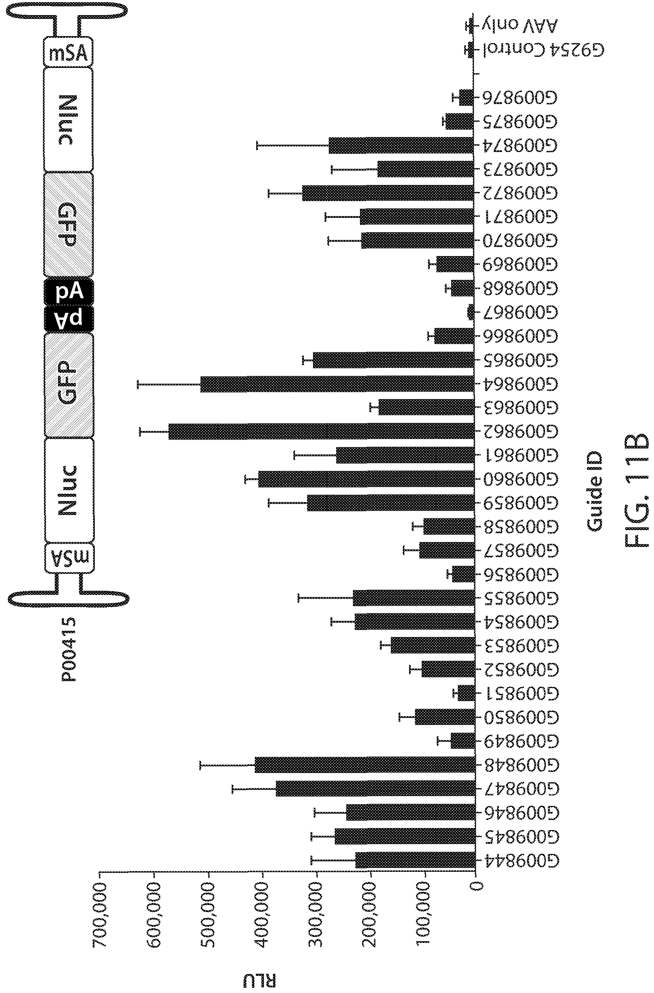

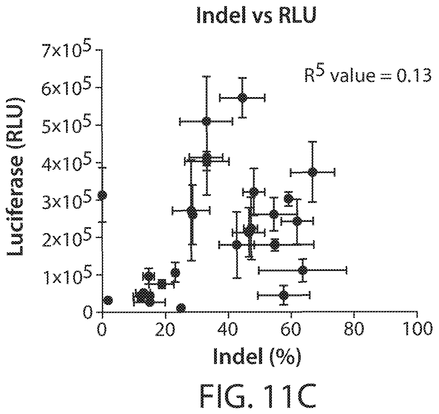

Figs. 11A-11C show results from screening bidirectional constructs across target sites in primary cynomolgus hepatocytes. Fig. 11A shows varied levels of editing as measured by indel formation detected for each of the samples. Fig. 11B and Fig. 11C show that significant levels of indel formation was not predictive for insertion or expression of the bidirectional constructs into intron 1 of albumin.

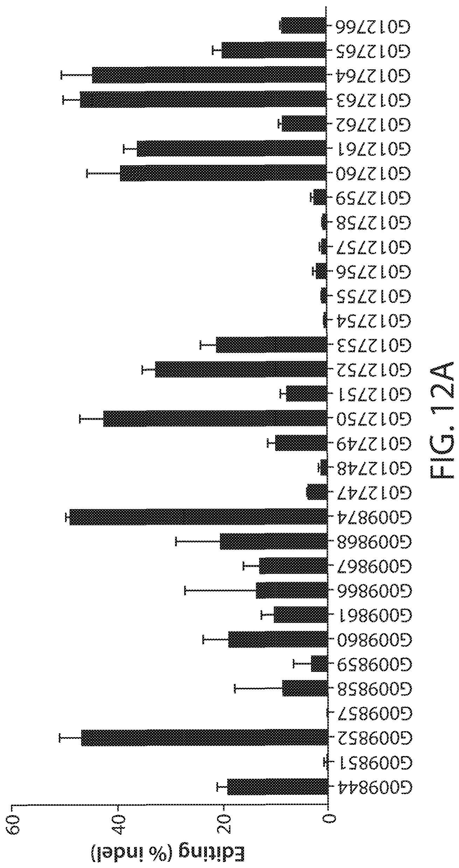

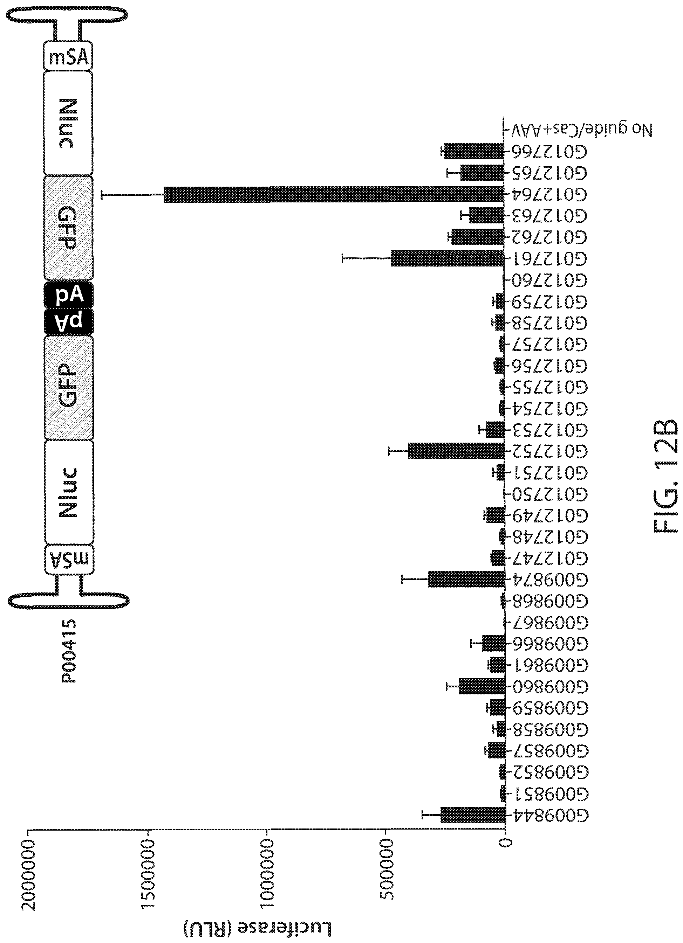

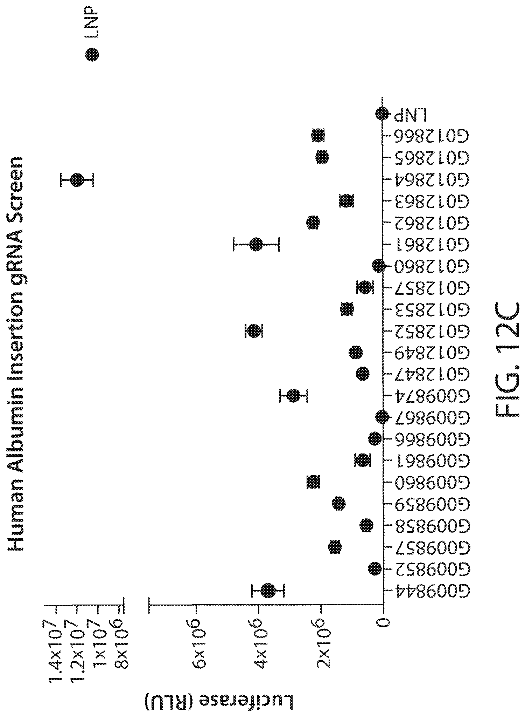

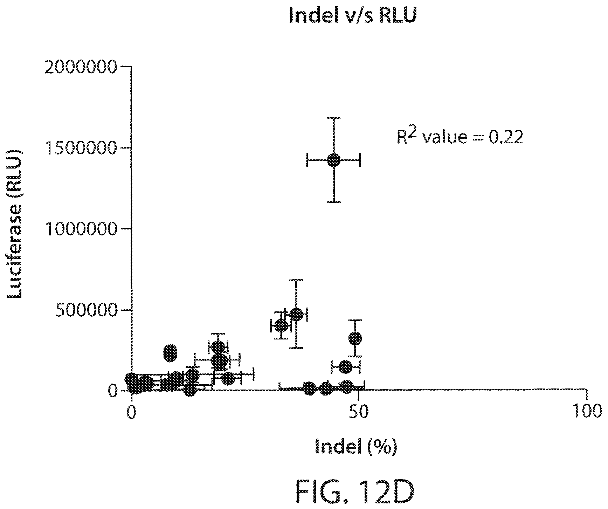

Figs. 12A-12C show results from screening bidirectional constructs across target sites in primary human hepatocytes. Fig. 12A shows editing as measured by indel formation detected for each of the samples. Fig. 12B, Fig. 12C and Fig. 12D show that significant levels of indel formation was not predictive for insertion or expression of the bidirectional constructs into intron 1 of the albumin gene.

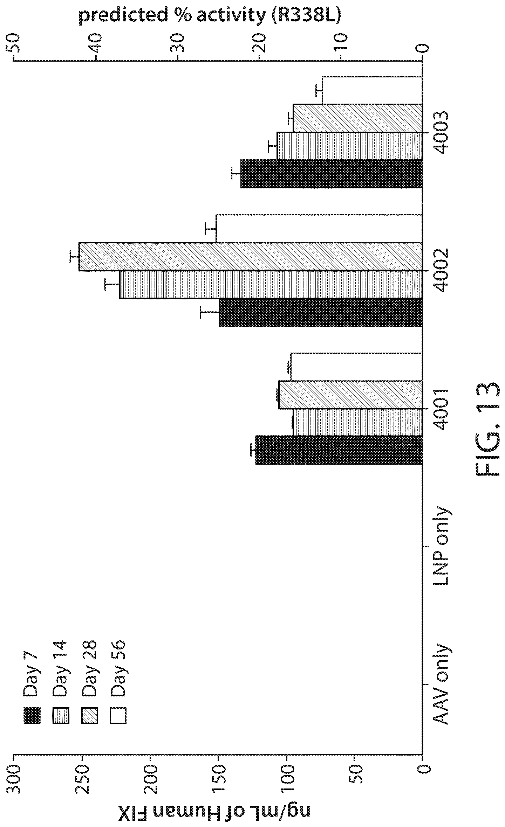

Fig. 13 shows the results of *in vivo* studies where non-human primates were dosed with LNPs along with a bi-directional hFIX insertion template (derived from P00147). Systemic hFIX levels were acheived only in animals treated with both LNPs and AAV, with no hFIX detectable using AAV or LNPs alone.

### DETAILED DESCRIPTION

Reference will now be made in detail to certain embodiments of the invention, examples of which are illustrated in the accompanying drawings. While the present teachings are described in conjunction with various embodiments, it is not intended to limit the present teachings to those embodiments. On the contrary, the present teachings encompass various alternatives, modifications, and equivalents, as will be appreciated by those of skill in the art.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context dictates otherwise. Thus, for example, reference to "a conjugate" includes a plurality of conjugates and reference to "a cell" includes a plurality of cells and the like. As used herein, the term "include" and its grammatical variants are intended to be non-limiting, such that recitation of items in a list is not to the exclusion of other like items that can be substituted or added to the listed items.

Numeric ranges are inclusive of the numbers defining the range. Measured and measureable values are understood to be approximate, taking into account significant digits and the error associated with the measurement. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the specification, embodiments in the specification that recite "comprising" various components are also contemplated as "consisting of" or "consisting essentially of' the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims). The term "or" is used in an inclusive sense, *i.e.,* equivalent to "and/or," unless the context clearly indicates otherwise. The term "about", when used before a list, modifies each member of the list. The term "about" or "approximately" means an acceptable error for a particular value as determined by one of ordinary skill in the art, which depends in part on how the value is measured or determined.

The section headings used herein are for organizational purposes only and are not to be construed as limiting the desired subject matter in any way. In the event that any material incorporated by reference contradicts any term defined in this specification or any other express content of this specification, this specification controls.

### I. Definitions

Unless stated otherwise, the following terms and phrases as used herein are intended to have the following meanings:
"Polynucleotide" and "nucleic acid" are used herein to refer to a multimeric compound comprising nucleosides or nucleoside analogs which have nitrogenous heterocyclic bases or base analogs linked together along a backbone, including conventional RNA, DNA, mixed RNA-DNA, and polymers that are analogs thereof. A nucleic acid "backbone" can be made up of a variety of linkages, including one or more of sugar-phosphodiester linkages, peptide-nucleic acid bonds ("peptide nucleic acids" or PNA; PCT No. WO 95/32305), phosphorothioate linkages, methylphosphonate linkages, or combinations thereof. Sugar moieties of a nucleic acid can be ribose, deoxyribose, or similar compounds with optional substitutions, *e.g*., 2' methoxy or 2' halide substitutions. Nitrogenous bases can be conventional bases (A, G, C, T, U), analogs thereof (*e.g*., modified uridines such as 5-methoxyuridine, pseudouridine, or N1-methylpseudouridine, or others); inosine; derivatives of purines or pyrimidines (*e.g*., N⁴-methyl deoxyguanosine, deaza- or aza-purines, deaza- or aza-pyrimidines, pyrimidine bases with substituent groups at the 5 or 6 position (*e.g.,* 5-methylcytosine), purine bases with a substituent at the 2, 6, or 8 positions, 2-amino-6-methylaminopurine, O⁶-methylguanine, 4-thio-pyrimidines, 4-amino-pyrimidines, 4-dimethylhydrazine-pyrimidines, and O⁴-alkyl-pyrimidines; US Pat. No. 5,378,825 and PCT No. WO 93/13121). For general discussion see The Biochemistry of the Nucleic Acids 5-36, Adams et al., ed., 11th ed., 1992). Nucleic acids can include one or more "abasic" residues where the backbone includes no nitrogenous base for position(s) of the polymer (US Pat. No. 5,585,481). A nucleic acid can comprise only conventional RNA or DNA sugars, bases and linkages, or can include both conventional components and substitutions (*e.g*., conventional nucleosides with 2' methoxy substituents, or polymers containing both conventional nucleotides and one or more nucleotide analogs). Nucleic acid includes "locked nucleic acid" (LNA), an analogue containing one or more LNA nucleotide monomers with a bicyclic furanose unit locked in an RNA mimicking sugar conformation, which enhance hybridization affinity toward complementary RNA and DNA sequences (Vester and Wengel, 2004, Biochemistry 43(42):13233-41). RNA and DNA have different sugar moieties and can differ by the presence of uracil or analogs thereof in RNA and thymine or analogs thereof in DNA.

"Guide RNA", "gRNA", and simply "guide" are used herein interchangeably to refer to either a guide that comprises a guide sequence, *e.g*., crRNA (also known as CRISPR RNA), or the combination of a crRNA and a trRNA (also known as tracrRNA). The crRNA and trRNA may be associated as a single RNA molecule (single guide RNA, sgRNA) or, for example, in two separate RNA molecules (dual guide RNA, dgRNA). "Guide RNA" or "gRNA" refers to each type. The trRNA may be a naturally-occurring sequence, or a trRNA sequence with modifications or variations compared to naturally-occurring sequences. Guide RNAs, such as sgRNAs or dgRNAs, can include modified RNAs as described herein.

As used herein, a "guide sequence" refers to a sequence within a guide RNA that is complementary to a target sequence and functions to direct a guide RNA to a target sequence for binding or modification (*e.g*., cleavage) by an RNA-guided DNA-binding agent. A "guide sequence" may also be referred to as a "targeting sequence," or a "spacer sequence." A guide sequence can be 20 base pairs in length, *e.g.,* in the case of *Streptococcus pyogenes* (*i.e.,* Spy Cas9) and related Cas9 homologs/orthologs. Shorter or longer sequences can also be used as guides, *e.g.,* 15-, 16-, 17-, 18-, 19-, 21-, 22-, 23-, 24-, or 25-nucleotides in length. In some embodiments, the target sequence is in a gene or on a chromosome, for example, and is complementary to the guide sequence. In some embodiments, the degree of complementarity or identity between a guide sequence and its corresponding target sequence may be at least about 75%, 80%, 85%, 90%, 95%, 96%, 97%, 98%, 99%, or 100%. In some embodiments, the guide sequence and the target region may be 100% complementary or identical. In other embodiments, the guide sequence and the target region may contain at least one mismatch. For example, the guide sequence and the target sequence may contain 1, 2, 3, or 4 mismatches, where the total length of the target sequence is at least 17, 18, 19, 20 or more base pairs. In some embodiments, the guide sequence and the target region may contain 1-4 mismatches where the guide sequence comprises at least 17, 18, 19, 20 or more nucleotides. In some embodiments, the guide sequence and the target region may contain 1, 2, 3, or 4 mismatches where the guide sequence comprises 20 nucleotides.

Target sequences for RNA-guided DNA-binding agents include both the positive and negative strands of genomic DNA (*i.e.,* the sequence given and the sequence's reverse complement), as a nucleic acid substrate for an RNA-guided DNA-binding agent is a double stranded nucleic acid. Accordingly, where a guide sequence is said to be "complementary to a target sequence", it is to be understood that the guide sequence may direct a guide RNA to bind to the sense or antisense strand (*e.g*. reverse complement) of a target sequence. Thus, in some embodiments, where the guide sequence binds the reverse complement of a target sequence, the guide sequence is identical to certain nucleotides of the target sequence (*e.g*., the target sequence not including the PAM) except for the substitution of U for T in the guide sequence.

As used herein, an "RNA-guided DNA-binding agent" means a polypeptide or complex of polypeptides having RNA and DNA binding activity, or a DNA-binding subunit of such a complex, wherein the DNA binding activity is sequence-specific and depends on the sequence of the RNA. The term RNA-guided DNA binding-agent also includes nucleic acids encoding such polypeptides. Exemplary RNA-guided DNA-binding agents include Cas cleavases/nickases. Exemplary RNA-guided DNA-binding agents may include inactivated forms thereof ("dCas DNA-binding agents"), *e.g.* if those agents are modified to permit DNA cleavage, *e.g*. via fusion with a FokI cleavase domain. "Cas nuclease", as used herein, encompasses Cas cleavases and Cas nickases. Cas cleavases and Cas nickases include a Csm or Cmr complex of a type III CRISPR system, the Cas10, Csm1, or Cmr2 subunit thereof, a Cascade complex of a type I CRISPR system, the Cas3 subunit thereof, and Class 2 Cas nucleases. As used herein, a "Class 2 Cas nuclease" is a single-chain polypeptide with RNA-guided DNA binding activity. Class 2 Cas nucleases include Class 2 Cas cleavases/nickases (e.g., H840A, D10A, or N863A variants), which further have RNA-guided DNA cleavases or nickase activity, and Class 2 dCas DNA-binding agents, in which cleavase/nickase activity is inactivated"), if those agents are modified to permit DNA cleavage. Class 2 Cas nucleases include, for example, Cas9, Cpfl, C2c1, C2c2, C2c3, HF Cas9 (*e.g.,* N497A, R661A, Q695A, Q926A variants), HypaCas9 (*e.g*., N692A, M694A, Q695A, H698A variants), eSPCas9(1.0) (e.g, K810A, K1003A, R1060A variants), and eSPCas9(1.1) (*e.g*., K848A, K1003A, R1060A variants) proteins and modifications thereof. Cpfl protein, Zetsche et al., Cell, 163: 1-13 (2015), also contains a RuvC-like nuclease domain. Cpfl sequences of Zetsche are incorporated by reference in their entirety. *See, e.g.,* Zetsche, Tables S1 and S3. See, *e.g.,* Makarova et al., Nat Rev Microbiol, 13(11): 722-36 (2015); Shmakov et al., Molecular Cell, 60:385-397 (2015). As used herein, delivery of an RNA-guided DNA-binding agent (*e.g.* a Cas nuclease, a Cas9 nuclease, or an *S. pyogenes* Cas9 nuclease) includes delivery of the polypeptide or mRNA.

As used herein, "ribonucleoprotein" (RNP) or "RNP complex" refers to a guide RNA together with an RNA-guided DNA-binding agent, such as a Cas nuclease, *e.g*., a Cas cleavase, Cas nickase, Cas9 cleavase or Cas9 nickase. In some embodiments, the guide RNA guides the RNA-guided DNA-binding agent such as a Cas9 to a target sequence, and the guide RNA hybridizes with and the agent binds to the target sequence; and binding can be followed by cleaving or nicking.

As used herein, a first sequence is considered to "comprise a sequence with at least X% identity to" a second sequence if an alignment of the first sequence to the second sequence shows that X% or more of the positions of the second sequence in its entirety are matched by the first sequence. For example, the sequence AAGA comprises a sequence with 100% identity to the sequence AAG because an alignment would give 100% identity in that there are matches to all three positions of the second sequence. The differences between RNA and DNA (generally the exchange of uridine for thymidine or vice versa) and the presence of nucleoside analogs such as modified uridines do not contribute to differences in identity or complementarity among polynucleotides as long as the relevant nucleotides (such as thymidine, uridine, or modified uridine) have the same complement (*e.g*., adenosine for all of thymidine, uridine, or modified uridine; another example is cytosine and 5-methylcytosine, both of which have guanosine or modified guanosine as a complement). Thus, for example, the sequence 5'-AXG where X is any modified uridine, such as pseudouridine, N1-methyl pseudouridine, or 5-methoxyuridine, is considered 100% identical to AUG in that both are perfectly complementary to the same sequence (5'-CAU). Exemplary alignment algorithms are the Smith-Waterman and Needleman-Wunsch algorithms, which are well-known in the art. One skilled in the art will understand what choice of algorithm and parameter settings are appropriate for a given pair of sequences to be aligned; for sequences of generally similar length and expected identity >50% for amino acids or >75% for nucleotides, the Needleman-Wunsch algorithm with default settings of the Needleman-Wunsch algorithm interface provided by the EBI at the www.ebi.ac.uk web server is generally appropriate.

As used herein, a first sequence is considered to be "X% complementary to" a second sequence if X% of the bases of the first sequence base pair with the second sequence. For example, a first sequence 5'AAGA3' is 100% complementary to a second sequence 3'TTCT5', and the second sequence is 100% complementary to the first sequence. In some embodiments, a first sequence 5'AAGA3' is 100% complementary to a second sequence 3'TTCTGTGA5', whereas the second sequence is 50% complementary to the first sequence.

"mRNA" is used herein to refer to a polynucleotide that is entirely or predominantly RNA or modified RNA and comprises an open reading frame that can be translated into a polypeptide (*i.e.,* can serve as a substrate for translation by a ribosome and amino-acylated tRNAs). mRNA can comprise a phosphate-sugar backbone including ribose residues or analogs thereof, *e.g*., 2'-methoxy ribose residues. In some embodiments, the sugars of an mRNA phosphate-sugar backbone consist essentially of ribose residues, 2'-methoxy ribose residues, or a combination thereof. Bases of an mRNA can modified bases such as pseudouridine, N-1-methyl-psuedouridine, or other naturally occurring or non-naturally occurring bases.

Exemplary guide sequences useful in the compositions and methods described herein are shown in Table 1 and throughout the application.

As used herein, "indels" refer to insertion/deletion mutations consisting of a number of nucleotides that are either inserted or deleted at the site of double-stranded breaks (DSBs) in a target nucleic acid.

As used herein, "polypeptide" refers to a wild-type or variant protein (*e.g.,* mutant, fragment, fusion, or combinations thereof). A variant polypeptide may possess at least or about 5%, 10%, 15%, 20%, 30%, 35%, 40%, 45%, 50%, 55%, 60%, 65%, 70%, 75%, 80%, 85%, 90%, 95%, or 100% functional activity of the wild-type polypeptide. In some embodiments, the variant is at least 70%, 75%, 80%, 85%, 90%, 92%, 93%, 94%, 95%, 96%, 97%, 98%, or 99% identical to the sequence of the wild-type polypeptide. In some embodiments, a variant polypeptide may be a hyperactive variant. In certain instances, the variant possesses between about 80% and about 120%, 140%, 160%, 180%, 200%, 300%, 400%, 500%, or more of a functional activity of the wild-type polypeptide.

As used herein, a "heterologous gene" refers to a gene that has been introduced as an exogenous source to a site within a host cell genome (*e.g.,* at a genomic locus such as a safe harbor locus, including an albumin intron 1 site). That is, the introduced gene is heterologous with respect to its insertion site. A polypeptide expressed from such heterologous gene is referred to as a "heterologous polypeptide." The heterologous gene can be naturally-occuring or engineered, and can be wild type or a variant. The heterologous gene may include nucleotide sequences other than the sequence that encodes the heterologous polypeptide (*e.g.,* an internal ribosomal entry site). The heterologous gene can be a gene that occurs naturally in the host genome, as a wild type or a variant (*e.g*., mutant). For example, although the host cell contains the gene of interest (as a wild type or as a variant), the same gene or variant thereof can be introduced as an exogenous source for, *e.g*., expression at a locus that is highly expressed. The heterologous gene can also be a gene that is not naturally occurring in the host genome, or that expresses a heterologous polypeptide that does not naturally occur in the host genome. "Heterologous gene", "exogenous gene", and "transgene" are used interchangeably. In some embodiments, the heterologous gene or transgene includes an exogenous nucleic acid sequence, *e.g.* a nucleic acid sequence is not endogenous to the recipient cell. In certain embodiments, the heterologous gene does not naturally ocurr in the recipient cell. For example, the heterologous gene may be heterologous with respect to both its insertion site and with respect to its recipient cell.

As used herein, a "target sequence" refers to a sequence of nucleic acid in a target gene that has complementarity to the guide sequence of the gRNA. The interaction of the target sequence and the guide sequence directs an RNA-guided DNA-binding agent to bind, and potentially nick or cleave (depending on the activity of the agent), within the target sequence.

As used herein, a "bidirectional nucleic acid construct" (interchangeably referred to herein as "bidirectional construct") comprises at least two nucleic acid segments, wherein one segment (the first segment) comprises a coding sequence that encodes an agent of interest (the coding sequence may be referred to herein as "transgene" or a first transgene), while the other segment (the second segment) comprises a sequence wherein the complement of the sequence encodes an agent of interest, or a second transgene. The agent may be therapeutic agent, such as a polypeptide, functional RNA, mRNA, or the like. The transgene may encode for an agent such as a polypeptide, functional RNA, or mRNA. In some embodiments, the bidirectional nucleic acid construct comprises at least two nucleic acid segments, wherein one segment (the first segment) comprises a coding sequence that encodes a polypeptide of interest, while the other segment (the second segment) comprises a sequence wherein the complement of the sequence encodes a polypeptide of interest, or a second transgene. That is, the at least two segments can encode identical or different polypeptides or identical or different agents. When the two segments encode an identical polypeptide, the coding sequence of the first segment need not be identical to the complement of the sequence of the second segment. In some embodiments, the sequence of the second segment is a reverse complement of the coding sequence of the first segment. A bidirectional construct can be single-stranded or double-stranded. The bidirectional construct disclosed herein encompasses a construct that is capable of expressing any polypeptide of interest. The bidirectional constructs are useful for genomic insertion of transgene sequences, in particular targeted insertion of the transgene.

As used herein, a "reverse complement" refers to a sequence that is a complement sequence of a reference sequence, wherein the complement sequence is written in the reverse orientation. For example, for a hypothetical sequence 5' CTGGACCGA 3' (SEQ ID NO: 500), the "perfect" complement sequence is 3' GACCTGGCT 5' (SEQ ID NO: 501), and the "perfect" reverse complement is written 5' TCGGTCCAG 3' (SEQ ID NO: 502). A reverse complement sequence need not be "perfect" and may still encode the same polypeptide or a similar polypeptide as the reference sequence. Due to codon usage redundancy, a reverse complement can diverge from a reference sequence that encodes the same polypeptide. As used herein, "reverse complement" also includes sequences that are, *e.g.,* at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% identical to the reverse complement sequence of a reference sequence.

In some embodiments, a bidirectional nucleic acid construct comprises a first segment that comprises a coding sequence that encodes a first polypeptide (a first transgene), and a second segment that comprises a sequence wherein the complement of the sequence encodes a second polypeptide (a second transgene). In some embodiments, the first and the second polypeptides are at least 50%, 55%, 60%, 65%, 70%, 75%, 80%, 85%, 90%, 91%, 92%, 93%, 94%, 95%, 96%, 97%, 98%, 99%, or 100% identical. In some embodiments, the first and the second polypeptides comprise an amino acid sequence that is at least 80%, 85%, 90%, 91%, 92%, 93%, 94%, 95%, 96%, 97%, 98%, 99%, or 100% identical, e.g. across 50, 100, 200, 500, 1000 or more amino acid residues.

### II. Bidirectional Nucleic Acid Construct

Described herein are bidirectional nucleic acid constructs that facilitate enhanced insertion, *e.g*., enhance productive insertion, and expression of a gene of interest. Briefly, various bidirectional constructs disclosed herein comprise at least two nucleic acid segments, wherein one segment (the first segment) comprises a coding sequence that encodes an agent of interest, *e.g.,* a heterologous gene (the coding sequence may be referred to herein as "transgene" or a first transgene), while the other segment (the second segment) comprises a sequence wherein the complement of the sequence encodes an agent of interest, *e.g*., a heterologous gene, or a second transgene. The agent may be therapeutic agent, such as a polypeptide, functional RNA, mRNA, or the like. The transgene may encode for an agent such as a polypeptide, a functional RNA, an mRNA, or a transcription factor. In some embodiments, a coding sequence encodes a therapeutic agent, such as a polypeptide, or functional RNA. The at least two segments can encode identical or different polypeptides or identical or different agents. In some embodiments, the bidirectional constructs disclosed herein comprise at least two nucleic acid segments, wherein one segment (the first segment) comprises a coding sequence that encodes a polypeptide of interest, while the other segment (the second segment) comprises a sequence wherein the complement of the sequence encodes a polypeptide of interest.

In one embodiment, a bidirectional construct comprise at least two nucleic acid segments in *cis,* wherein one segment (the first segment) comprises a coding sequence (sometimes interchangeably referred to herein as "transgene"), while the other segment (the second segment) comprises a sequence wherein the complement of the sequence encodes a transgene. The first transgene and the second transgene may be the same or different. The bidirectional constructs may comprise at least two nucleic acid segments in *cis,* wherein one segment (the first segment) comprises a coding sequence that encodes a heterologous gene in one orientation, while the other segment (the second segment) comprises a sequence wherein its complement encodes the heterologous gene in the other orientation. That is, the first segment is a complement of the second segment (not necessarily a perfect complement); the complement of the second segment is the reverse complement of the first segment (not necessarily a perfect reverse complement though both encode the same heterologous protein). A bidirectional construct may comprise a first coding sequence that encodes a heterologous gene linked to a splice acceptor and a second coding sequence wherein the complement encodes a heterologous gene in the other orientation, also linked to a splice acceptor.

As used herein, such a construct is sometimes referred to as a "donor construct/template". In some embodiments, the construct is a DNA construct. Methods of designing and making various functional/structural modifications to donor constructs are known in the art. In some embodiments, the construct may comprise any one or more of a polyadenylation tail sequence, a polyadenylation signal sequence, splice acceptor site, or selectable marker. In some embodiments, the polyadenylation tail sequence is encoded, *e.g.,* as a "poly-A" stretch, at the 3' end of the coding sequence.

When used in combination with a gene editing system as described herein, the bidirectionality of the nucleic acid constructs allows the construct to be inserted in either direction (is not limited to insertion in one direction) within a target insertion site, allowing the expression of the polypeptide of interest from either a) a coding sequence of one segment (*e.g.,* the left segment encoding "Human F9" in the upper left ssAAV construct of Fig. 1), or b) a complement of the other segment (*e.g*., the complement of the right segment encoding "Human F9" indicated upside down in the upper left ssAAV construct Fig. 1), thereby enhancing insertion and expression efficiency, as exemplified herein. Targeted cleavage by a gene editing system can facilitate construct integration and/or transgene expression. Various known gene editing systems can be used in the practice of the present disclosure, including, *e.g*., site-specific DNA cleavage systems including a CRISPR/Cas system; zinc finger nuclease (ZFN) system; or transcription activator-like effector nuclease (TALEN) system.

In some embodiments, the bidirectional nucleic acid construct does not comprise a promoter that drives the expression of the agent or polypeptide. For example, the expression of the polypeptide is driven by a promoter of the host cell (*e.g.,* the endogenous albumin promoter when the transgene is integrated into a host cell's albumin locus).

In some embodiments, the bidirectional nucleic acid construct comprises a first segment comprising a coding sequence for a polypeptide and a second segment comprising a reverse complement of a coding sequence of the polypeptide. The same is true for non-polypeptide agents. Thus, the coding sequence in the first segment is capable of expressing a polypeptide, while the complement of the reverse complement in the second segment is also capable of expressing the polypeptide. As used herein, "coding sequence" when referring to the second segment comprising a reverse complement sequence refers to the complementary (coding) strand of the second segment (*i.e.,* the complement coding sequence of the reverse complement sequence in the second segment).

In some embodiments, the coding sequence that encodes Polypeptide A in the first segment is less than 100% complementary to the reverse complement of a coding sequence that also encodes Polypeptide A. That is, in some embodiments, the first segment comprises a coding sequence (1) for Polypeptide A, and the second segment is a reverse complement of a coding sequence (2) for Polypeptide A, wherein the coding sequence (1) is not identical to the coding sequence (2). For example, coding sequence (1) and/or coding sequence (2) that encodes for Polypeptide A can utilize different codons. In some embodiments, one or both sequences can be codon optimized, such that coding sequence (1) and the reverse complement of coding sequence (2) possess 100% or less than 100% complementarity. In some embodiments, the coding sequence of the second segment encodes the polypeptide using one or more alternative codons for one or more amino acids of the same polypeptide encoded by the coding sequence in the first segment. An "alternative codon" as used herein refers to variations in codon usage for a given amino acid, and may or may not be a preferred or optimized codon (codon optimized) for a given expression system. Preferred codon usages, or codons that are well-tolerated in a given system of expression, are known in the art.

In some embodiments, the second segment comprises a reverse complement sequence that adopts different codon usage from that of the coding sequence of the first segment in order to reduce hairpin formation. Such a reverse complement forms base pairs with fewer than all nucleotides of the coding sequence in the first segment, yet it optionally encodes the same polypeptide. In such cases, the coding sequence, *e.g.* for Polypeptide A, of the first segment many be homologous to, but not identical to, the coding sequence, *e.g*. for Polypeptide A of the second half of the bidirectional construct. In some embodiments, the second segment comprises a reverse complement sequence that is not substantially complementary (*e.g.*, not more than 70% complementary) to the coding sequence in the first segment. In some embodiments, the second segment comprises a reverse complement sequence that is highly complementary (*e.g*., at least 90% complementary) to the coding sequence in the first segment. In some embodiments, the second segment comprises a reverse complement sequence having at least about 30%, about 35%, about 40%, about 45%, about 50%, about 55%, about 60%, about 65%, about 70%, about 75%, about 80%, about 85%, about 90%, about 95%, about 97%, or about 99% complementarity to the coding sequence in the first segment.

In some embodiments, the second segment comprises a reverse complement sequence having 100% complementarity to the coding sequence in the first segment. That is, the sequence in the second segment is a perfect reverse complement of the coding sequence in the first segment. By way of example, the first segment comprises a hypothetical sequence 5' CTGGACCGA 3' (SEQ ID NO: 500) and the second segment comprises the reverse complement of SEQ ID NO: 1 *- i.e., 5'* TCGGTCCAG 3' (SEQ ID NO: 502).

In some embodiments, the bidirectional nucleic acid construct comprises a first segment comprising a coding sequence for a polypeptide or agent (*e.g.* a first polypeptide) and a second segment comprising a reverse complement of a coding sequence of a polypeptide or agent (*e.g.* a second polypeptide). In some embodiments, the first polypeptide and the second polypeptide are the same, as described above. In some embodiments, the first therapeutic agent and the second therapeutic agent are the same, as described above. In some embodiments, the first polypeptide and the second polypeptides are different. In some embodiments, the first therapeutic agent and the second therapeutic agent are different. For example, the first polypeptide is Polypeptide A and the second polypeptide is Polypeptide B. As a further example, the first polypeptide is Polypeptide A and the second polypeptide is a variant (*e.g*., a fragment (such as a functional fragment), mutant, fusion (including addition of as few as one amino acid at a polypeptide terminus), or combinations thereof) of Polypeptide A. A coding sequence that encodes a polypeptide may optionally comprise one or more additional sequences, such as sequences encoding amino- or carboxy- terminal amino acid sequences such as a signal sequence, label sequence (*e.g*. HiBit), or heterologous functional sequence (*e.g*. nuclear localization sequence (NLS) or self-cleaving) linked to the polypeptide. A coding sequence that encodes a polypeptide may optionally comprise sequences encoding one or more amino- terminal signal peptide sequences. Each of these additional sequences can be the same or different in the first segment and second segment of the construct.

The bidirectional construct described herein can be used to express any polypeptide according to the methods disclosed herein. In some embodiments, the polypeptide is a secreted polypeptide. In some embodiments, the polypeptide is one in which its function is normally effected (*e.g.*, functionally active) as a secreted polypeptide. A "secreted polypeptide" as used herein refers to a protein that is secreted by the cell and/or is functionally active as a soluble extracellular protein.

In some embodiments, the polypeptide is an intracellular polypeptide. In some embodiments, the polypeptide is one in which its function is normally effected (*e.g.,* functionally active) inside a cell. An "intracellular polypeptide" as used herein refers to a protein that is not secreted by the cell, including soluble cytosolic polypeptides.
In some embodiments, the polypeptide is a wild-type polypeptide.

In some embodiments, the polypeptide is a liver protein or variant thereof. As used herein, a "liver protein" is a protein that is, *e.g*., endogenously produced in the liver and/or functionally active in the liver. In some embodiments, the liver protein is a circulating protein produced by the liver or a variant thereof. In some embodiments, the liver protein is a protein that is functionally active in the liver or a variant thereof. In some embodiments, the liver protein exhibits an elevated expression in liver compared to one or more other tissue types. In some embodiments, the polypeptide is a non-liver protein. In some embodiments, the polypeptide includes, but is not limited to Factor IX and variants thereof.

In some embodiments, the bidirectional nucleic acid construct is linear. For example, the first and second segments are joined in a linear manner through a linker sequence. In some embodiments, the 5' end of the second segment that comprises a reverse complement sequence is linked to the 3' end of the first segment. In some embodiments, the 5' end of the first segment is linked to the 3' end of the second segment that comprises a reverse complement sequence. In some embodiments, the linker sequence is about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250, 300, 500, 1000, 1500, 2000 or more nucleotides in length. As would be appreciated by those of skill in the art, other structural elements in addition to, or instead of a linker sequence, can be inserted between the first and second segments.

The constructs disclosed herein can be modified to include any suitable structural feature as needed for any particular use and/or that confers one or more desired function. In some embodiments, the bidirectional nucleic acid construct disclosed herein does not comprise a homology arm. In some embodiments, the bidirectional nucleic acid construct disclosed herein is a homology-independent donor construct. In some embodiments, owing in part to the bidirectional function of the nucleic acid construct, the bidirectional construct can be inserted into a genomic locus in either direction (orientation) as described herein to allow for efficient insertion and/or expression of a polypeptide of interest. In some embodiments, the bidirectional nucleic acid construct includes a first segment and a second segment, each having a splice acceptor upstream of a transgene. In certain embodiments, the splice acceptor is compatible with the splice donor sequence of the host cell's safe harbor site, *e.g.* the splice donor of intron 1 of a human albumin gene.

In some embodiments, the composition described herein comprises one or more internal ribosome entry site (IRES). First identified as a feature Picorna virus RNA, IRES plays an important role in initiating protein synthesis in absence of the 5' cap structure. An IRES may act as the sole ribosome binding site, or may serve as one of multiple ribosome binding sites of polynucleotides. Constructs containing more than one functional ribosome binding site may encode several peptides or polypeptides that are translated independently by the ribosomes ("multicistronic nucleic acid molecules"). Alternatively, constructs may comprise an IRES in order to express a heterologous protein which is not fused to an endogenous polypeptide (*i.e.* an albumin signal peptide). Examples of IRES sequences that can be utilized include without limitation, those from picornaviruses (*e.g.* FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SIV) or cricket paralysis viruses (CrPV).

In some embodiments, the nucleic acid construct comprises a sequence encoding a self cleaving peptide such as a 2A sequence or a 2A-like sequence. In some embodiment, the self cleaving peptide is located upstream of the polypeptide of interest. In one embodiment, the sequence encoding the 2A peptide may be used to separate the coding region of two or more polypeptides of interest. In another embodiment, this sequence may be used to separate the coding sequence from the construct and the coding sequence from the endogenous locus (*i.e.* endogenous albumin signal sequence). As a non-limiting example, the sequence encoding the 2A peptide may be between region A and region B (A-2A-B). The presence of the 2A peptide would result in the cleavage of one long protein into protein A, protein B and the 2A peptide. Protein A and protein B may be the same or different polypeptides of interest.

In some embodiments, one or both of the first and second segment comprises a polyadenylation tail sequence and/or a polyadenylation signal sequence downstream of an open reading frame. In some embodiments, the polyadenylation tail sequence is encoded, *e.g.,* as a "poly-A" stretch, at the 3' end of the first and/or second segment. In some embodiments, a polyadenylation tail sequence is provided co-transcriptionally as a result of a polyadenylation signal sequence that is encoded at or near the 3' end of the first and/or second segment. In some embodiments, a poly-A tail comprises at least 20, 30, 40, 50, 60, 70, 80, 90, or 100 adenines, optionally up to 300 adenines. In some embodiments, the poly-A tail comprises 95, 96, 97, 98, 99, or 100 adenine nucleotides. Methods of designing a suitable polyadenylation tail sequence and/or polyadenylation signal sequence are well known in the art. Suitable splice acceptor sequences are disclosed and exemplified herein, including mouse albumin and human FIX splice acceptor sites. In some embodiments, the polyadenylation signal sequence AAUAAA (SEQ ID NO: 800) is commonly used in mammalian systems, although variants such as UAUAAA (SEQ ID NO: 801) or AU/GUAAA (SEQ ID NO: 802) have been identified. See, *e.g.,* NJ Proudfoot, Genes & Dev. 25(17):1770-82, 2011. In some embodiments, a polyA tail sequence is included.

In some embodiments, the constructs disclosed herein can be DNA or RNA, single-stranded, double-stranded, or partially single- and partially double-stranded and can be introduced into a host cell in linear or circular (*e*.*g.,* minicircle) form. See, *e.g.,* U.S. Patent Publication Nos. 2010/0047805, 2011/0281361, 2011/0207221. If introduced in linear form, the ends of the donor sequence can be protected (e.g., from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad. Sci. USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues.

In some embodiments, the construct may be inserted so that its expression is driven by the endogenous promoter at the insertion site (*e.g.,* the endogenous albumin promoter when the donor is integrated into the host cell's albumin locus). In such cases, the transgene may lack control elements (*e.g.,* promoter and/or enhancer) that drive its expression (*e.g.,* a promoterless construct). Nonetheless, it will be apparent that in other cases the construct may comprise a promoter and/or enhancer, for example a constitutive promoter or an inducible or tissue specific (*e.g.*, liver- or platelet-specific) promoter that drives expression of the functional protein upon integration. The construct may comprise a sequence encoding a heterologous protein downstream of and operably linked to a signal sequence encoding a signal peptide.

In some embodiments, the nucleic acid construct works in homology-independent insertion of a nucleic acid that encodes a heterologous polypeptide. In some embodiments, the nucleic acid construct works in non-dividing cells, *e.g.,* cells in which NHEJ, not HR, is the primary mechanism by which double-stranded DNA breaks are repaired. The nucleic acid may be a homology-independent donor construct. For example, the constructs can be single- or double-stranded DNA. In some embodiments, the nucleic acid can be modified (*e.g.*, using nucleoside analogs), as described herein.

In some embodiments, the constructs disclosed herein comprise a splice acceptor site on either or both ends of the construct, *e.g.,* 5' of an open reading frame in the first and/or second segments, or 5' of one or both transgene sequences. In some embodiments, the splice acceptor site comprises NAG. In further embodiments, the splice acceptor site consists of NAG. In some embodiments, the splice acceptor is an albumin splice acceptor, *e.g.,* an albumin splice acceptor used in the splicing together of exons 1 and 2 of albumin. In some embodiments, the splice acceptor is derived from the human albumin gene. In some embodiments, the splice acceptor is derived from the mouse albumin gene. In some embodiments, the splice acceptor is a F9 (or "FIX") splice acceptor, *e.g*., the F9 splice acceptor used in the splicing together of exons 1 and 2 of F9. In some embodiments, the splice acceptor is derived from the human F9 gene. In some embodiments, the splice acceptor is derived from the mouse F9 gene. Additional suitable splice acceptor sites useful in eukaryotes, including artificial splice acceptors are known and can be derived from the art. See, *e.g.,* Shapiro, et al., 1987, Nucleic Acids Res., 15, 7155-7174, Burset, et al., 2001, Nucleic Acids Res., 29, 255-259.

In some embodiments, the constructs disclosed herein can be modified on either or both ends to include one or more suitable structural features as needed, and/or to confer one or more functional benefit. For example, structural modifications can vary depending on the method(s) used to deliver the constructs disclosed herein to a host cell - *e.g.,* use of viral vector delivery or packaging into lipid nanoparticles for delivery. Such modifications include, without limitation, *e.g*., terminal structures such as inverted terminal repeats (ITR), hairpin, loops, and other structures such as toroid. In some embodiments, the constructs disclosed herein comprise one, two, or three ITRs. In some embodiments, the constructs disclosed herein comprise no more than two ITRs. Various methods of structural modifications are known in the art.

In some embodiments, one or both ends of the construct can be protected (*e.g.,* from exonucleolytic degradation) by methods known in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad. Sci. USA 84:4959-4963; Nehls et al. (1996) Science 272:886-889. Additional methods for protecting the constructs from degradation include, but are not limited to, addition of terminal amino group(s) and the use of modified internucleotide linkages such as, for example, phosphorothioates, phosphoramidates, and O-methyl ribose or deoxyribose residues.

In some embodiments, the constructs disclosed herein can be introduced into a cell as part of a vector having additional sequences such as, for example, replication origins, promoters and genes encoding antibiotic resistance. A construct may omit viral elements. In some embodiments, the constructs can be introduced as naked nucleic acid, as nucleic acid complexed with an agent such as a liposome, polymer, or poloxamer, or can be delivered by viral vectors (*e.g*., adenovirus, AAV, herpesvirus, retrovirus, lentivirus).

In some embodiments, although not required for expression, the constructs disclosed herein may also include transcriptional or translational regulatory sequences, for example, promoters, enhancers, insulators, internal ribosome entry sites, sequences encoding peptides, and/or polyadenylation signals.

In some embodiments, the constructs comprising a coding sequence for a polypeptide of interest may include one or more of the following modifications: codon optimization (*e.g*., to human codons) and/or addition of one or more glycosylation sites. See, *e.g*., McIntosh et al. (2013) Blood (17):3335-44.

### III. Gene Editing System

Various known gene editing systems can be used in the practice of the present disclosure, including, *e.g.,* a CRISPR/Cas system; zinc finger nuclease (ZFN) system; and transcription activator-like effector nuclease (TALEN) system. Generally, these methods can involve the use of engineered cleavage systems to induce a double strand break (DSB) or a nick (*e.g.,* a single strand break, or SSB) in a target DNA sequence. Cleavage or nicking can occur through the use of specific nucleases such as engineered ZFN, TALENs, or using the CRISPR/Cas system with an engineered guide RNA to guide specific cleavage or nicking of a target DNA sequence. Further, targeted nucleases have been developed, and additional nucleases are being developed, for example based on the Argonaute system (*e.g.,* from *T. thermophilus,* known as 'TtAgo', see Swarts et al (2014) Nature 507(7491): 258-261), which also may have the potential for uses in genome editing and gene therapy.

In some embodiments, a CRISPR/Cas system can be used to create a site of insertion at a desired locus within a host genome, at which site a bidirectional construct disclosed herein can be inserted to express one or more polypeptides of interest. Methods of designing suitable guide RNAs that target any desired locus of a host genome for insertion are well known in the art. A bidirectional construct comprising a transgene may be heterologous with respect to its insertion site, for example, insertion of a heterologous transgene into a "safe harbor" locus. A bidirectional construct comprising a transgene may be non-heterologous with respect to its insertion site, for example, insertion of a wild-type transgene into its endogenous locus.

A "safe harbor" locus is a locus within the genome wherein an exogenous nucleic acid may be inserted without significant deleterious effects on the host cell, *e.g.* hepatocyte, *e.g.,* without causing apoptosis, necrosis, and/or senescence, or without causing more than 5%, 10%, 15%, 20%, 25%, 30%, or 40% apoptosis, necrosis, and/or senescence as compared to a control cell. See, *e.g.,* Hsin et al., "Hepatocyte death in liver inflammation, fibrosis, and tumorigenesis," 2017. In some embodiments, a safe harbor locus allows expression of an exogenous nucleic acid (*e.g.,* an exogenous gene) without significant deleterious effects on the host cell or cell population, such as hepatocytes or liver cells, *e.g.* without causing apoptosis, necrosis, and/or senescence, or without causing more than 5%, 10%, 15%, 20%, 25%, 30%, or 40% apoptosis, necrosis, and/or senescence as compared to a control cell population. The safe harbor may be within an albumin gene, such as a human albumin gene. The safe harbor may be within an albumin intron 1 region, *e.g*., human albumin intron 1. The safe harbor may be a human safe harbor, *e.g.,* for a liver tissue or hepatocyte host cell. Non-limiting examples of safe harbor loci that are targeted by nuclease(s) include CCR5, HPRT, AAVS1, Rosa, albumin, AAVS1 (PPP1 R12C), AngptiS, ApoC3, ASGR2, FIX (F9), G6PC, Gys2, HGD, Lp(a), Pcsk9, SERPINAl, TF, and TTR. See, *e.g.,* U.S. Pat. Nos. 7,951,925 and 8,110,379; U.S. Publication Nos. 2008/0159996; 2010/00218264; 2012/0017290; 2011/0265198; 2013/0137104; 2013/0122591; 2013/0177983;2013/0177960; and WO 2017093804. As exemplified herein, in some embodiments, guide RNAs can be designed to target a human or mouse albumin locus (*e.g*., intron 1). Examples of guide RNAs exemplified herein are shown in Tables 5-10. It will be appreciated that any other locus can be targeted for insertion of a bidirectional construct comprising a transgene according to the present methods.

In some embodiments, the heterologous gene may be inserted into a safe harbor locus and use the safe harbor locus's endogenous signal sequence, *e.g*., the albumin signal sequence encoded by exon 1. For example, an coding sequence may be inserted into human albumin intron 1 such that it is downstream of and fuses to the signal sequence of human albumin exon 1.

In some embodiments, the gene may comprise its own signal sequence, may be inserted into the safe harbor locus, and may further use the safe habor locus's endogenous signal sequence. For example, an coding sequence comprising its native signal sequence may be inserted into human albumin intron 1 such that it is downstream of and and fuses to the signal sequence of human albumin encoded by exon 1.

In some embodiments, the gene may comprise its own signal sequence and an internal ribosomal entry site (IRES), may be inserted into the safe harbor locus, and may further use the safe habor locus's endogenous signal sequence. For example, a coding sequence comprising its native signal sequence and an IRES sequence may be inserted into human albumin intron 1 such that it is downstream of and fuses to the signal sequence of human albumin encoded by exon 1.

In some embodiments, the gene may comprise its own signal sequence and IRES, may be inserted into the safe harbor locus, and does not use the safe habor locus's endogenous signal sequence. For example, a coding sequence comprising its native signal sequence and an IRES sequence may be inserted into human albumin intron 1 such that it does not fuse to the signal sequence of human albumin encoded by exon 1. In these embodiments, the protein is translated from the IRES site and is not chimeric (*e.g.*, albumin signal peptide fused to heterologous protein), which may be advantageously non- or low-immunogenic. In some embodiments, the protein is not secreted and/or transported extracellularly.

In some embodiments, the gene may be inserted into the safe harbor locus and may comprise an IRES and does not not use any signal sequence. For example, a coding sequence comprising an IRES sequence and no native signal sequence may be inserted into human albumin intron 1 such that it does not fuse to the signal sequence of human albumin encoded by exon 1. In some embodiments, the proteins is translated from the IRES site without any signal sequence. In some embodiments, the protein is not secreted and/or transported extracellularly.

It will also be appreciated that a guide RNA for a Cas nuclease, such as a Cas9 nuclease that can be used in the present methods can include any of the various known variations and modifications (*e.g.,* chemical modifications), including the presence of one or more non-naturally and/or naturally occurring components or configurations that are used instead of or in addition to the canonical A, G, C, and U residues. For example, each of the guide sequences exemplified herein (Tables 5-10) may further comprise additional nucleotides to form a crRNA, guide RNA, and/or sgRNA, *e.g.,* from a SpyCas9 CRISPR/Cas system. For example, each of the guide sequences exemplified herein (Tables 5-10) may further comprise additional nucleotides to form a crRNA or sgRNA with the following exemplary nucleotide sequence following the guide sequence at its 3' end: GUUUUAGAGCUAUGCUGUUUUG (SEQ ID NO: 200) in 5' to 3' orientation. In the case of a sgRNA, the guide sequences, such as the guide sequences listed in Tables 5-10 may further comprise additional nucleotides to form a sgRNA, *e.g*., with the following exemplary nucleotide sequence (a SpyCas9 guide sequence) following the 3' end of the guide sequence: 
GUUUUAGAGCUAGAAAUAGCAAGUUAAAAUAAGGCUAGUCCGUUAUCAACUU
GAAAAAGUGGCACCGAGUCGGUGCUUUU (SEQ ID NO: 201)
 GUUUUAGAGCUAGAAAUAGCAAGUUAAAAUAAGGCUAGUCCGUUAUCAACUU GAAAAAGUGGCACCGAGUCGGUGC (SEQ ID NO: 202) in 5' to 3' orientation.

The guide RNA may optionally comprise a trRNA. In each composition and method embodiment described herein, a crRNA and trRNA may be associated as a single RNA (sgRNA) or may be on separate RNAs (dgRNA). In the context of sgRNAs, the crRNA and trRNA components may be covalently linked, *e.g*., via a phosphodiester bond or other covalent bond. In some embodiments, the sgRNA comprises one or more linkages between nucleotides that is not a phosphodiester linkage. In each of the composition, use, and method embodiments described herein, the guide RNA may comprise two RNA molecules as a "dual guide RNA" or "dgRNA". The dgRNA comprises a first RNA molecule comprising a crRNA comprising, *e.g*., a guide sequence shown in any one of Tables 5-10, and a second RNA molecule comprising a trRNA. The first and second RNA molecules may not be covalently linked, but may form a RNA duplex via the base pairing between portions of the crRNA and the trRNA.

In some embodiments, the guide RNAs disclosed herein bind to a region upstream of a propospacer adjacent motif (PAM). As would be understood by those of skill in the art, the PAM sequence occurs on the strand opposite to the strand that contains the target sequence. That is, the PAM sequence is on the complement strand of the target strand (the strand that contains the target sequence to which the guide RNA binds). In some embodiments, the PAM is selected from the group consisting of NGG, NNGRRT, NNGRR(N), NNAGAAW, NNNNG(A/C)TT, and NNNNRYAC. In some embodiments, the PAM is NGG.

In some embodiments, the guide RNA sequences provided herein are complementary to a sequence adjacent to a PAM sequence.

In some embodiments, the guide RNA sequence comprises a sequence that is complementary to a sequence within a genomic region selected from tables herein according to coordinates in human reference genome hg38. In some embodiments, the guide RNA sequence comprises a sequence that is complementary to a sequence that comprises 5, 6, 7, 8, 9, 10, 11, 12, 13, 14, 15, 16, 17, 18, 19, 20, 21, 22, 23, 24, or 25 consecutive nucleotides from within a genomic region selected from Tables 5-10. In some embodiments, the guide RNA sequence comprises a sequence that is complementary to a sequence that comprises 5, 6, 7, 8, 9, 10, 11, 12, 13, 14, 15, 16, 17, 18, 19, 20, 21, 22, 23, 24, or 25 consecutive nucleotides spanning a genomic region selected from Tables 5-10.

The guide RNAs disclosed herein mediate a target-specific cutting resulting in a double-stranded break (DSB). The guide RNAs disclosed herein mediate a target-specific cutting resulting in a single-stranded break (SSB or nick).

Methods of using various RNA-guided DNA-binding agents, *e.g*., a nuclease, such as a Cas nuclease, *e.g*., Cas9, are also well known in the art. While the use of a bidirectional nucleic acid with a CRISPR/Cas system is exemplified herein, it will be appreciated that suitable variations to the system can also be used. It will be appreciated that, depending on the context, the RNA-guided DNA-binding agent can be provided as a nucleic acid (*e.g.,* DNA or mRNA) or as a protein. In some embodiments, the present method can be practiced in a host cell that already comprises and/or expresses an RNA-guided DNA-binding agent.

In some embodiments, the RNA-guided DNA-binding agent, such as a Cas9 nuclease, has cleavase activity, which can also be referred to as double-strand endonuclease activity. In some embodiments, the RNA-guided DNA-binding agent, such as a Cas9 nuclease, has nickase activity, which can also be referred to as single-strand endonuclease activity. In some embodiments, the RNA-guided DNA-binding agent comprises a Cas nuclease. Examples of Cas nucleases include those of the type II CRISPR systems of *S. pyogenes, S. aureus,* and other prokaryotes (see, *e.g.,* the list in the next paragraph), and variant or mutant (*e.g*., engineered, non-naturally occurring, naturally occurring, or or other variant) versions thereof. See, *e.g*., US2016/0312198 A1; US 2016/0312199 A1.

Non-limiting exemplary species that the Cas nuclease can be derived from include *Streptococcus pyogenes, Streptococcus thermophilus, Streptococcus sp., Staphylococcus aureus, Listeria innocua, Lactobacillus gasseri, Francisella novicida, Wolinella succinogenes, Sutterella wadsworthensis, Gammaproteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria, Acidaminococcus sp., Lachnospiraceae* bacterium ND2006, and *Acaryochloris marina.*

In some embodiments, the Cas nuclease is the Cas9 nuclease from *Streptococcus pyogenes.* In some embodiments, the Cas nuclease is the Cas9 nuclease from *Streptococcus thermophilus.* In some embodiments, the Cas nuclease is the Cas9 nuclease from *Neisseria meningitidis.* In some embodiments, the Cas nuclease is the Cas9 nuclease is from *Staphylococcus aureus.* In some embodiments, the Cas nuclease is the Cpfl nuclease from *Francisella novicida.* In some embodiments, the Cas nuclease is the Cpfl nuclease from *Acidaminococcus sp.* In some embodiments, the Cas nuclease is the Cpfl nuclease from *Lachnospiraceae* bacterium ND2006. In further embodiments, the Cas nuclease is the Cpfl nuclease from *Francisella tularensis, Lachnospiraceae bacterium, Butyrivibrio proteoclasticus, Peregrinibacteria bacterium, Parcubacteria bacterium, Smithella, Acidaminococcus, Candidatus Methanoplasma termitum, Eubacterium eligens, Moraxella bovoculi, Leptospira inadai, Porphyromonas crevioricanis, Prevotella disiens,* or *Porphyromonas macacae.* In certain embodiments, the Cas nuclease is a Cpfl nuclease from an *Acidaminococcus* or *Lachnospiraceae.*

In some embodiments, the gRNA together with an RNA-guided DNA-binding agent is called a ribonucleoprotein complex (RNP). In some embodiments, the RNA-guided DNA-binding agent is a Cas nuclease. In some embodiments, the gRNA together with a Cas nuclease is called a Cas RNP. In some embodiments, the RNP comprises Type-I, Type-II, or Type-III components. In some embodiments, the Cas nuclease is the Cas9 protein from the Type-II CRISPR/Cas system. In some embodiment, the gRNA together with Cas9 is called a Cas9 RNP.

Wild type Cas9 has two nuclease domains: RuvC and HNH. The RuvC domain cleaves the non-target DNA strand, and the HNH domain cleaves the target strand of DNA. In some embodiments, the Cas9 protein comprises more than one RuvC domain and/or more than one HNH domain. In some embodiments, the Cas9 protein is a wild type Cas9. In each of the composition, use, and method embodiments, the Cas induces a double strand break in target DNA.

In some embodiments, chimeric Cas nucleases are used, where one domain or region of the protein is replaced by a portion of a different protein. In some embodiments, a Cas nuclease domain may be replaced with a domain from a different nuclease such as Fok1. In some embodiments, a Cas nuclease may be a modified nuclease.

In other embodiments, the Cas nuclease may be from a Type-I CRISPR/Cas system. In some embodiments, the Cas nuclease may be a component of the Cascade complex of a Type-I CRISPR/Cas system. In some embodiments, the Cas nuclease may be a Cas3 protein. In some embodiments, the Cas nuclease may be from a Type-III CRISPR/Cas system. In some embodiments, the Cas nuclease may have an RNA cleavage activity.

In some embodiments, the RNA-guided DNA-binding agent has single-strand nickase activity, i.e., can cut one DNA strand to produce a single-strand break, also known as a "nick." In some embodiments, the RNA-guided DNA-binding agent comprises a Cas nickase. A nickase is an enzyme that creates a nick in dsDNA, *i.e.,* cuts one strand but not the other of the DNA double helix. In some embodiments, a Cas nickase is a version of a Cas nuclease (*e.g.,* a Cas nuclease discussed above) in which an endonucleolytic active site is inactivated, *e.g.,* by one or more alterations (*e.g.,* point mutations) in a catalytic domain. See, *e.g.,* US Pat. No. 8,889,356 for discussion of Cas nickases and exemplary catalytic domain alterations. In some embodiments, a Cas nickase such as a Cas9 nickase has an inactivated RuvC or HNH domain.

In some embodiments, the RNA-guided DNA-binding agent is modified to contain only one functional nuclease domain. For example, the agent protein may be modified such that one of the nuclease domains is mutated or fully or partially deleted to reduce its nucleic acid cleavage activity. In some embodiments, a nickase is used having a RuvC domain with reduced activity. In some embodiments, a nickase is used having an inactive RuvC domain. In some embodiments, a nickase is used having an HNH domain with reduced activity. In some embodiments, a nickase is used having an inactive HNH domain.

In some embodiments, a conserved amino acid within a Cas protein nuclease domain is substituted to reduce or alter nuclease activity. In some embodiments, a Cas nuclease may comprise an amino acid substitution in the RuvC or RuvC-like nuclease domain. Exemplary amino acid substitutions in the RuvC or RuvC-like nuclease domain include D10A (based on the S. *pyogenes* Cas9 protein). *See, e.g.,* Zetsche et al. (2015) *Cell* Oct 22:163(3): 759-771. In some embodiments, the Cas nuclease may comprise an amino acid substitution in the HNH or HNH-like nuclease domain. Exemplary amino acid substitutions in the HNH or HNH-like nuclease domain include E762A, H840A, N863A, H983A, and D986A (based on the S. *pyogenes* Cas9 protein). *See, e.g.,* Zetsche et al. (2015). Further exemplary amino acid substitutions include D917A, E1006A, and D1255A (based on the *Francisella novicida* U112 Cpfl (FnCpf1) sequence (UniProtKB - A0Q7Q2 (CPF1_FRATN)).

In some embodiments, a nickase is provided in combination with a pair of guide RNAs that are complementary to the sense and antisense strands of the target sequence, respectively. In this embodiment, the guide RNAs direct the nickase to a target sequence and introduce a DSB by generating a nick on opposite strands of the target sequence (*i.e.,* double nicking). In some embodiments, a nickase is used together with two separate guide RNAs targeting opposite strands of DNA to produce a double nick in the target DNA. In some embodiments, a nickase is used together with two separate guide RNAs that are selected to be in close proximity to produce a double nick in the target DNA.

In some embodiments, the RNA-guided DNA-binding agent comprises one or more heterologous functional domains (*e.g*., is or comprises a fusion polypeptide).

In some embodiments, the heterologous functional domain may facilitate transport of the RNA-guided DNA-binding agent into the nucleus of a cell. For example, the heterologous functional domain may be a nuclear localization signal (NLS). In some embodiments, the RNA-guided DNA-binding agent may be fused with 1-10 NLS(s). In some embodiments, the RNA-guided DNA-binding agent may be fused with 1-5 NLS(s). In some embodiments, the RNA-guided DNA-binding agent may be fused with one NLS. Where one NLS is used, the NLS may be linked at the N-terminus or the C-terminus of the RNA-guided DNA-binding agent sequence. It may also be inserted within the RNA-guided DNA-binding agent sequence. In other embodiments, the RNA-guided DNA-binding agent may be fused with more than one NLS. In some embodiments, the RNA-guided DNA-binding agent may be fused with 2, 3, 4, or 5 NLSs. In some embodiments, the RNA-guided DNA-binding agent may be fused with two NLSs. In certain circumstances, the two NLSs may be the same (*e.g.,* two SV40 NLSs) or different. In some embodiments, the RNA-guided DNA-binding agent is fused to two SV40 NLS sequences linked at the carboxy terminus. In some embodiments, the RNA-guided DNA-binding agent may be fused with two NLSs, one linked at the N-terminus and one at the C-terminus. In some embodiments, the RNA-guided DNA-binding agent may be fused with 3 NLSs. In some embodiments, the RNA-guided DNA-binding agent may be fused with no NLS. In some embodiments, the NLS may be a monopartite sequence, such as, *e.g.,* the SV40 NLS, PKKKRKV (SEQ ID NO: 600) or PKKKRRV (SEQ ID NO: 601). In some embodiments, the NLS may be a bipartite sequence, such as the NLS of nucleoplasmin, KRPAATKKAGQAKKKK (SEQ ID NO: 602). In a specific embodiment, a single PKKKRKV (SEQ ID NO: 600) NLS may be linked at the C-terminus of the RNA-guided DNA-binding agent. One or more linkers are optionally included at the fusion site.

As noted above, RNA-guided DNA binding agent can be a nucleic acid encoding an RNA-guided DNA binding polypeptides. In some embodiments, an RNA-guided DNA binding agent comprises an mRNA comprising an open reading frame (ORF) encoding an RNA-guided DNA binding agent, such as a Casintegrate nuclease as described herein. In some embodiments, an mRNA comprising an ORF encoding an RNA-guided DNA binding agent, such as a Cas nuclease, is provided, used, or administered. As described below, the mRNA comprising a Cas nuclease may comprise a Cas9 nuclease, such as an *S. pyogenes* Cas9 nuclease having cleavase, nickase, and/or site-specific DNA binding activity. In some embodiments, the ORF encoding an RNA-guided DNA nuclease is a "modified RNA-guided DNA binding agent ORF" or simply a "modified ORF," which is used as shorthand to indicate that the ORF is modified.

Cas9 ORFs, including modified Cas9 ORFs, are provided herein and are known in the art. As one example, the Cas9 ORF can be codon optimized, such that coding sequence includes one or more alternative codons for one or more amino acids. An "alternative codon" as used herein refers to variations in codon usage for a given amino acid, and may or may not be a preferred or optimized codon (codon optimized) for a given expression system. Preferred codon usage, or codons that are well-tolerated in a given system of expression, is known in the art. The Cas9 coding sequences, Cas9 mRNAs, and Cas9 protein sequences of WO2013/176772, WO2014/065596, WO2016/106121, and WO2019/067910 are hereby incorporated by reference. In particular, the ORFs and Cas9 amino acid sequences of the table at paragraph [0449] WO2019/067910, and the Cas9 mRNAs and ORFs of paragraphs [0214] - [0234] of WO2019/067910 are hereby incorporated by reference.

In some embodiments, the modified ORF may comprise a modified uridine at least at one, a plurality of, or all uridine positions. In some embodiments, the modified uridine is a uridine modified at the 5 position, *e.g*., with a halogen, methyl, or ethyl. In some embodiments, the modified uridine is a pseudouridine modified at the 1 position, *e.g*., with a halogen, methyl, or ethyl. The modified uridine can be, for example, pseudouridine, N1-methyl-pseudouridine, 5-methoxyuridine, 5-iodouridine, or a combination thereof. In some embodiments, the modified uridine is 5-methoxyuridine. In some embodiments, the modified uridine is 5-iodouridine. In some embodiments, the modified uridine is pseudouridine. In some embodiments, the modified uridine is N1-methyl-pseudouridine. In some embodiments, the modified uridine is a combination of pseudouridine and N1-methyl-pseudouridine. In some embodiments, the modified uridine is a combination of pseudouridine and 5-methoxyuridine. In some embodiments, the modified uridine is a combination of N1-methyl pseudouridine and 5-methoxyuridine. In some embodiments, the modified uridine is a combination of 5-iodouridine and N1-methyl-pseudouridine. In some embodiments, the modified uridine is a combination of pseudouridine and 5-iodouridine. In some embodiments, the modified uridine is a combination of 5-iodouridine and 5-methoxyuridine.

In some embodiments, an mRNA disclosed herein comprises a 5' cap, such as a Cap0, Cap1, or Cap2. A 5' cap is generally a 7-methylguanine ribonucleotide (which may be further modified, as discussed below *e.g*. with respect to ARCA) linked through a 5'-triphosphate to the 5' position of the first nucleotide of the 5'-to-3' chain of the mRNA, *i.e.,* the first cap-proximal nucleotide. In Cap0, the riboses of the first and second cap-proximal nucleotides of the mRNA both comprise a 2'-hydroxyl. In Cap1, the riboses of the first and second transcribed nucleotides of the mRNA comprise a 2'-methoxy and a 2'-hydroxyl, respectively. In Cap2, the riboses of the first and second cap-proximal nucleotides of the mRNA both comprise a 2'-methoxy. See, *e.g.,* Katibah et al. (2014) Proc Natl Acad Sci USA 111(33):12025-30; Abbas et al. (2017) Proc Natl Acad Sci USA 114(11):E2106-E2115. Most endogenous higher eukaryotic mRNAs, including mammalian mRNAs such as human mRNAs, comprise Cap1 or Cap2. Cap0 and other cap structures differing from Cap1 and Cap2 may be immunogenic in mammals, such as humans, due to recognition as "non-self" by components of the innate immune system such as IFIT-1 and IFIT-5, which can result in elevated cytokine levels including type I interferon. Components of the innate immune system such as IFIT-1 and IFIT-5 may also compete with eIF4E for binding of an mRNA with a cap other than Cap1 or Cap2, potentially inhibiting translation of the mRNA.

A cap can be included co-transcriptionally. For example, ARCA (anti-reverse cap analog; Thermo Fisher Scientific Cat. No. AM8045) is a cap analog comprising a 7-methylguanine 3'-methoxy-5'-triphosphate linked to the 5' position of a guanine ribonucleotide which can be incorporated in vitro into a transcript at initiation. ARCA results in a Cap0 cap in which the 2' position of the first cap-proximal nucleotide is hydroxyl. See, *e.g*., Stepinski et al., (2001) "Synthesis and properties of mRNAs containing the novel 'anti-reverse' cap analogs 7-methyl(3'-O-methyl)GpppG and 7-methyl(3'deoxy)GpppG," RNA 7: 1486-1495. The ARCA structure is shown below.

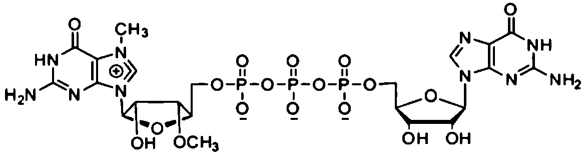

CleanCap^{™} AG (m7G(5')ppp(5')(2'OMeA)pG; TriLink Biotechnologies Cat. No. N-7113) or CleanCap^{™} GG (m7G(5')ppp(5')(2'OMeG)pG; TriLink Biotechnologies Cat. No. N-7133) can be used to provide a Cap1 structure co-transcriptionally. 3'-O-methylated versions of CleanCap^{™} AG and CleanCap^{™} GG are also available from TriLink Biotechnologies as Cat. Nos. N-7413 and N-7433, respectively. The CleanCap^{™} AG structure is shown below.

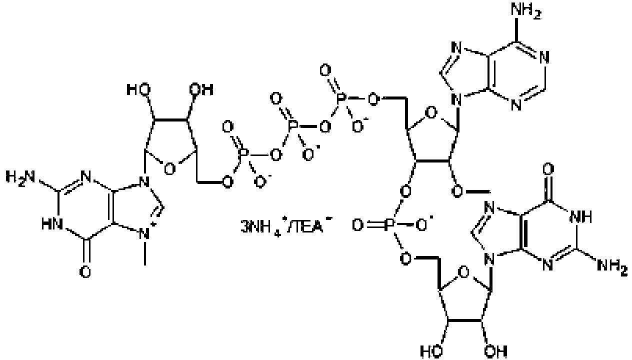

Alternatively, a cap can be added to an RNA post-transcriptionally. For example, Vaccinia capping enzyme is commercially available (New England Biolabs Cat. No. M2080S) and has RNA triphosphatase and guanylyltransferase activities, provided by its D1 subunit, and guanine methyltransferase, provided by its D12 subunit. As such, it can add a 7-methylguanine to an RNA, so as to give Cap0, in the presence of S-adenosyl methionine and GTP. See, *e.g.,* Guo, P. and Moss, B. (1990) Proc. Natl. Acad. Sci. USA 87, 4023-4027; Mao, X. and Shuman, S. (1994) J. Biol. Chem. 269, 24472-24479.

In some embodiments, the mRNA further comprises a poly-adenylated (poly-A) tail. In some embodiments, the poly-A tail comprises at least 20, 30, 40, 50, 60, 70, 80, 90, or 100 adenines, optionally up to 300 adenines. In some embodiments, the poly-A tail comprises 95, 96, 97, 98, 99, or 100 adenine nucleotides.

### IV. Delivery Methods

The nucleic acid constructs disclosed herein can be delivered to a host cell or subject, in vivo or ex vivo, using various known and suitable methods available in the art. The nucleic acid constructs can be delivered together with components of a suitable gene editing system (*e.g*., RNA-guided DNA-binding agent such as a Cas nuclease with its corresponding guide RNA) as described herein.

Conventional viral and non-viral based gene delivery methods can be used to introduce the constructs disclosed herein and components of the gene editing system in cells (*e.g*., mammalian cells) and target tissues. As further provided herein, non-viral vector delivery systems include nucleic acids such as non-viral vectors, plasmid vectors, and, *e.g.* nucleic acid complexed with a delivery vehicle such as a liposome, lipid nanoparticle (LNP), or poloxamer. Viral vector delivery systems include DNA and RNA viruses.

Methods and compositions for non-viral delivery of nucleic acids include electroporation, lipofection, microinjection, biolistics, virosomes, liposomes, immunoliposomes, LNPs, polycation or lipid:nucleic acid conjugates, naked nucleic acid (*e.g.,* naked DNA/RNA), artificial virions, and agent-enhanced uptake of DNA. Sonoporation using, *e.g.,* the Sonitron 2000 system (Rich-Mar) can also be used for delivery of nucleic acids.

Additional exemplary nucleic acid delivery systems include those provided by AmaxaBiosystems (Cologne, Germany), Maxcyte, Inc. (Rockville, Md.), BTX Molecular Delivery Systems (Holliston, Ma.) and Copernicus Therapeutics Inc., (see for example U.S. Pat. No. 6,008,336). Lipofection is described in *e.g.,* U.S. Pat. Nos. 5,049,386; 4,946,787; and 4,897,355) and lipofection reagents are sold commercially (*e.g*., Transfectam^{™} and Lipofectin^{™}). The preparation of lipid:nucleic acid complexes, including targeted liposomes such as immunolipid complexes, is well known in the art, and as described herein.

Various delivery systems (*e.g*., vectors, liposomes, LNPs) containing the bidirectional constructs and/or gene editing components (*e.g*., guide RNA and Cas) can also be administered to an organism for delivery to cells in vivo or administered to a cell or cell culture ex vivo. Administration is by any of the routes normally used for introducing a molecule into ultimate contact with blood, fluid, or cells including, but not limited to, injection, infusion, topical application and electroporation. Suitable methods of administering such nucleic acids are available and well known to those of skill in the art.

In certain embodiments, the present disclosure provides vectors comprising the bidirectional nucleic acid constructs disclosed herein for delivery to a host cell. In certain embodiments, components of the gene editing system (*e.g*., RNA-guided DNA-binding agent and guide RNA) are also delivered to a host cell as part of a vector. In certain embodiments, viral vectors can be used to deliver any one or more of a bidirectional nucleic acid construct, guide RNA, and/or RNA-guided DNA-binding agent to a host cell.

In some embodiments, provided herein are compositions and methods for delivering the bidirectional nucleic acid construct disclosed herein to a host cell or subject, wherein the construct is part of a vector system as described herein. In some embodiments, the vector system comprises additional components, such as components of a gene editing system (*e.g*., guide RNA and/or an RNA-guided DNA-binding agent).

In some embodiments, a vector composition comprising the bidirectional nucleic acid construct disclosed herein is provided. In some embodiments, the composition further comprises components of a gene editing system (*e.g*., guide RNA and/or an RNA-guided DNA-binding agent).

In some embodiments, the vector may be circular. In other embodiments, the vector may be linear. In some embodiments, the vector may be delivered via a lipid nanoparticle, liposome, non-lipid nanoparticle, or viral capsid. Non-limiting exemplary vectors include plasmids, phagemids, cosmids, artificial chromosomes, minichromosomes, transposons, viral vectors, and expression vectors.

In some embodiments, the vector system may be capable of driving expression of one or more nuclease components in a cell. In some embodiments, the bidirectional construct, optionally as part of a vector system, may comprise a promoter capable of driving expression of a coding sequence in a cell. In some embodiments, the cell may be a eukaryotic cell, such as, *e.g.,* a yeast, plant, insect, or mammalian cell. In some embodiments, the eukaryotic cell may be a mammalian cell. In some embodiments, the eukaryotic cell may be a rodent cell. In some embodiments, the eukaryotic cell may be a human cell. Suitable promoters to drive expression in different types of cells are known in the art. In some embodiments, the promoter may be wild type. In other embodiments, the promoter may be modified for more efficient or efficacious expression. In yet other embodiments, the promoter may be truncated yet retain its function. For example, the promoter may have a normal size or a reduced size that is suitable for proper packaging of the vector into a virus. In some embodiments, the vector does not comprise a promoter that drives expression of one or more coding sequences in a cell (*e.g.,* the expression of the coding sequence, once inserted into a target endogenous locus, is driven by an endogenous promoter).

In some embodiments, the vector may be a viral vector. In some embodiments, the viral vector may be genetically modified from its wild type counterpart. For example, the viral vector may comprise an insertion, deletion, or substitution of one or more nucleotides to facilitate cloning or such that one or more properties of the vector is changed. Such properties may include packaging capacity, transduction efficiency, immunogenicity, genome integration, replication, transcription, and translation. In some embodiments, a portion of the viral genome may be deleted such that the virus is capable of packaging exogenous sequences having a larger size. In some embodiments, the viral vector may have an enhanced transduction efficiency. In some embodiments, the immune response induced by the virus in a host may be reduced. In some embodiments, viral genes (such as, *e.g*., integrase) that promote integration of the viral sequence into a host genome may be mutated such that the virus becomes non-integrating. In some embodiments, the viral vector may be replication defective. In some embodiments, the viral vector may comprise exogenous transcriptional or translational control sequences to drive expression of coding sequences on the vector. In some embodiments, the virus may be helper-dependent. For example, the virus may need one or more helper virus to supply viral components (such as, *e.g.,* viral proteins) required to amplify and package the vectors into viral particles. In such a case, one or more helper components, including one or more vectors encoding the viral components, may be introduced into a host cell along with the vector system described herein. In other embodiments, the virus may be helper-free. For example, the virus may be capable of amplifying and packaging the vectors without a helper virus. In some embodiments, the vector system described herein may also encode the viral components required for virus amplification and packaging.

The use of RNA or DNA viral based systems for the delivery of nucleic acids take advantage of highly evolved processes for targeting a virus to specific cells in the body and trafficking the viral payload to the nucleus. Viral vectors can be administered directly to a subject (in vivo) or they can be used to treat cells in vitro. In some embodiments, the cells modified in vitro are administered to a subject (*e.g.,* as an ex vivo manipulation of cells derived from the subject or from a donor source). Non-limiting exemplary viral vectors include adeno-associated virus (AAV) vector, lentivirus vectors, adenovirus vectors, helper dependent adenoviral vectors (HDAd), herpes simplex virus (HSV-1) vectors, bacteriophage T4, baculovirus vectors, and retrovirus vectors. Integration in the host genome is possible with, *e.g.,* the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the bidirectional construct comprising a transgene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (see, *e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In some embodiments, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Replication-deficient recombinant adenoviral vectors can be produced at high titer and readily infect a number of different cell types. Most adenovirus vectors are engineered such that a transgene replaces the Ad E1*a,* E1*b,* and/or E3 genes; subsequently the replication defective vector is propagated in human 293 cells that supply deleted gene function in trans. Ad vectors can transduce multiple types of tissues in vivo, including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 01998); Sterman et. al., Hum. Gene Ther. 7:1083-1089 (1998).

In some embodiments, adeno-associated virus (AAV) vectors are used to deliver bidirectional nucleic acid constructs provided herein. AAV vectors are well known and have been used to transduce cells with target nucleic acids, *e.g.,* in the in vitro production of nucleic acids and peptides, and for in vivo and ex vivo gene therapy procedures (see, *e.g.,* West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors is described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989). In some embodiments, the viral vector may be an AAV vector. In some embodiments, the AAV vector is, *e.g.,* AAV1, AAV2, AAV3, AAV3B, AAV4, AAV5, AAV6, AAV6.2, AAV7, AAVrh.64R1, AAVhu.37, AAVrh.8, AAVrh.32.33, AAV8, AAV9, AAV-DJ, AAV2/8, AAVrh10, or AAVLK03 as well as any novel AAV serotype can also be used in accordance with the present invention. The AAV vector Recombinant adeno-associated virus vectors are a promising alternative nucleic acid delivery systems, for example those based on the defective and nonpathogenic parvovirus adeno-associated type 2 virus.

As used herein, "AAV" refers all serotypes, subtypes, and naturally-occuring AAV as well as recombinant AAV. "AAV" may be used to refer to the virus itself or a derivative thereof. The term "AAV" includes AAV1, AAV2, AAV3, AAV3B, AAV4, AAV5, AAV6, AAV6.2, AAV7, AAVrh.64R1, AAVhu.37, AAVrh.8, AAVrh.32.33, AAV8, AAV9, AAV-DJ, AAV2/8, AAVrh10, AAVLK03, AV10, AAV11, AAV12, rh10, and hybrids thereof, avian AAV, bovine AAV, canine AAV, equine AAV, primate AAV, nonprimate AAV, and ovine AAV. The genomic sequences of various serotypes of AAV, as well as the sequences of the native terminal repeats (TRs), Rep proteins, and capsid subunits are known in the art. Such sequences may be found in the literature or in public databases such as GenBank. A "AAV vector" as used herein refers to an AAV vector comprising a heterologous sequence not of AAV origin (*i.e.,* a nucleic acid sequence heterologous to AAV), typically comprising a sequence encoding a heterologous polypeptide of interest. The construct may comprise an AAV1, AAV2, AAV3, AAV3B, AAV4, AAV5, AAV6, AAV6.2, AAV7, AAVrh.64R1, AAVhu.37, AAVrh.8, AAVrh.32.33, AAV8, AAV9, AAV-DJ, AAV2/8, AAVrh10, AAVLK03, AV10, AAV11, AAV12, rh10, and hybrids thereof, avian AAV, bovine AAV, canine AAV, equine AAV, primate AAV, nonprimate AAV, and ovine AAV capside sequence. In general, the heterologous nucleic acid sequence (the transgene) is flanked by at least one, and generally by two, AAV inverted terminal repeat sequences (ITRs). An AAV vector may either be single-stranded (ssAAV) or self-complementary (scAAV).

In other embodiments, the viral vector may a lentivirus vector. In some embodiments, the lentivirus may be non-integrating. In some embodiments, the viral vector may be an adenovirus vector. In some embodiments, the adenovirus may be a high-cloning capacity or "gutless" adenovirus, where all coding viral regions apart from the 5' and 3' inverted terminal repeats (ITRs) and the packaging signal ('I') are deleted from the virus to increase its packaging capacity. In yet other embodiments, the viral vector may be an HSV-1 vector. In some embodiments, the HSV-1-based vector is helper dependent, and in other embodiments it is helper independent. For example, an amplicon vector that retains only the packaging sequence requires a helper virus with structural components for packaging, while a 30kb-deleted HSV-1 vector that removes non-essential viral functions does not require helper virus. In additional embodiments, the viral vector may be bacteriophage T4. In some embodiments, the bacteriophage T4 may be able to package any linear or circular DNA or RNA molecules when the head of the virus is emptied. In further embodiments, the viral vector may be a baculovirus vector. In yet further embodiments, the viral vector may be a retrovirus vector. In embodiments using AAV or lentiviral vectors, which have smaller cloning capacity, it may be necessary to use more than one vector to deliver all the components of a vector system as disclosed herein. For example, one AAV vector may contain sequences encoding an RNA-guided DNA binding agent such as a Cas protein (*e.g*., Cas9), while a second AAV vector may contain one or more guide sequences.

Packaging cells are used to form virus particles that are capable of infecting a host cell. Such cells include 293 cells, which can package adenovirus and AAV, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging, other viral sequences being replaced by sequences encoding the protein to be expressed. The missing viral functions are supplied in trans by the packaging cell line. For example, AAV vectors used in gene therapy typically only possess inverted terminal repeat (ITR) sequences from the AAV genome which are required for packaging. Viral DNA is packaged in a cell line, which contains a helper plasmid encoding the other AAV genes, namely rep and cap, but lacking ITR sequences. The cell line may also be infected with adenovirus as a helper. The helper virus promotes replication of the AAV vector and expression of AAV genes from the helper plasmid.

Gene therapy vectors can be delivered in vivo by administration to an individual patient, typically by systemic administration (*e.g*., intravenous, intraperitoneal, intramuscular, subdermal, or intracranial infusion) or topical application, as described below. Alternatively, vectors can be delivered to cells ex vivo, such as cells explanted from an individual patient (*e.g.,* lymphocytes, bone marrow aspirates, tissue biopsy) or universal donor hematopoietic stem cells, followed by reimplantation of the cells into a patient, usually after selection for cells which have incorporated the vector.

In some embodiments, in addition to the bidirectional nucleic acid constructs disclosed herein, the vector system may further comprise nucleic acids that encode a nuclease. In some embodiments, in addition to the bidirectional nucleic acid constructs disclosed herein, the vector system may further comprise nucleic acids that encode guide RNAs and/or nucleic acid encoding an RNA-guided DNA-binding agent, which can be a Cas protein such as Cas9. In some embodiments, a nucleic acid encoding a guide RNA and/or a nucleic acid encoding an RNA-guided DNA-binding agent or nuclease are each or both on a separate vector from a vector that comprises the bidirectional constructs disclosed herein. In any of the embodiments, the vector system may include other sequences that include, but are not limited to, promoters, enhancers, regulatory sequences, as described herein. In some embodiments, a promoter within the vector system does not drive the expression of a transgene of the bidirectional construct. In some embodiments, the vector system comprises one or more nucleotide sequence(s) encoding a crRNA, a trRNA, or a crRNA and trRNA. In some embodiments, the vector comprises one or more nucleotide sequence(s) encoding a sgRNA and an mRNA encoding an RNA-guided DNA binding agent, which can be a Cas nuclease (*e.g*., Cas9). In some embodiments, the vector system comprises one or more nucleotide sequence(s) encoding a crRNA, a trRNA, and an mRNA encoding an RNA-guided DNA binding agent, which can be a Cas nuclease, such as, Cas9. In some embodiments, the Cas9 is from *Streptococcus pyogenes* (*i.e.,* Spy Cas9). In some embodiments, the nucleotide sequence encoding the crRNA, trRNA, or crRNA and trRNA (which may be a sgRNA) comprises or consists of a guide sequence flanked by all or a portion of a repeat sequence from a naturally-occurring CRISPR/Cas system. The vector system may comprise a nucleic acid comprising or consisting of the crRNA, trRNA, or crRNA and trRNA, wherein the vector system comprises or consists of nucleic acids that are not naturally found together with the crRNA, trRNA, or crRNA and trRNA. Any of the vectors described herein may be delivered by liposome, a nanoparticle, an exosome, a microvesicle, and/or lipid nanoparticles (LNP). One or more guide RNA, RNA-binding DNA binding agent (*e.g*. mRNA), or donor construct comprising a sequence encoding a heterologous protein, individually or in any combination, may be delivered by liposome, a nanoparticle, an exosome, or a microvesicle. One or more guide RNA, RNA-binding DNA binding agent (*e.g*. mRNA), or donor construct comprising a sequence encoding a heterologous protein, individually or in any combination, may be delivered by LNP. Any of the LNPs and LNP formulations described herein are suitable for delivery of the guides

Lipid nanoparticles (LNPs) are a well-known means for delivery of nucleotide and protein cargo, and may be used for delivery of the bidirectional nucleic acid constructs disclosed herein. In some embodiments, LNPs may be used to deliver components of a gene editing system. In some embodiments, the LNPs deliver nucleic acid (*e.g.,* DNA or RNA), protein (*e.g*., RNA-guided DNA binding agent), or nucleic acid together with protein.

In some embodiments, provided herein is a method for delivering the bidirectional nucleic acid construct disclosed herein to a host cell or subject, wherein the construct is delivered via an LNP. In some embodiments, provided herein is a method for delivering the bidirectional nucleic acid construct disclosed herein to a host cell or subject, wherein one or more components of a gene editing system, such as a CRISPR/Cas nuclease system are delivered via an LNP. In some embodiments, the LNPs comprise a bidirectional construct and/or one or more components of a gene editing system (*e.g.,* guide RNA and/or RNA-guided DNA binding agent or an mRNA encoding RNA-guided DNA binding agent).

In some embodiments, provided herein is a composition comprising the bidirectional nucleic acid construct disclosed herein and an LNP. In some embodiments, the composition further comprises components of a gene editing system (*e.g*., guide RNA and/or an RNA-guided DNA binding agent such as Cas9 or a vector system capable of encoding the same). In some embodiments, a composition comprising the bidirectional nucleic acid construct disclosed herein and an LNP comprising a guide RNA and/or an mRNA encoding an RNA-guided DNA binding agent such as Cas9 is provided herein.

In some embodiments, the LNPs comprise biodegradable, ionizable lipids. In some embodiments, the LNPs comprise (9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate) or another ionizable lipid. See, *e.g.,* lipids of PCT/US2018/053559 (filed September 28, 2018), WO/2017/173054, WO2015/095340, and WO2014/136086, as well as references provided therein. In some embodiments, the term cationic and ionizable in the context of LNP lipids is interchangeable, *e.g.*, wherein ionizable lipids are cationic depending on the pH.

Electroporation is a well-known means for delivery of cargo, and any electroporation methodology may be used for delivery of the bidirectional construct disclosed herein. In some embodiments, electroporation may be used to deliver the bidirectional construct disclosed herein, optionally with a guide RNA and/or an RNA-guided DNA binding agent (*e.g.,* Cas9) or an mRNA encoding an RNA-guided DNA binding agent (*e.g.,* Cas9) delivered by the same or different means.

In some embodiments, the present disclosure includes a method for delivering the bidirectional construct disclosed herein to a cell in vitro, wherein the bidirectional construct is delivered via an LNP. In some embodiments, the bidirectional construct is delivered by a non-LNP means, such as via an AAV system, and a guide RNA and/or an RNA-guided DNA binding agent (*e.g.,* Cas9) or an mRNA encoding an RNA-guided DNA binding agent (*e.g.,* Cas9) is delivered by an LNP.

In some embodiments, the bidirectional construct described herein, alone or part of a vector, is formulated in or administered via a lipid nanoparticle; see *e.g*., WO/2017/173054, the contents of which are hereby incorporated by reference in their entirety.

Any of the vectors described herein may be delivered by LNP. Any of the LNPs and LNP formulations described herein are suitable for delivery of the gRNAs, a Cas nuclease or an mRNA encoding a Cas nuclease, combinations therof, and/or the bidirectional construct disclosed herein. In some embodiments, an LNP composition is encompassed comprising: an RNA component and a lipid component, wherein the lipid component comprises an amine lipid, such as a biodegradable, ionizable lipid; and wherein the RNA component comprises a guide RNA and/or an mRNA encoding a Cas nuclease.

In some instances, the lipid component comprises a biodegradable, ionizable lipid, cholesterol, DSPC, and PEG-DMG.

It will be apparent that components of the gene editing system (*e.g*., guide RNA and/or RNA-guided DNA binding agent) and bidirectional constructs can be delivered using the same or different systems. For example, the guide RNA, RNA-guided DNA binding agent sequence, and bidirectional construct can be carried by the same vector (*e.g*., AAV vector) or be formulated in one or more LNP compositions. Alternatively, the RNA-guided DNA binding agent (as a protein or mRNA) and/or gRNA can be carried by or associated with a LNP, while the bidirectional constructs can be carried by a vector, or vice versa. Furthermore, the different delivery systems can be administered by the same or different routes.

The different delivery systems can be delivered in vitro or in vivo simultaneously or in any sequential order. In some embodiments, the bidirectional construct, guide RNA, and RNA-guided DNA binding agent can be delivered in vitro or in vivo simultaneously, *e.g*., in one vector, two vectors, individual vectors, one LNP, two LNPs, individual LNPs, or a combination thereof. In some embodiments, the bidirectional construct can be delivered in vivo or in vitro, as a vector and/or associated with a LNP, prior to (*e.g.*, about 1, 2, 3, 4, 5, 6, 7, 8, 9, 10, 11, 12, 13, 14, or more days) delivering the guide RNA and/or RNA-guided DNA binding agent, as a vector and/or associated with a LNP singly or together as a ribonucleoprotein (RNP). In some embodiments, the donor construct can be delivered in multiple administerations, *e.g*., every day, every two days, every three days, every four days, every week, every two weeks, every three weeks, or every four weeks. In some embodiments, the donor construct can be delivered at one-week intervals, *e.g*., at week 1, week 2, and week 3, etc. As a further example, the guide RNA and/or RNA-guided DNA binding agent, as a vector and/or associated with a LNP singly or together as a ribonucleoprotein (RNP), can be delivered in vivo or in vitro, prior to (*e.g.,* about 1, 2, 3, 4, 5, 6, 7, 8, 9, 10, 11, 12, 13, 14, or more days) delivering the bidirectional construct, as a vector and/or associated with a LNP. In some embodiments, the albumin guide RNA can be delivered in multiple administerations, *e.g*., every day, every two days, every three days, every four days, every week, every two weeks, every three weeks, or every four weeks. In some embodiments, the the albumin guide RNA can be delivered at one-week intervals, *e.g*., at week 1, week 2, and week 3, etc. In some embodiments, the Cas nuclease can be delivered in multiple administerations, *e.g*., can be delivered every day, every two days, every three days, every four days, every week, every two weeks, every three weeks, or every four weeks. In some embodiments, the Cas nuclease can be delivered at one-week intervals, *e.g*., at week 1, week 2, and week 3, etc.

### V. Methods of Use

The present disclosure provides methods of using the bidirectional nucleic acid construct described herein in various applications. In some embodiments, the methods of using the bidirectional nucleic acid construct described herein in various applications include the use of a gene editing system such as the CRISPR/Cas system, as described herein.

In some embodiments, provided herein is an in vitro or in vivo method of modifying a target locus (*e.g*., inserting a transgene at a target site within a locus) comprising administering or delivering to a host cell a bidirectional nucleic acid construct described herein, a guide RNA, and an RNA-guided DNA binding agent as described herein (*e.g.,* a Cas nuclease such as Cas9). In some embodiments, provided herein is an in vitro or in vivo method of modifying a target locus comprising cleaving a target sequence in a host cell and inserting a bidirectional nucleic acid construct described herein, optionally utilizing a guide RNA and an RNA-guided DNA binding agent as described herein (*e.g.,* a Cas nuclease such as Cas9) for the cleaving step.

In some embodiments, provided herein is an in vitro or in vivo method of introducing a construct into a host cell comprising administering or delivering to a host cell a bidirectional nucleic acid construct described herein, a guide RNA, and an RNA-guided DNA binding agent as described herein (*e.g.,* a Cas nuclease such as Cas9). In some embodiments, provided herein is an in vitro or in vivo method of introducing a construct into a host cell comprising administering or delivering to a host cell a bidirectional nucleic acid construct described herein, and a gene editing system such as a ZFN, TALEN, or CRISPR/Cas9 system.

In some embodiments, provided herein is an in vitro or in vivo method of increasing expression of a polypeptide in a host cell comprising administering or delivering to a host cell a bidirectional nucleic acid construct described herein, a guide RNA, and an RNA-guided DNA binding agent as described herein (*e.g.,* a Cas nuclease such as Cas9). In some embodiments, provided herein is an in vitro or in vivo method of increasing expression of a polypeptide in a host cell, comprising administering or delivering to a host cell a bidirectional nucleic acid construct described herein, and a gene editing system such as a ZFN, TALEN, or CRISPR/Cas9 system. The polypeptide may be extracellular.

The bidirectional construct may be administered via a vector such as a nucleic acid vector. The guide RNA and RNA-guided DNA binding agent, can be administered individually, or in any combination, e.g. via an LNP comprising a guide RNA and an mRNA encoding the RNA-guided DNA binding agent. Administration and delivery to a host cell can be effected by any of the delivery methods described herein.

In some embodiments, provided herein is an in vitro or in vivo method of expressing a polypeptide encoded by a transgene at a target locus comprising administering or delivering to a host cell a bidirectional nucleic acid construct described herein, a guide RNA, and an RNA-guided DNA binding agent as described herein (*e.g.,* a Cas nuclease such as Cas9). In some embodiments, provided herein is an in vitro or in vivo method of expressing a polypeptide encoded by a transgene at a target locus comprising administering or delivering to a host cell a bidirectional nucleic acid construct described herein, and a gene editing system such as a ZFN, TALEN, or CRISPR/Cas9 system. In some embodiments, a method of making a host cell for expressing a polypeptide comprises administering or delivering to a host cell a bidirectional nucleic acid construct described herein, and a gene editing system such as a ZFN, TALEN, or CRISPR/Cas9 system.

The bidirectional construct, guide RNA, and RNA-guided DNA binding agent, for example, can be administered individually, or in any combination, as described herein. In some embodiments, the bidirectional construct, guide RNA, and RNA-guided DNA binding agent can be delivered simultaneously or sequentially, *e.g*., in one vector, two vectors, individual vectors, one LNP, two LNPs, individual LNPs, or a combination thereof. Administration and delivery to a host cell can be effected by any of the delivery methods described herein.

In addition, in some embodiments, the methods involve insertion in to the albumin locus, such as albumin intron 1, for example using a guide RNA comprising a sequence selected from any of Tables 5, 6, 7, 8, 9, and 10. In certain embodiments involving insertion into the albumin locus, the individual's circulating albumin levels are normal. The method may comprise maintaining the individual's circulating albumin levels within ±5, ±10, ±15, ±20, or ±50% of normal circulating albumin levels. In certain embodiments, the individual's albumin levels are unchanged as compared to the albumin levels of untreated individuals by at least week 4, week 8, week 12, or week 20. In certain embodiments, the individual's albumin levels transiently drop then return to normal levels. In particular, the methods may comprise detecting no significant alterations in levels of plasma albumin.

In some embodiments, the invention comprises a method or use of modifying (*e.g.*, creating a double strand break in) an albumin gene, such as a human albumin gene, comprising, administering or delivering to a host cell or population of host cells any one or more of the gRNAs, donor construct (*e.g.,* bidirectional construct comprising a sequence encoding Factor IX), and RNA-guided DNA binding agents (*e.g.,* Cas nuclease) described herein. In some embodiments, the invention comprises a method or use of modifying (*e.g*., creating a double strand break in) an albumin intron 1 region, such as a human albumin intron 1, comprising, administering or delivering to a host cell or population of host cells any one or more of the gRNAs, donor construct (*e.g*., bidirectional construct comprising a nucleic acid encoding a heterologous polypeptide), and RNA-guided DNA binding agents (*e.g.,* Cas nuclease or nucleic acid encoding a Cas nuclease) described herein. In some embodiments, the invention comprises a method or use of modifying (*e.g*., creating a double strand break in) a human safe harbor, such as liver tissue or hepatocyte host cell, comprising, administering or delivering to a host cell or population of host cells any one or more of the gRNAs, donor construct (*e.g.*, bidirectional construct comprising a sequence encoding a heterologous polypeptide), and RNA-guided DNA binding agents (*e.g*., Cas nuclease or nucleic acid encoding a Cas nuclease) described herein.

Insertion and/or expression of a transgene may be at its cognate locus, (*e.g*., insertion of a wild type transgene into the endogenous locus) or into a non-cognate locus (*e.g.,* safe harbor locus, such as albumin) as described herein.

In some embodiments, the host cell is a non-dividing cell type. As used herein, a "non-dividing cell" refers to cells that are terminally differentiated and do not divide, as well as quiescent cells that do not divide but retain the ability to re-enter cell division and proliferation. Liver cells, for example, retain the ability to divide (*e.g.,* when injured or resected), but do not typically divide. During mitotic cell division, homologous recombination is a mechanism by which the genome is protected and double-stranded breaks are repaired. In some embodiments, a "non-dividing" cell refers to a cell in which homologous recombination (HR) is not the primary mechanism by which double-stranded DNA breaks are repaired in the cell, *e.g.,* as compared to a control dividing cell. In some embodiments, a "non-dividing" cell refers to a cell in which non-homologous end joining (NHEJ) is the primary mechanism by which double-stranded DNA breaks are repaired in the cell, *e.g.,* as compared to a control dividing cell. Non-dividing cell types have been described in the literature, e.g. by active NHEJ double-stranded DNA break repair mechanisms. See, *e.g.* Iyama, DNA Repair (Amst.) 2013, 12(8): 620-636. In some embodiments, the host cell includes, but is not limited to, a liver cell, a muscle cell, or a neuronal cell. In some embodiments, the host cell is a hepatocyte, such as a mouse, cyno, or human hepatocyte. In some embodiments, the host cell is a myocyte, such as a mouse, cyno, or human myocyte. In some embodiments, provided herein is a host cell, described above, that comprises the bidirectional construct disclosed herein. In some embodiments the host cell expresses the transgene polypeptide encoded by the bidirectional construct disclosed herein. In some embodiments, provided herein is a host cell made by a method disclosed herein. In certain embodiments, the host cell is made by administering or delivering to a host cell a bidirectional nucleic acid construct described herein, and a gene editing system such as a ZFN, TALEN, or CRISPR/Cas9 system.

A method of expressing a polypeptide from the bidirectional construct described herein is also provided. Similarly a host cell comprising the bidirectional construct described herein can express a polypeptide encoded by the construct. In some embodiments, the polypeptide is a secreted polypeptide. In some embodiments, the polypeptide is one in which its function is normally effected (*e.g*., functionally active) as a secreted polypeptide. A "secreted polypeptide" as used herein refers to a protein that is secreted by the cell. In some embodiments, the polypeptide is an intracellular polypeptide. In some embodiments, the polypeptide is one in which its function is normally effected (*e.g.,* functionally active) inside a cell. An "intracellular polypeptide" as used herein refers to a protein that is not secreted by the cell, including soluble cytosolic polypeptides. In some embodiments, the polypeptide is a wild-type polypeptide. In some embodiments, the polypeptide is a mutant polypeptide (*e.g*., a hyperactive mutant of a wild-type polypeptide). In some embodiments, the polypeptide is a liver protein. In some embodiments, the polypeptide is a non-liver protein. In some embodiments, the polypeptide includes, but is not limited to, Factor IX and variants thereof. In some embodiments, the liver polypeptide is, for example, a polypeptide to address a liver disorder such as, without limitation, tyrosinemia, Wilson's disease, Tay-Sachs disease, hyperbilirubinema (Crigler-Najjar), acute intermittent porphyria, citrullinemia type 1, progressive familiar intrahepatic cholestasis, or maple syrup urine disease.

In some embodiments, the method further comprises achieving a durable effect, *e.g.* at least 1 month, 2 months, 6 months, 1 year, or 2 year effect. In some embodiments, the method further comprises achieving the therapeutic effect in a durable and sustained manner, *e.g.* at least 1 month, 2 months, 6 months, 1 year, or 2 year effect. In some embodiments, the level of heterologous polypeptide activity and/or level is stable for at least 1 month, 2 months, 6 months, 1 year, or more. In some embodiments a steady-state activity and/or level of the polypeptide is achieved by at least 7 days, at least 14 days, or at least 28 days. In additional embodiments, the method comprises maintaining the heterologous polypeptide activity and/or protein leves after a single dose of bidirectional construct for at least 1, 2, 4, or 6 months, or at least 1, 2, 3, 4, or 5 years.

In some embodiments, expression of the polypeptide by the host cell (whether in vitro or in vivo) is increased by at least 2%, 3%, 4%, 5%, 6%, 7%, 8%, 9%, 10%, 15%, 20%, 25%, 30%, 35%, 40%, 45%, 50%, 55%, 60%, 65%, 70%, or more relative to a level expressed by a host cell control that was not administered the construct comprising the transgene. In some embodiments, expression of the polypeptide by the host cell (whether in vitro or in vivo) is increased to at least 2%, 3%, 4%, 5%, 6%, 7%, 8%, 9%, 10%, 15%, 20%, 25%, 30%, 35%, 40%, 45%, 50%, 55%, 60%, 65%, 70%, 75%, or more, of a known normal level (*e.g.,* a level of a polypeptide in a healthy subject). In some embodiments, expression of the polypeptide by the host cell (whether in vitro or in vivo) is increased to at least about 1 µg/ml, 2 µg/ml, 3 µg/ml, 4 µg/ml, 5 µg/ml, 6 µg/ml, 7 µg/ml, 8 µg/ml, 9 µg/ml, 10 µg/ml, 15 µg/ml, 20 µg/ml, 25 µg/ml, 30 µg/ml, 35 µg/ml, 40 µg/ml, 45 µg/ml, 50 µg/ml, 55 µg/ml, 60 µg/ml, 65 µg/ml, 70 µg/ml, 75 µg/ml, 80 µg/ml, 85 µg/ml, 90 µg/ml, 95 µg/ml, 100 µg/ml, 120 µg/ml, 140 µg/ml, 160 µg/ml, 180 µg/ml, 200 µg/ml, 225 µg/ml, 250 µg/ml, 275 µg/ml, 300 µg/ml, 325 µg/ml, 350 µg/ml, 400 µg/ml, 450 µg/ml, 500 µg/ml, 550 µg/ml, 600 µg/ml, 650 µg/ml, 700 µg/ml, 750 µg/ml, 800 µg/ml, 850 µg/ml, 900 µg/ml, 1000 µg/ml, 1100 µg/ml, 1200 µg/ml, 1300 µg/ml, 1400 µg/ml, 1500 µg/ml, 1600 µg/ml, 1700 µg/ml, 1800 µg/ml, 1900 µg/ml, 2000 µg/ml, or more, as determined, *e.g.,* in the cell, plasma, and/or serum of a subject.

In some embodiments, provided herein is a method of treating a liver-associated disorder according to the methods described herein. As used herein, a "liver-associated disorder" refers to disorders that cause damage to the liver tissue directly, disorders that result from damage to the liver tissue, and/or disorders of non-liver organs or tissue that resulted from a defect in the liver.

In some embodiments, the bidirectional construct, guide RNA, and RNA-guided DNA binding agent are administered individually or in any combination locally or systemically, *e.g*. intravenously. In some embodiments, the bidirectional construct, guide RNA, and RNA-guided DNA binding agent are administered individually or in any combination into the hepatic circulation.

In some embodiments, the host or subject is a mammal. In some embodiments, the host or subject is a human. In some embodiments, the host or subject is a primate. In some embodiments, the host or subject is a rodent (*e.g*., mouse, rat), cow, pig, monkey, sheep, dog, cat, fish, or poultry.

This description and exemplary embodiments should not be taken as limiting. For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

### EXAMPLES

The following examples are provided to illustrate certain disclosed embodiments and are not to be construed as limiting the scope of this disclosure in any way.

### Example 1 - Materials and Methods

### Cloning and plasmid preparation

A bidirectional insertion construct flanked by ITRs was synthesized and cloned into pUC57-Kan by a commercial vendor. The resulting construct (P00147) was used as the parental cloning vector for other vectors. The other insertion constructs (without ITRs) were also commercially synthesized and cloned into pUC57. Purified plasmid was digested with BglII restriction enzyme (New England BioLabs, cat# R0144S), and the insertion constructs were cloned into the parental vector. Plasmid was propagated in Stbl3^{™} Chemically Competent *E. coli* (Thermo Fisher, Cat# C737303).

### AAV production

Triple transfection in HEK293 cells was used to package genomes with constructs of interest for AAV8 and AAVDJ production and resulting vectors were purified from both lysed cells and culture media through iodixanol gradient ultracentrifugation method (See, *e.g.,* Lock et al., Hum Gene Ther. 2010 Oct;21(10):1259-71). The plasmids used in the triple transfection that contained the genome with constructs of interest are referenced in the Examples by a "PXXXX" number, see also *e.g.,* Table 11. Isolated AAV was dialyzed in storage buffer (PBS with 0.001% Pluronic F68). AAV titer was determined by qPCR using primers/probe located within the ITR region.

### In vitro transcription ("IVT") of nuclease mRNA

Capped and polyadenylated *Streptococcus pyogenes* ("Spy") Cas9 mRNA containing N1-methyl pseudo-U was generated by *in vitro* transcription using a linearized plasmid DNA template and T7 RNA polymerase. Generally, plasmid DNA containing a T7 promoter and a 100 nt poly (A/T) region was linearized by incubating at 37°C with XbaI to complete digestion followed by heat inactivation of XbaI at 65°C. The linearized plasmid was purified from enzyme and buffer salts. The IVT reaction to generate Cas9 modified mRNA was incubated at 37°C for 4 hours in the following conditions: 50 ng/µL linearized plasmid; 2 mM each of GTP, ATP, CTP, and N1-methyl pseudo-UTP (Trilink); 10 mM ARCA (Trilink); 5 U/µL T7 RNA polymerase (NEB); 1 U/µL Murine Rnase inhibitor (NEB); 0.004 U/µL Inorganic *E. coli* pyrophosphatase (NEB); and 1x reaction buffer. TURBO Dnase (ThermoFisher) was added to a final concentration of 0.01 U/µL, and the reaction was incubated for an additional 30 minutes to remove the DNA template. The Cas9 mRNA was purified using a MegaClear Transcription Clean-up kit according to the manufacturer's protocol (ThermoFisher). Alternatively, the Cas9 mRNA was purified using LiCl precipitation, ammonium acetate precipitation, and sodium acetate precipitation or using a LiCl precipitation method followed by further purification by tangential flow filtration. The transcript concentration was determined by measuring the light absorbance at 260 nm (Nanodrop), and the transcript was analyzed by capillary electrophoresis by Bioanlayzer (Agilent).

Cas9 mRNAs below comprise Cas9 ORF SEQ ID NO: 703 or SEQ ID NO: 704 or a sequence of Table 24 of PCT/US2019/053423 (which is hereby incorporated by reference).

### Lipid formulations for delivery of Cas9 mRNA and gRNA

Cas9 mRNA and gRNA were delivered to cells and animals utilizing lipid formulations comprising ionizable lipid ((9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate), cholesterol, DSPC, and PEG2k-DMG.

For experiments utilizing pre-mixed lipid formulations (referred to herein as "lipid packets"), the components were reconstituted in 100% ethanol at a molar ratio of ionizable lipid:cholesterol:DSPC:PEG2k-DMG of 50:38:9:3, prior to being mixed with RNA cargos (*e.g.,* Cas9 mRNA and gRNA) at a lipid amine to RNA phosphate (N:P) molar ratio of about 6.0, as further described herein.

For experiments utilizing the components formulated as lipid nanoparticles (LNPs), the components were dissolved in 100% ethanol at various molar ratios. The RNA cargos (*e.g.,* Cas9 mRNA and gRNA) were dissolved in 25 mM citrate, 100 mM NaCl, pH 5.0, resulting in a concentration of RNA cargo of approximately 0.45 mg/mL.

For the experiments described in Example 2, the LNPs were formed by microfluidic mixing of the lipid and RNA solutions using a Precision Nanosystems NanoAssemblr^{™} Benchtop Instrument, according to the manufacturer's protocol. A 2:1 ratio of aqueous to organic solvent was maintained during mixing using differential flow rates. After mixing, the LNPs were collected, diluted in water (approximately 1:1 v/v), held for 1 hour at room temperature, and further diluted with water (approximately 1: 1 v/v) before final buffer exchange. The final buffer exchange into 50 mM Tris, 45 mM NaCl, 5% (w/v) sucrose, pH 7.5 (TSS) was completed with PD-10 desalting columns (GE). If required, formulations were concentrated by centrifugation with Amicon 100 kDa centrifugal filters (Millipore). The resulting mixture was then filtered using a 0.2 µm sterile filter. The final LNP was stored at -80 °C until further use. The LNPs were formulated at a molar ratio of ionizable lipid:cholesterol:DSPC:PEG2k-DMG of 45:44:9:2, with a lipid amine to RNA phosphate (N:P) molar ratio of about 4.5, and a ratio of gRNA to mRNA of 1:1 by weight.

For the experiments described in other examples, the LNPs were prepared using a cross-flow technique utilizing impinging jet mixing of the lipid in ethanol with two volumes of RNA solutions and one volume of water. The lipid in ethanol was mixed through a mixing cross with the two volumes of RNA solution. A fourth stream of water was mixed with the outlet stream of the cross through an inline tee (*See* WO2016010840 Fig. 2.). The LNPs were held for 1 hour at room temperature, and further diluted with water (approximately 1:1 v/v). Diluted LNPs were concentrated using tangential flow filtration on a flat sheet cartridge (Sartorius, 100kD MWCO) and then buffer exchanged by diafiltration into 50 mM Tris, 45 mM NaCl, 5% (w/v) sucrose, pH 7.5 (TSS). Alternatively, the final buffer exchange into TSS was completed with PD-10 desalting columns (GE). If required, formulations were concentrated by centrifugation with Amicon 100 kDa centrifugal filters (Millipore). The resulting mixture was then filtered using a 0.2 µm sterile filter. The final LNP was stored at 4°C or -80°C until further use. The LNPs were formulated at a molar ratio of ionizable lipid:cholesterol:DSPC:PEG2k-DMG of 50:38:9:3, with a lipid amine to RNA phosphate (N:P) molar ratio of about 6.0, and a ratio of gRNA to mRNA of 1:1 by weight.

### Cell culture and in vitro delivery of Cas9 mRNA, gRNA, and insertion constructs Hepa1-6 cells

Hepa 1-6 cells were plated at density of 10,000 cells/well in 96-well plates. 24 hours later, cells were treated with LNP and AAV. Before treatment the media was aspirated off from the wells. LNP was diluted to 4ng/ul in DMEM+10% FBS media and further diluted to 2ng/ul in 10% FBS (in DMEM) and incubated at 37°C for 10 min (at a final concentration of 5% FBS). Target MOI of AAV was 1e6, diluted in DMEM+10% FBS media. 50 µl of the above diluted LNP at 2ng/ul was added to the cells (delivering a total of 100 ng of RNA cargo) followed by 50 µl of AAV. The treatment of LNP and AAV were minutes apart. Total volume of media in cells was 100 µl. After 72 hours post-treatment and 30 days post-treatment, supernatant from these treated cells were collected for human FIX ELISA analysis as described below.

### Primary Hepatocytes

Primary mouse hepatocytes (PMH), primary cyno hepatocytes (PCH) and primary human hepatocytes (PHH) were thawed and resuspended in hepatocyte thawing medium with supplements (ThermoFisher) followed by centrifugation. The supernatant was discarded, and the pelleted cells resuspended in hepatocyte plating medium plus supplement pack (ThermoFisher). Cells were counted and plated on Bio-coat collagen I coated 96-well plates at a density of 33,000 cells/well for PHH and 50,000 cells/well for PCH and 15,000 cells/well for PMH. Plated cells were allowed to settle and adhere for 5 hours in a tissue culture incubator at 37°C and 5% CO₂ atmosphere. After incubation cells were checked for monolayer formation and were washed thrice with hepatocyte maintenance prior and incubated at 37°C.

For experiments utilizing lipid packet delivery, Cas9 mRNA and gRNA were each separately diluted to 2mg/ml in maintenance media and 2.9 µl of each were added to wells (in a 96-well Eppendorf plate) containing 12.5 µl of 50mM sodium citrate, 200mM sodium chloride at pH 5 and 6.9 µl of water. 12.5 µl of lipid packet formulation was then added, followed by 12.5 µl of water and 150 µl of TSS. Each well was diluted to 20 ng/µl (with respect to total RNA content) using hepatocyte maintenance media, and then diluted to 10 ng/µl (with respect to total RNA content) with 6% fresh mouse serum. Media was aspirated from the cells prior to transfection and 40 µl of the lipid packet/RNA mixtures were added to the cells, followed by addition of AAV (diluted in maintenance media) at an MOI of 1e5. Media was collected 72 hours post-treatment for analysis and cells were harvested for further analysis, as described herein.

### Luciferase assays

For experiments involving NanoLuc detection in cell media, one volume of Nano-Glo^{®} Luciferase Assay Substrate was combined with 50 volumes of Nano-Glo^{®} Luciferase Assay Buffer. The assay was run on a Promega Glomax runner at an integration time of 0.5 sec using 1:10 dilution of samples (50 µl of reagent + 40 µl water + 10 µl cell media).

For experiments involving detection of the HiBit tag in cell media, LgBiT Protein and Nano-GloR HiBiT Extracellular Substrate were diluted 1:100 and 1:50, respectively, in room temperature Nano-GloR HiBiT Extracellular Buffer. The assay was run on a Promega Glomax runner at an integration time of 1.0 sec using 1:10 dilution of samples (50 µl of reagent + 40 µl water + 10 µl cell media).

### In vivo delivery of LNP and/or AAV

Mice were dosed with AAV, LNP, both AAV and LNP, or vehicle (PBS + 0.001% Pluronic for AAV vehicle, TSS for LNP vehicle) via the lateral tail vein. AAV were administered in a volume of 0.1 mL per animal with amounts (vector genomes/mouse, "vg/ms") as described herein. LNPs were diluted in TSS and administered at amounts as indicated herein, at about 5 µl/gram body weight. Typically, mice were injected first with AAV and then with LNP, if applicable. At various times points post-treatment, serum and/or liver tissue was collected for certain analyses as described further below.

### Human Factor IX (hFIX) ELISA analysis

For *in vitro* studies, total human Factor IX levels secreted in cell media were determined using a Human Factor IX ELISA Kit (Abcam, Cat# ab188393) according to manufacturer's protocol. Secreted hFIX levels were quantitated off a standard curve using 4 parameter logistic fit and expressed as ng/ml of media.

For *in vivo* studies, blood was collected and the serum was isolated as indicated. The total human Factor IX serum levels were determined using a Human Factor IX ELISA Kit (Abcam, Cat# ab188393) according to manufacturer's protocol. Serum hFIX levels were quantitated off a standard curve using 4 parameter logistic fit and expressed as µg/mL of serum.

### Next-generation sequencing ("NGS") and analysis for on-target cleavage efficiency

Deep sequencing was utilized to identify the presence of insertions and deletions introduced by gene editing, e.g., within intron 1 of albumin. PCR primers were designed around the target site and the genomic area of interest was amplified. Primer sequence design was done as is standard in the field.

Additional PCR was performed according to the manufacturer's protocols (Illumina) to add chemistry for sequencing. The amplicons were sequenced on an Illumina MiSeq instrument. The reads were aligned to the reference genome after eliminating those having low quality scores. The resulting files containing the reads were mapped to the reference genome (BAM files), where reads that overlapped the target region of interest were selected and the number of wild type reads versus the number of reads which contain an insertion or deletion ("indel") was calculated.

The editing percentage (*e.g.,* the "editing efficiency" or "percent editing") is defined as the total number of sequence reads with insertions or deletions ("indels") over the total number of sequence reads, including wild type.

### In situ hybridization analysis

BaseScope (ACDbio, Newark, CA) is a specialized RNA *in situ* hybridization technology that can provide specific detection of exon junctions, *e*.*g*., in a hybrid mRNA transcript that contains an insertion transgene (hFIX) and coding sequence from the site of insertion (exon 1 of albumin). BaseScope was used to measure the percentage of liver cells expressing the hybrid mRNA.

To detect the hybrid mRNA, two probes against the hybrid mRNAs that may arise following insertion of a bidirectional construct were designed by ACDbio (Newark, CA). One of the probes was designed to detect a hybrid mRNA resulting from insertion of the construct in one orientation, while the other probe was designed to detect a hybrid mRNA resulting from insertion of the construct in the other orientation. Livers from different groups of mice were collected and fresh-frozen sectioned. The BaseScope assay, using a single probe or pooled probes was performed according to the manufacture's protocol. Slides were scanned and analyzed by the HALO software. The background (saline treated group) of this assay was 0.58%.

### Example 2- in vitro testing of insertion templates with and without homology arms

In this Example, Hepa1-6 cells were cultured and treated with AAV harboring insertion templates of various forms (*e.g.,* having either a single-stranded genome ("ssAAV") or a self-complementary genome ("scAAV")), in the presence or absence of LNP delivering Cas9 mRNA and G000551 *e.g.,* as described in Example 1 (n=3). The AAV and LNP were prepared as described in Example 1. Following treatment, the media was collected for transgene expression (*e.g.,* human Factor IX levels) as described in Example 1.

Hepa1-6 cells are an immortalized mouse liver cell line that continues to divide in culture. As shown in Fig. 2 (72 hour post-treatment time point), only the vector (scAAV derived from plasmid P00204) comprising 200 bp homology arms resulted in detectable expression of hFIX. Use of the AAV vectors derived from P00123 (scAAV lacking homology arms) and P00147 (ssAAV bidirectional construct lacking homology arms) did not result in any detectable expression of hFIX in this experiment. The cells were kept in culture and these results were confirmed when re-assayed at 30 days post-treatment (data not shown).

### Example 3- in vivo testing of insertion templates with and without homology arms

In this Example, mice were treated with AAV derived from the same plasmids (P00123, P00204, and P00147) as tested *in vitro* in Example 2. The dosing materials were prepared and dosed as described in Example 1. C57Bl/6 mice were dosed (n=5 for each group) with 3e11 vector genomes each (vg/ms) followed by LNP comprising G000551 ("G551") at a dose of 4 mg/kg (with respect to total RNA cargo content). Four weeks post dose, the animals were euthanized and liver tissue and sera were collected for editing and transgene *(*e.g., hFIX) expression, respectively.

As shown in Fig. 3A and Table 12, liver editing levels of ~60% were detected in each group of animals treated with LNP comprising gRNA targeting intron 1 of murine albumin. However, despite robust and consistent levels of editing in each treatment group, animals receiving the ssAAV vector without homology arms (ssAAV vector derived from P00147) in combination with LNP treatment resulted in the highest level of hFIX expression in serum (Fig. 3B and Table 13).

**Table 12: % Indel**

| Template | Average Indel (%) | St.Dev Indel (%) |
|---|---|---|
| scAAV Blunt (P00123) | 66.72 | 4.09 |
| ssAAV Blunt (P00147) | 68.10 | 2.27 |
| ssAAV HR (P00204) | 70.16 | 3.68 |
| LNP only | 68.24 | 6.47 |
| Vehicle | 0.28 | 0.08 |

**Table 13: Factor IX Levels**

| Template | Average Factor IX (ug/mL) | St.Dev Factor IX (ug/mL) |
|---|---|---|
| scAAV Blunt (P00123) | 0.75 | 0.28 |
| ssAAV Blunt (P00147) | 2.92 | 1.04 |
| ssAAV HR (P00204) | 0.96 | 0.35 |
| LNP only | 0 | 0 |
| Vehicle | 0 | 0 |

### Example 4- in vivo testing of ssAAV insertion templates with and without homology arms

The experiment described in this Example examined the effect of incorporating homology arms into ssAAV vectors *in vivo.*

The dosing materials used in this experiment were prepared and dosed as described in Example 1. C57Bl/6 mice were dosed (n=5 for each group) with 3e11 vg/ms followed by LNP comprising G000666 ("G666") or G000551 ("G551") at a dose of 0.5 mg/kg (with respect to total RNA cargo content). Four weeks post dose, the animals sera was collected for transgene (*e.g.,* hFIX) expression.

As shown in Fig. 4A and Table 14, use of the ssAAV vectors with asymmetrical homology arms (300/600bp arms, 300/2000bp arms, and 300/1500bp arms for vectors derived from plasmids P00350, P00356, and P00362, respectively) for insertion into the site targeted by G551 resulted in levels of circulating hFIX that were below the lower limit of detection for the assay. However, use of the ssAAV vector (derived from P00147) without homology arms and having two hFIX open reading frames (ORF) in a bidirectional orientation resulted in detectable levels of circulating hFIX in each animal.

Similarly, use of the ssAAV vectors with symmetrical homology arms (500bp arms and 800bp arms for vectors derived from plasmids P00353 and P00354, respectively) for insertion into the site targeted by G666 resulted in lower but detectable levels, as compared to use of the bidirectional vector without homology arms (derived from P00147) (see Fig. 4B and Table 15).

**Table 14: Serum FIX Levels**

| AAV | Average Serum FIX (ug/mL) | St.Dev Serum FIX (ug/mL) |
|---|---|---|
| P00147 | 5.13 | 1.31 |
| P00350 | -0.22 | 0.08 |
| P00356 | -0.23 | 0.04 |
| P00362 | -0.09 | 0.16 |

**Table 15: Serum FIX Levels**

| AAV | Average Serum FIX (ug/mL) | St.Dev Serum FIX (ug/mL) |
|---|---|---|
| P00147 | 7.72 | 4.67 |
| P00353 | 0.20 | 0.23 |
| P00354 | 0.46 | 0.26 |

### Example 5- in vitro screening of bidirectional constructs across target sites in primary mouse hepatocytes

Having demonstrated that bidirectional constructs lacking homology arms outperformed vectors with other configurations, the experiment described in this Example examined the effects of altering the modules of the bidirectional construct, here the ORF and the splice acceptors, and altering the gRNAs for targeting CRISPR/Cas9-mediated insertion. These varied bidirectional constructs were tested across a panel of target sites utilizing 20 different gRNAs targeting intron 1 of murine albumin in primary mouse hepatocytes (PMH).

The ssAAV and lipid packet delivery materials tested in this Example were prepared and delivered to PMH as described in Example 1, with the AAV at an MOI of 1e5. Following treatment, isolated genomic DNA and cell media was collected for editing and transgene expression analysis, respectively. Each of the vectors comprised a reporter that can be measured through luciferase-based fluorescence detection as described in Example 1, plotted in Fig. 5C as relative luciferase units ("RLU"). For example, the AAV vectors comprising the hFIX ORFs contained a HiBit peptide fused at their 3' ends, and the AAV vector comprising only reporter genes comprised a NanoLuc ORF (in addition to GFP). Schematics of each of the vectors tested are provided in Fig. 5A. The gRNAs tested are shown in Fig. 5B and 5C, using a shortened number for those listed in Table 4 *(e.g.,* where the leading zeros are omitted, for example where "G551" corresponds to "G000551" in Table 4).

As shown in Fig. 5B and Table 16, consistent but varied levels of editing were detected for each of the treatment groups across each combination tested. Transgene expression using various combinations of template and guide RNA is shown in Fig. 5C and Table 17. As shown in Fig. 5D, a significant level of indel formation did not necessarily result in more efficient expression of the transgenes. Using P00411- and P00418-derived templates, the R² values were 0.54 and 0.37, respectively, when guides with less than 10% editing are not included. The mouse albumin splice acceptor and human FIX splice acceptor each resulted in effective transgene expression. Interestingly, despite differing ORFs and splice acceptors, the relative levels of expression as measured in RLUs was consistent between the three vectors tested, demonstrating the robustness, reproducibility and modularity of the bidirectional construct system (see Fig. 5C).

**Table 16: % Indel**

| Guide ID | P00411 | | P00418 | | P00415 | |
|---|---|---|---|---|---|---|
| | Average Indel (%) | St.Dev Indel (%) | Average Indel (%) | St.Dev Indel (%) | Average Indel (%) | St.Dev Indel (%) |
| G000551 | 67.4 | 1.42 | 70.67 | 2.29 | 66.73 | 4.90 |
| G000552 | 90.93 | 0.15 | 91.10 | 2.43 | 90.37 | 1.01 |
| G000553 | 77.80 | 3.83 | 77.47 | 1.87 | 80.50 | 0.85 |
| G000554 | 72.37 | 6.49 | 70.53 | 3.16 | 70.60 | 2.91 |
| G000555 | 35.37 | 2.63 | 35.77 | 9.34 | 40.47 | 4.75 |
| G000666 | 62.47 | 3.87 | 50.90 | 19.41 | 65.90 | 3.99 |
| G000667 | 30.57 | 2.73 | 25.30 | 3.67 | 31.67 | 2.29 |
| G000668 | 63.60 | 2.02 | 66.65 | 4.60 | 68.30 | 4.90 |
| G000669 | 19.10 | 2.51 | 19.33 | 1.53 | 18.70 | 1.25 |
| G000670 | 47.80 | 3.27 | 49.10 | 4.42 | 51.97 | 2.06 |
| G011722 | 4.20 | 0.72 | 4.27 | 1.20 | 4.20 | 0.26 |
| G011723 | 5.63 | 1.27 | 6.07 | 0.15 | 5.93 | 0.15 |
| G011724 | 6.10 | 1.28 | 8.50 | 2.69 | 7.13 | 1.27 |
| G011725 | 1.93 | 0.29 | 2.60 | 0.79 | 2.53 | 0.65 |
| G011726 | 10.73 | 1.46 | 11.70 | 0.50 | 12.43 | 1.33 |
| G011727 | 14.20 | 1.56 | 14.80 | 2.36 | 16.20 | 2.69 |
| G011728 | 10.55 | 1.20 | 13.65 | 0.92 | 15.50 | 1.56 |
| G011729 | 5.00 | 0.10 | 5.63 | 0.25 | 6.00 | 1.01 |
| G011730 | 7.83 | 0.97 | 9.13 | 0.59 | 7.33 | 0.59 |
| G011731 | 23.70 | 0.66 | 25.27 | 1.21 | 24.87 | 1.01 |
| AAV Only | 0.15 | 0.07 | 0.05 | 0.07 | 0.10 | 0.00 |

**Table 17: Luciferase Levels**

| Guide ID | P00411 | | P00418 | | P00415 | |
|---|---|---|---|---|---|---|
| | Average Luciferase (RLU) | St.Dev Luciferase (RLU) | Average Luciferase (RLU) | St.Dev Luciferase (RLU) | Average Luciferase (RLU) | St.Dev Luciferase (RLU) |
| G000551 | 58000.00 | 4331.28 | 41800.00 | 2165.64 | 78633.33 | 20274.70 |
| G000552 | 95700.00 | 10573.08 | 80866.67 | 27911.35 | 205333.33 | 30664.86 |
| G000553 | 205333.33 | 52993.71 | 177333.33 | 32929.22 | 471666.67 | 134001.00 |
| G000554 | 125333.33 | 55949.38 | 91933.33 | 19194.10 | 232666.67 | 67002.49 |
| G000555 | 59933.33 | 11566.04 | 77733.33 | 11061.80 | 155666.67 | 15947.83 |
| G000666 | 88500.00 | 28735.87 | 93266.67 | 30861.19 | 313000.00 | 15394.80 |
| G000667 | 75333.33 | 22653.11 | 68966.67 | 27222.11 | 153000.00 | 30805.84 |
| G000668 | 164000.00 | 56320.51 | 133400.00 | 65111.29 | 429000.00 | 120751.80 |
| G000669 | 28933.33 | 11636.29 | 22033.33 | 2413.16 | 46466.67 | 6543.19 |
| G000670 | 162666.67 | 32959.57 | 200000.00 | 33867.39 | 424666.67 | 36473.73 |
| G011722 | 16766.67 | 3384.28 | 8583.33 | 4103.10 | 24000.00 | 8915.16 |
| G011723 | 22733.33 | 7252.82 | 17133.33 | 4905.44 | 26100.00 | 8109.87 |
| G011724 | 17300.00 | 2400.00 | 28033.33 | 9091.94 | 30933.33 | 3365.02 |
| G011725 | 8253.33 | 1163.20 | 8890.00 | 1429.27 | 20366.67 | 13955.05 |
| G011726 | 12223.33 | 3742.54 | 11610.00 | 2490.44 | 14950.00 | 8176.03 |
| G011727 | 35600.00 | 8128.35 | 36300.00 | 12301.22 | 86700.00 | 5023.94 |
| G011728 | 14900.00 | 5011.99 | 22466.67 | 7130.45 | 38166.67 | 13829.08 |
| G011729 | 10460.00 | 2543.95 | 11223.33 | 2220.28 | 26966.67 | 16085.50 |
| G011730 | 14833.33 | 2307.24 | 21700.00 | 8681.59 | 41233.33 | 25687.03 |
| G011731 | 16433.33 | 3274.65 | 22566.67 | 2205.30 | 20756.67 | 13096.20 |
| AAV Only | 217.00 | 15.56 | 215.00 | 15.56 | 207.00 | 1.41 |

### Example 6- in vivo screening of bidirectional constructs across target sites

The ssAAV and LNPs tested in this Example were prepared and delivered to C57B1/6 mice as described in Example 1 to assess the performance of the bidirectional constructs across target sites *in vivo.* Four weeks post dose, the animals were euthanized and liver tissue and sera were collected for editing and transgene (*e.g.,* hFIX) expression, respectively.

In an initial experiment, 10 different LNP formulations containing 10 different gRNA targeting intron 1 of albumin were delivered to mice along with ssAAV derived from P00147. The AAV and LNP were delivered at 3e11 vg/ms and 4 mg/kg (with respect to total RNA cargo content), respectively. The gRNAs tested in this experiment are shown in Fig. 6 and Table 18. As shown in Fig. 6 and as observed *in vitro,* a significant level of indel formation was not predictive for insertion or expression of the transgenes.

In a separate experiment, the full panel of 20 gRNAs targeting the 20 different target sites tested *in vitro* in Example 5 were tested *in vivo.* To this end, 20 LNP formulations containing the 20 gRNAs targeting intron 1 of albumin were delivered to mice along with ssAAV derived from P00147. The AAV and LNP were delivered at 3e11 vg/ms and 1 mg/kg (with respect to total RNA cargo content), respectively. The gRNAs tested in this experiment are shown in Fig. 7A and 7B and Tables 19 and 20, using a shortened number for those listed in Table 4.

As shown, in Fig. 7A, varied levels of editing were detected for each of the treatment groups across each LNP/vector combination tested. However, as shown in Fig. 7B and consistent with the *in vitro* data described in Example 5, higher levels of editing did not necessarily result in higher levels of expression of the transgenes *in vivo,* indicating a lack of correlation between editing and insertion/expression of the bidirectional constructs. Indeed, very little correlation exists between the amount of editing achieved and the amount of transgene (hFIX) expression as viewed in the plot provided in Fig. 7D. In particular, an R² value of only 0.34 is calculated between the editing and expression data sets for this experiment, when those gRNAs achieving less than 10% editing are removed from the analysis. Interestingly, as shown in Fig. 7C, a correlation plot is provided comparing the levels of expression as measured in RLU from the *in vitro* experiment of Example 5 to the transgene expression levels *in vivo* detected in this experiment, with an R² value of 0.70, demonstrating a positive correlation between the primary cell screening and the *in vivo* treatments.

To assess insertion of the bidirectional construct at the cellular level, liver tissues from treated animals were assayed using an *in situ* hybridization method (BaseScope), *e.g.,* as described in Example 1. This assay utilized probes that can detect the junctions between the hFIX transgene and the mouse albumin exon 1 sequence, as a hybrid transcript. As shown in Fig. 8A, cells positive for the hybrid transcript were detected in animals that received both AAV and LNP. Specifically, when AAV alone is administered, less than 1.0% of cells were positive for the hybrid transcript. With administration of LNPs comprising G011723, G000551, or G000666, 4.9%, 19.8%, or 52.3% of cells were positive for the hybrid transcript. Additionally, as shown in Fig. 8B and Table 14, circulating hFIX levels correlated with the number of cells that were positive for the hybrid transcript. Lastly, the assay utilized pooled probes that can detect insertion of the bidirectional construct in either orientation. However, when a single probe was used that only detects a single orientation, the amount of cells that were positive for the hybrid transcript was about half that detected using the pooled probes (in one example, 4.46% vs 9.68%), suggesting that the bidirectional construct indeed is capable of inserting in either orientation giving rise to expressed hybrid transcripts that correlate with the amount of transgene expression at the protein level.

**Table 18: Factor IX Levels and % Indel**

| Guide | Average Indel (%) | St. Dev Indel (%) | Average Luciferase (RLU) | St. Dev Luciferase (RLU) |
|---|---|---|---|---|
| G000551 | 75.02 | 1.27 | 3.82 | 3.38 |
| G000555 | 51.18 | 1.19 | 32.56 | 9.05 |
| G000553 | 62.78 | 2.64 | 25.07 | 4.04 |
| G000667 | 52.96 | 4.96 | 32.03 | 6.74 |
| G000554 | 55.24 | 2.28 | 29.48 | 7.34 |
| G000552 | 67.56 | 1.73 | 14.79 | 5.34 |
| G000668 | 43.14 | 5.78 | 26.72 | 7.97 |
| G000669 | 50.68 | 2.97 | 10.70 | 4.43 |
| G000666 | 64.62 | 1.34 | 26.19 | 5.56 |
| G000670 | 55.90 | 1.30 | 30.96 | 8.44 |

**Table 19: % Liver Editing**

| Guide | Average Liver Editing (%) | St. Dev Liver Editing (%) |
|---|---|---|
| G000551 | 59.48 | 4.02 |
| G000555 | 58.72 | 3.65 |
| G000553 | 51.26 | 2.81 |
| G000554 | 33.04 | 8.76 |
| G000555 | 12.72 | 4.46 |
| G000666 | 53.60 | 4.92 |
| G000667 | 26.74 | 4.98 |
| G000668 | 39.22 | 3.04 |
| G000669 | 33.34 | 4.77 |
| G000670 | 47.50 | 5.58 |
| G011722 | 10.34 | 1.68 |
| G011723 | 4.02 | 0.84 |
| G011724 | 2.46 | 0.64 |
| G011725 | 8.26 | 1.24 |
| G011726 | 6.90 | 1.01 |
| G011727 | 13.33 | 6.43 |
| G011728 | 35.78 | 9.34 |
| G011729 | 4.62 | 1.46 |
| G011730 | 12.68 | 3.14 |
| G011731 | 26.70 | 1.86 |

**Table 20: FIX Levels**

| Guide | Week 1 | | Week 2 | | Week 4 | |
|---|---|---|---|---|---|---|
| | Average FIX (ug/mL) | St.Dev FIX (ug/mL) | Average FIX (ug/mL) | St.Dev FIX (ug/mL) | Average FIX (ug/mL) | St.Dev FIX (ug/mL) |
| G000551 | 10.88 | 2.74 | 10.25 | 2.51 | 9.39 | 3.48 |
| G000555 | 13.34 | 2.09 | 12.00 | 2.75 | 12.43 | 2.57 |
| G000553 | 17.64 | 4.34 | 20.27 | 6.35 | 15.31 | 2.43 |
| G000554 | 12.79 | 4.99 | 14.29 | 6.09 | 12.74 | 4.93 |
| G000555 | 11.94 | 5.79 | 11.99 | 5.76 | 8.61 | 4.02 |
| G000666 | 21.63 | 1.32 | 20.65 | 1.55 | 17.23 | 0.62 |
| G000667 | 16.77 | 2.86 | 12.35 | 2.85 | 12.57 | 5.60 |
| G000668 | 21.35 | 1.51 | 18.20 | 3.18 | 17.72 | 2.25 |
| G000669 | 5.76 | 2.10 | 6.72 | 2.93 | 3.39 | 0.78 |
| G000670 | 18.18 | 2.17 | 19.16 | 3.05 | 15.49 | 3.61 |
| G011722 | 8.07 | 1.74 | 7.74 | 2.41 | 8.07 | 1.74 |
| G011723 | 2.11 | 0.28 | 1.65 | 0.28 | 2.11 | 0.28 |
| G011724 | 0.92 | 0.43 | 0.60 | 0.30 | 0.92 | 0.43 |
| G011725 | 1.75 | 0.77 | 1.14 | 0.67 | 1.75 | 0.77 |
| G011726 | 0.59 | 0.30 | 1.01 | 0.64 | 0.59 | 0.30 |
| G011727 | 6.71 | 2.80 | 6.90 | 3.68 | 6.71 | 2.80 |
| G011728 | 11.77 | 3.12 | 12.29 | 3.43 | 11.77 | 3.12 |
| G011729 | 0.94 | 0.35 | 0.89 | 0.29 | 0.94 | 0.35 |
| G011730 | 5.93 | 1.77 | 6.33 | 1.73 | 5.93 | 1.77 |
| G011731 | 3.56 | 0.87 | 3.78 | 0.50 | 3.56 | 0.87 |
| AAV Only | 0.00 | 0.00 | 0.00 | 0.00 | 0.00 | 0.00 |
| Vehicle | 0.00 | 0.00 | 0.00 | 0.00 | 0.00 | 0.00 |
| Human Serum | 3.63 | 0.32 | 3.61 | 0.35 | 3.28 | 0.03 |

### Example 7- durability of hFIX expression in vivo

The durability of hFIX expression over time in treated animals was assessed in this Example. To this end, hFIX was measured in the serum of treated animals post-dose, as part of a one-year durability study.

The ssAAV and LNPs tested in this Example were prepared and delivered to C57Bl/6 mice as described in Example 1. The LNP formulation contained G000551 and the ssAAV was derived from P00147. The AAV was delivered at 3e11 vg/ms and the LNP was delivered at either 0.25 or 1.0 mg/kg (with respect to total RNA cargo content) (n=5 for each group).

As shown in Fig. 9A and 9B and Tables 21 and 22, hFIX expression was sustained at each time point assessed for both groups out to 41 weeks or 52 weeks, respectively. A drop in the levels observed at 8 weeks in Fig. 9A is believed to be due to the variability of the ELISA assay. Serum albumin levels were measured by ELISA at week 2 and week 41, showing that circulating albumin levels are maintained across the study.

**Table 21: hFIX Levels**

| Week | Dose | | | |
|---|---|---|---|---|
| | 0.25 mpk LNP | | 1 mpk LNP | |
| | Average hFIX (ug/mL) | StDev hFIX (ug/mL) | Average hFIX (ug/mL) | StDev hFIX (ug/mL) |
| 2 | 0.48 | 0.21 | 2.24 | 1.12 |
| 4 | 0.55 | 0.18 | 2.82 | 1.67 |
| 8 | 0.40 | 0.17 | 1.72 | 0.77 |
| 12 | 0.48 | 0.20 | 2.85 | 1.34 |
| 20 | 0.48 | 0.27 | 2.45 | 1.26 |
| 41 | 0.79 | 0.49 | 4.63 | 0.95 |

**Table 22: hFIX Levels**

| Week | Dose | | | |
|---|---|---|---|---|
| | 0.25 mpk LNP | | 1 mpk LNP | |
| | Average hFIX (ug/mL) | StDev hFIX (ug/mL) | Average hFIX (ug/mL) | StDev hFIX (ug/mL) |
| 2 | 0.87 | 0.15 | 4.02 | 1.75 |
| 8 | 0.99 | 0.15 | 4.11 | 1.41 |
| 12 | 0.93 | 0.14 | 4.15 | 1.35 |
| 20 | 0.83 | 0.22 | 4.27 | 1.54 |
| 41 | 0.83 | 0.37 | 4.76 | 1.62 |
| 52 | 0.82 | 0.25 | 4.72 | 1.54 |

### Example 8 - effects of varied doses of AAV and LNP to modulate hFIX expression in vivo

In this Example, the effects of varying the dose of both AAV and LNP to modulate expression of hFIX was assessed in C57Bl/6 mice.

The ssAAV and LNPs tested in this Example were prepared and delivered to mice as described in Example 1. The LNP formulation contained G000553 and the ssAAV was derived from P00147. The AAV was delivered at 1e11, 3e11, 1e12 or 3e12 vg/ms and the LNP was delivered at 0.1, 0.3, or 1.0 mg/kg (with respect to total RNA cargo content) (n=5 for each group). Two weeks post-dose, the animals were euthanized. Sera were collected at two timepoints for hFIX expression analysis.

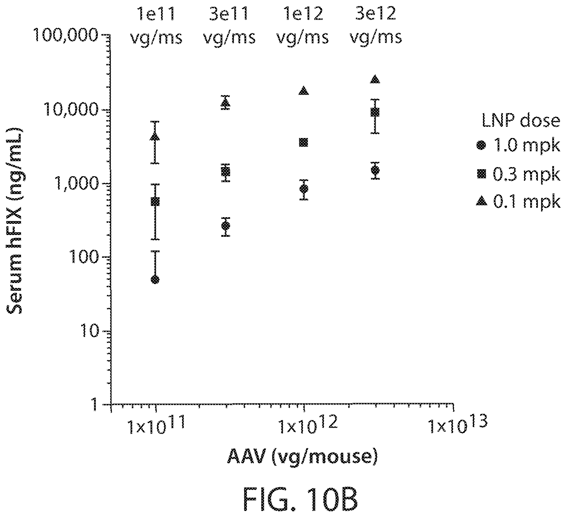

As shown in Fig. 10A (1 week), Fig. 10B (2 weeks) and Table 23, varying the dose of either AAV or LNP can modulate the amount of expression of hFIX *in vivo.*

**Table 23: Serum hFIX**

| **Timepoint** | **RNP Dose (mg/kg)** | **AAV Dose (MOI)** | **Mean FIX (ng/ml)** | **SD** | **N** |
|---|---|---|---|---|---|
| Week 1 | 0.1 | 1E+11 | 0.08 | 0.02 | 2 |
| | | 3E+11 | 0.11 | 0.04 | 5 |
| | | 1E+12 | 0.41 | 0.15 | 5 |
| | | 3E+12 | 0.61 | 0.17 | 5 |
| | 0.3 | 1E+11 | 0.36 | 0.14 | 5 |
| | | 3E+11 | 0.67 | 0.26 | 5 |
| | | 1E+12 | 1.76 | 0.14 | 5 |
| | | 3E+12 | 4.70 | 2.40 | 5 |
| | 1.0 | 1E+11 | 3.71 | 0.31 | 4 |
| | | 3E+11 | 8.00 | 0.51 | 5 |
| | | 1E+12 | 14.17 | 1.38 | 5 |
| | | 3E+12 | 20.70 | 2.79 | 5 |
| | Human serum 1:1000 | | 6.62 | - | 1 |
| Week 2 | 0.1 | 1E+11 | 0.12 | 0.01 | 2 |
| | | 3E+11 | 0.26 | 0.07 | 5 |
| | | 1E+12 | 0.83 | 0.24 | 5 |
| | | 3E+12 | 1.48 | 0.35 | 5 |
| | 0.3 | 1E+11 | 0.70 | 0.26 | 4 |
| | | 3E+11 | 1.42 | 0.37 | 5 |
| | | 1E+12 | 3.53 | 0.49 | 5 |
| | | 3E+12 | 8.94 | 4.39 | 5 |
| | 1.0 | 1E+11 | 5.40 | 0.47 | 4 |
| | | 3E+11 | 12.31 | 2.45 | 5 |
| | | 1E+12 | 17.89 | 1.95 | 5 |
| | | 3E+12 | 25.52 | 3.62 | 5 |
| | Human serum 1:1000 | | 4.47 | - | 1 |

### Example 9- in vitro screening of bidirectional constructs across target sites in primary cynomolgus and primary human hepatocytes

In this Example, ssAAV vectors comprising a bidirectional construct were tested across a panel of target sites utilizing gRNAs targeting intron 1 of cynomolgus ("cyno") and human albumin in primary cyno (PCH) and primary human hepatocytes (PHH), respectively.

The ssAAV and lipid packet delivery materials tested in this Example were prepared and delivered to PCH and PHH as described in Example 1. Following treatment, isolated genomic DNA and cell media was collected for editing and transgene expression analysis, respectively. Each of the vectors comprised a reporter that can be measured through luciferase-based fluorescence detection as described in Example 1 (derived from P00415), plotted in Fig.s 11B and 12B as relative luciferase units ("RLU"). For example, the AAV vectors contained the NanoLuc ORF (in addition to GFP). Schematics of the vectors tested are provided in Fig.s 11B and 12B. The gRNAs tested are shown in each of the Fig.s using a shortened number for those listed in Table 1 and Table 7.

As shown in Fig. 11A for PCH and Fig. 12A for PHH, varied levels of editing were detected for each of the combinations tested (editing data for some combinations tested in the PCH experiment are not reported in Fig. 11A and Table 1 due to failure of certain primer pairs used for the amplicon based sequencing). The editing data shown in Fig.s 11A and 12A graphically, are reproduced numerically in Table 1 and Table 2 below. However, as shown in Fig.s 11B, 11C and Fig.s 12B and 12C, a significant level of indel formation was not predictive for insertion or expression of the transgenes, indicating little correlation between editing and insertion/expression of the bidirectional constructs in PCH and PHH, respectively. As one measure, the R² value calculated in Fig. 11C is 0.13, and the R² value of Fig. 12D is 0.22.

**Table 1: Albumin intron 1 editing and transgene expression data for sgRNAs delivered to primary cynomolgus hepatocytes**

| **GUIDE ID** | **Avg % Edit** | **Std Dev % Edit** | **Avg RLU** | **Std Dev RLU** |
|---|---|---|---|---|
| G009867 | 25.05 | 0.21 | 10650.67 | 1455.97 |
| G009866 | 18.7 | 3.96 | 75556.67 | 12182.98 |
| G009876 | 14.85 | 4.88 | 27463.33 | 10833.53 |
| G009875 | 12.85 | 2.33 | 51660.00 | 6362.36 |
| G009874 | 28.25 | 6.01 | 270433.30 | 133734.10 |
| G009873 | 42.65 | 5.59 | 178600.00 | 87607.25 |
| G009865 | 59.15 | 0.21 | 301666.70 | 18610.03 |
| G009872 | 48.15 | 3.46 | 320233.30 | 63517.43 |
| G009871 | 46.5 | 5.23 | 211966.70 | 65852.44 |
| G009864 | 33.2 | 8.34 | 210033.30 | 61201.33 |
| G009863 | 54.8 | 12.45 | 69853.33 | 15216.92 |
| G009862 | 44.6 | 7.21 | 508666.70 | 119876.30 |
| G009861 | 28.65 | 0.21 | 178666.70 | 15821.93 |
| G009860 | 33.2 | 7.07 | 571333.30 | 52728.87 |
| G009859 | 0.05 | 0.07 | 258333.30 | 79052.73 |
| G009858 | 14.65 | 1.77 | 402333.30 | 25579.94 |
| G009857 | 23 | 0.99 | 312333.30 | 73036.52 |
| G009856 | 14.8 | 0.99 | 95900.00 | 21128.42 |
| G009851 | 1.5 | 0.42 | 105766.70 | 27048.91 |
| G009868 | 12.15 | 2.47 | 43033.33 | 9141.85 |
| G009850 | 63.45 | 13.93 | 228200.00 | 101542.10 |
| G009849 | 57.55 | 8.27 | 225400.00 | 46001.30 |
| G009848 | 33 | 5.37 | 156333.30 | 20647.84 |
| G009847 | 66.75 | 7 | 100866.70 | 22159.72 |
| G009846 | 61.85 | 5.02 | 31766.67 | 10107.59 |
| G009845 | 54.4 | 7.5 | 43020.00 | 11582.23 |
| G009844 | 47.15 | 2.05 | 110466.70 | 32031.44 |

**Table 2: Albumin intron 1 editing and transgene expression data for sgRNAs delivered to primary human hepatocytes**

| **GUIDE ID** | **Avg % Edit** | **Std Dev % Edit** | **Avg RLU** | **Std Dev RLU** |
|---|---|---|---|---|
| G009844 | 19.07 | 2.07 | 268333.30 | 80432.17 |
| G009851 | 0.43 | 0.35 | 18033.33 | 2145.54 |
| G009852 | 47.20 | 3.96 | 18400.00 | 2251.67 |
| G009857 | 0.10 | 0.14 | 71100.00 | 14609.24 |
| G009858 | 8.63 | 9.16 | 32000.00 | 18366.55 |
| G009859 | 3.07 | 3.50 | 59500.00 | 16014.99 |
| G009860 | 18.80 | 4.90 | 190333.30 | 54307.76 |
| G009861 | 10.27 | 2.51 | 62233.33 | 9865.26 |
| G009866 | 13.60 | 13.55 | 96200.00 | 46573.81 |
| G009867 | 12.97 | 3.04 | 3916.67 | 1682.03 |
| G009868 | 0.63 | 0.32 | 10176.67 | 2037.80 |
| G009874 | 49.13 | 0.60 | 318000.00 | 114118.40 |
| G012747 | 3.83 | 0.23 | 51000.00 | 6161.17 |
| G012748 | 1.30 | 0.35 | 17433.33 | 2709.86 |
| G012749 | 9.77 | 1.50 | 75066.67 | 11809.04 |
| G012750 | 42.73 | 4.58 | 5346.67 | 2977.35 |
| G012751 | 7.77 | 1.16 | 32066.67 | 18537.62 |
| G012752 | 32.93 | 2.27 | 402000.00 | 83144.45 |
| G012753 | 21.20 | 2.95 | 71800.00 | 32055.73 |
| G012754 | 0.60 | 0.10 | 16933.33 | 4254.80 |
| G012755 | 1.10 | 0.10 | 13833.33 | 3685.56 |
| G012756 | 2.17 | 0.40 | 35600.00 | 6055.58 |
| G012757 | 1.07 | 0.25 | 13993.33 | 6745.08 |
| G012758 | 0.90 | 0.10 | 34900.00 | 15308.82 |
| G012759 | 2.60 | 0.35 | 30566.67 | 15287.36 |
| G012760 | 39.10 | 6.58 | 6596.67 | 2133.13 |
| G012761 | 36.17 | 2.43 | 467666.70 | 210965.20 |
| G012762 | 8.50 | 0.57 | 217000.00 | 13000.00 |
| G012763 | 47.07 | 3.07 | 142333.30 | 37581.02 |
| G012764 | 44.57 | 5.83 | 1423333.00 | 261023.60 |
| G012765 | 19.90 | 1.68 | 179666.70 | 57011.69 |
| G012766 | 8.50 | 0.28 | 243333.30 | 17473.79 |

Additionally, ssAAV vectors comprising a bidirectional construct were tested across a panel of target sites utilizing single guide RNAs targeting intron 1 of human albumin in primary human hepatocytes (PHH).

The ssAAV and LNP materials were prepared and delivered to PHH as described in Example 1. Following treatment, isolated genomic DNA and cell media was collected for editing and transgene expression analysis, respectively. As above, each of the vectors comprised a reporter that can be measured through luciferase-based fluorescence detection as described in Example 1 (derived from plasmid P00415), plotted in Fig. 12C and shown in Table 23 as relative luciferase units ("RLU"). For example, the AAV vectors contained the NanoLuc ORF (in addition to GFP). Schematics of the vectors tested are provided in Figs. 11B and 12B. The gRNAs tested are shown in Fig. 12C using a shortened number for those listed in Table 1 and Table 7.

**Table 23: Albumin intron 1 transgene expression data for sgRNAs delivered to primary human hepatocytes**

| Guide | Average Luciferase (RLU) | St. Dev Luciferase (RLU) |
|---|---|---|
| G009844 | 3,700,000 | 509,117 |
| G009852 | 281,000 | 69,296 |
| G009857 | 1,550,000 | 127,279 |
| G009858 | 551,000 | 108,894 |
| G009859 | 1,425,000 | 77,782 |
| G009860 | 2,240,000 | 183,848 |
| G009861 | 663,500 | 238,295 |
| G009866 | 274,000 | 11,314 |
| G009867 | 44,700 | 566 |
| G009874 | 2,865,000 | 431,335 |
| G012747 | 651,000 | 59,397 |
| G012749 | 867,000 | 93,338 |
| G012752 | 4,130,000 | 268,701 |
| G012753 | 1,145,000 | 162,635 |
| G012757 | 579,000 | 257,387 |
| G012760 | 129,000 | 36,770 |
| G012761 | 4,045,000 | 728,320 |
| G012762 | 2,220,000 | 127,279 |
| G012763 | 1,155,000 | 205,061 |
| G012764 | 11,900,000 | 1,555,635 |
| G012765 | 1,935,000 | 134,350 |
| G012766 | 2,050,000 | 169,706 |
| LNP | 8,430 | 212 |

### Example 10- in vivo testing of Factor IX expression from an alternative safe harbor locus

In this Example, insertion of ssAAV comprising a bidirectional hFIX construct at an alternative safe harbor locus was evaluated. To test the insertion into an altenative safe harbor locus, AAV was prepared as described above. Mice were administered with AAVs at a dose of 3e11 vg/mouse immediately followed by administration of LNPs formulated with Cas9 mRNAs and guide RNAs at a dose of 0.3 mg/kg. Animals were sacrificed 4 weeks post-dose, and liver and blood samples were collected. Editing in the liver samples was determined by NGS. Human hFIX levels in the serum was determined by ELISA. The NGS and ELISA data showed effective insertion and expression of hFIX within the alternative safe harbor locus.

### Example 11- in vivo testing of the human Factor IX gene insertion in non-human primates

In this example, an 8 week study was performed to evaluate the human Factor IX gene insertion and hFIX protein expression in cynomolgus monkeys through administration of adeno-associated virus (AAV) and/or lipid nanoparticles (LNP) with various guides. This study was conducted with LNP formulations and AAV formulations prepared as described above. Each LNP formulation contained Cas9 mRNA and guide RNA (gRNA) with an mRNA:gRNA ratio of 2:1 by weight. The ssAAV was derived from P00147.

Male cynomologus monkeys were treated in cohorts of n=3. Animals were dosed with AAV by slow bolus injection or infusion in the doses described in Table 3. Following AAV treatment, animals received buffer or LNP as described in Table 3 by slow bolus or infusion.

Two weeks post-dose, liver specimens were collected through single ultrasound-guided percutaneous biopsy. Each biopsy specimen was flash frozen in liquid nitrogen and stored at -86 to -60 °C. Editing analysis of the liver specimens was performed by NGS Sequencing as previously described.

For Factor IX ELISA analysis, blood samples were collected from the animals on days 7, 14, 28, and 56 post-dose. Blood samples were collected and processed to plasma following blood draw and stored at -86 to -60 °C until analysis.

The total human Factor IX levels were determined from plasma samples by ELISA. Briefly, Reacti-Bind 96-well microplate (VWR Cat# PI15041) were coated with capture antibody (mouse mAB to human Factor IX antibody (HTI, Cat#AHIX-5041)) at a concentration of 1 µg/ml then blocked using 1x PBS with 5% Bovine Serum Albumin. Test samples or standards of purified human Factor IX protein (ERL, Cat# HFIX 1009, Lot#HFIX4840) diluted in Cynomolgus monkey plasma were next incubated in individual wells. The detection antibody (Sheep anti-human Factor 9 polyclonal antibody, Abcam, Cat# ab128048) was adsorbed at a concentration of 100 ng/ml. The secondary antibody (Donkey anti-Sheep IgG pAbs with HRP, Abcam, Cat# ab97125) was used at 100 ng/mL. TMB Substrate Reagent set (BD OptEIA Cat#555214) was used to develop the plate. Optical density was assessed spectrophotometrically at 450 nm on a microplate reader (Molecular Devices i3 system) and analyzed using SoftMax pro 6.4.

Indel formation was detected, confirming that editing occurred. The NGS data showed effective indel formation. Expression of hFIX from the albumin locus in NHPs was measured by ELISA and is depicted in Table 4 and Fig. 13. Plasma levels of hFIX reached levels previously described as therapeutically effective (George, et al., NEJM 377(23), 2215-27, 2017).

As measured, circulating hFIX protein levels were sustained through the eight week study (see Fig. 13, showing day 7, 14, 28, and 56 average levels of ~135, ~140, ~150, and ~110 ng/mL, respectively), achieving protein levels ranging from ~75 ng/mL to ~250 ng/mL. Plasma hFIX levels were calculated using a specific activity of ~8 fold higher for the R338L hyperfunctional hFIX variant (Simioni et al., NEJM 361(17), 1671-75, 2009) (which reports a protein-specific activity of hFIX-R338L of 390±28 U per milligram, and a protein-specific activity for wild-type factor IX of 45±2.4 U per milligram). Calculating the functionally normalized Factor IX activity for the hyperfunctional Factor IX variant tested in this example, the experiment achieved stable levels of human Factor IX protein in the NHPs over the 8 week study that correspond to about 20-40% of wild type Factor IX activity (range spans 12-67% of wild type Factor IX activity).

**Table 3: Editing in liver**

| Animal ID | Guide ID | F9-AAV (vg/kg) | F9-AAV Volume (mL/kg) | LNP (mg/kg) | LNP Volume (mL/kg) |
|---|---|---|---|---|---|
| 4001 | G009860 | 3E+13 | 1 | 3 | 2 |
| 4002 | G009860 | 3E+13 | 1 | 3 | 2 |
| 4003 | G009860 | 3E+13 | 1 | 3 | 2 |
| 5001 | TSS | 3E+13 | 1 | 0 | 0 |
| 5002 | TSS | 3E+13 | 1 | 0 | 0 |
| 5003 | TSS | 3E+13 | 1 | 0 | 0 |
| 6001 | G009862 | 0 | 0 | 3 | 2 |
| 6002 | G009862 | 0 | 0 | 3 | 2 |
| 6003 | G009862 | 0 | 0 | 3 | 2 |

**Table 4: hFIX expression**

| Animal ID | **Day 7 Factor IX (ng/mL)** | **Day 14 Factor IX (ng/mL)** | **Day 28 Factor IX (ng/mL)** | **Day 56 Factor IX (ng/mL)** |
|---|---|---|---|---|
| 4001 | 122.84/+-2.85 | 94.93/+-0.56 | 105.65/+-1.94 | 97.31/+- 1.49 |
| 4002 | 149.77/+-13.5 | 222.92/+-9.61 | 252.49/+-6.46 | 152.05/+-7.46 |
| 4003 | 134.06/+-6.17 | 107.04/+-6.46 | 95.30/+-3.18 | 74.23/+- 3.53 |
| 5001 | ND | ND | ND | ND |
| 5002 | ND | ND | ND | ND |
| 5003 | ND | ND | ND | ND |
| 6001 | ND | ND | ND | ND |
| 6002 | ND | ND | ND | ND |
| 6003 | ND | ND | ND | ND |

### Example 12 in vivo testing of Factor IX insertion in non-human primates

In this example, a study was performed to evaluate the Factor IX gene insertion and hFIX protein expression in cynomolgus monkeys following administration of ssAAV derived from P00147 and/or CRISPR/Cas9 lipid nanoparticles (LNP) with various guides including G009860 and various LNP components.

Indel formation was measured by NGS, confirming that editing occurred. Total human Factor IX levels were determined from plasma samples by ELISA, using a mouse mAB to human Factor IX antibody (HTI, Cat#AHIX-5041), sheep anti-human Factor 9 polyclonal antibody (Abcam, Cat# ab128048), and donkey anti-Sheep IgG pAbs with HRP (Abcam, Cat# ab97125), as described in Example 11. Human FIX protein levels >3 fold higher than those achieved in the experiment of Example 13 were obtained from the bidirectional template using alternative CRISPR/Cas9 LNP. In the study, ELISA assay results indicate that circulating hFIX protein levels at or above the normal range of human FIX levels (3-5 ug/mL; Amiral et al., Clin. Chem., 30(9), 1512-16, 1984) were achieved using G009860 in the NHPs by at least the day 14 and 28 timepoints. Initial data indicated circulating human FIX protein levels of ~3-4 µg/mL at day 14 after a single dose, with levels sustained through the first 28 days (~3-5 µg/mL) of the study.
Circulating albumin levels were measured by ELISA, indicating that baseline albumin levels are maintained at 28 days. Tested albumin levels in untreated animals varied ± ~15% in the study. In treated animals, circulating albumin levels changed minimally and did not drop out of the normal range, and the levels recovered to baseline within one month.

Circulating human FIX protein levels were also determined by a sandwich immunoassay with a greater dynamic range. Briefly, an MSD GOLD 96-well Streptavidin SECTOR Plate (Meso Scale Diagnostics, Cat. L15SA-1) was blocked with 1% ECL Blocking Agent (Sigma, GERPN2125). After tapping out the blocking solution, biotinylated capture antibody (Sino Biological, 11503-R044) was immobilized on the plate. Recombinant human FIX protein (Enzyme Research Laboratories, HFIX 1009) was used to prepare a calibration standard in 0.5% ECL Blocking Agent. Following a wash, calibration standards and plasma samples were added to the plate and incubated. Following a wash, a detection antibody (Haematologic Technologies, AHIX-5041) conjugated with a sulfo-tag label was added to the wells and incubated. After washing away any unbound detection antibody, Read Buffer T was applied to the wells. Without any additional incubation, the plate was imaged with an MSD Quick Plex SQ120 instrument and data was analyzed with Discovery Workbench 4.0 software package (Meso Scale Discovery). Concentrations are expressed as mean calculated concentrations in ug/ml. For the samples, N=3 unless indicated with an asterisk, in which case N=2. Expression of hFIX from the albumin locus in the treated study group as measured by the MSD ELISA is depicted in Table 24.

**Table 24: Serum human Factor IX protein levels**

| | Mean Calc. Conc. (ug/mL) | | |
|---|---|---|---|
| | 3001 | 3002 | 3003 |
| Day 7 | 7.85 | 5.63 | 11.20 |
| Day 14 | 8.65 | 11.06 | 14.70 |
| Day 28 | 9.14 | 14.12 | 10.85 |
| Day 42 | 9.03 | 33.12* | 13.22 |
| Day 56 | 10.24 | 16.72 | 33.84* |

### Example 13- Off-target analysis of Albumin Human Guides

A biochemical method (See, *e.g.,* Cameron et al., Nature Methods. 6, 600-606; 2017) was used to determine potential off-target genomic sites cleaved by Cas9 targeting Albumin. In this experiment, 13 sgRNA targeting human Albumin and two control guides with known off-target profiles were screened using isolated HEK293 genomic DNA. The number of potential off-target sites detected using a guide concentration of 16 nM in the biochemical assay were shown in Table 26. The assay identified potential off-target sites for the sgRNAs tested.

**Table 25: Off-Target Analysis**

| gRNA ID | Target | Guide Sequence (SEQ ID NO:) | Off-Target Site Count |
|---|---|---|---|
| G012753 | Albumin | GACUGAAACUUCACAGAAUA (SEQ ID NO: 20) | 62 |
| G012761 | Albumin | AGUGCAAUGGAUAGGUCUUU (SEQ ID NO: 28) | 75 |
| G012752 | Albumin | UGACUGAAACUUCACAGAAU (SEQ ID NO: 19) | 223 |
| G012764 | Albumin | CCUCACUCUUGUCUGGGCAA (SEQ ID NO: 31) | 3985 |
| G012763 | Albumin | UGGGCAAGGGAAGAAAAAAA (SEQ ID NO: 30) | 5443 |
| G009857 | Albumin | AUUUAUGAGAUCAACAGCAC (SEQ ID NO: 5) | 131 |
| G009859 | Albumin | UUAAAUAAAGCAUAGUGCAA (SEQ ID NO: 7) | 91 |
| G009860 | Albumin | UAAAGCAUAGUGCAAUGGAU (SEQ ID NO: 8) | 133 |
| G012762 | Albumin | UGAUUCCUACAGAAAAACUC (SEQ ID NO: 29) | 68 |
| G009844 | Albumin | GAGCAACCUCACUCUUGUCU (SEQ ID NO: 2) | 107 |
| G012765 | Albumin | ACCUCACUCUUGUCUGGGCA (SEQ ID NO: 32) | 41 |
| G012766 | Albumin | UGAGCAACCUCACUCUUGUC (SEQ ID NO: 33) | 78 |
| G009874 | Albumin | UAAUAAAAUUCAAACAUCCU (SEQ ID NO: 13) | 53 |
| G000644 | EMX1 | GAGUCCGAGCAGAAGAAGAA (SEQ ID NO: 1129) | 304 |
| G000645 | VEGFA | GACCCCCUCCACCCCGCCUC (SEQ ID NO: 1130) | 1641 |

In known off-target detection assays such as the biochemical method used above, a large number of potential off-target sites are typically recovered, by design, so as to "cast a wide net" for potential sites that can be validated in other contexts, *e*.*g*., in a primary cell of interest. For example, the biochemical method typically overrepresents the number of potential off-target sites as the assay utilizes purified high molecular weight genomic DNA free of the cell environment and is dependent on the dose of Cas9 RNP used. Accordingly, potential off-target sites identified by these methods may be validated using targeted sequencing of the identified potential off-target sites.

### Example 14 - Construction of constructs for the expression of secretory or non secretory proteins

Constructs, such as bidirectional constructs, can be designed such that they express secretory or non secretory proteins. For the production of a secretory protein, a construct may comprise a signal sequence which aids in translocating the polypeptide to the ER lumen. Alternatively, a construct may utilize the endogenous signal sequence of the host cell (*e.g.,* the endogenous albumin signal sequence when the transgene is integrated into a host cell's albumin locus).

In contrast, constructs for the expression of non secretory proteins may be designed such that they do not comprise a signal sequence and such that they do not utilize the endogenous signal sequence of the host cell. Some methods by which this may be achieved include the incorporation of an Internal ribosome entry site (IRES) sequence in the construct. IRES sequences, such as EMCV IRES, allow for the initiation of translation from any position within an mRNA immediately downstream from where the IRES is located. This would allow for the expression of a protein which lacks the endogenous signal sequence of the host cell from an insertion site that contains a signal sequence upstream (*e.g.* the signal sequence found in Exon 1 of albumin locus would not be included in the expressed protein). In the absence of a signal sequence, the protein would not be secreted. Examples of IRES sequences that can be used in a construct, include those from picornaviruses (e*.g.,* FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SIV) or cricket paralysis viruses (CrPV).

An alternative approach for expressing non secretory proteins is to include one or more self-cleaving peptides upstream of the polypeptide of interest in the construct. A self cleaving peptide, such as 2A or 2A-like sequences, serve as ribosome skipping signals to produce multiple individual proteins from a single mRNA transcript. As shown in Plasmid ID P00415 from Table 11, a self cleaving peptide (*e.g.* P2A) can be used to generate a bicistronic vector which expresses two transgenes (*e.g.,* nanoluciferase and GFP). Alternatively, a self cleaving peptide can be used to express a protein which lacks the endogenous signal sequence of the host cell (*e.g.* the 2A sequence located upstream of the protein of interest would result in cleavage between the endogenous albumin signal sequence and the protein of interest). Representative 2A peptides which could be utilized are shown in Table 12. Additionally, (GSG) residues may be added to the 5' end of the peptide to improve cleavage efficiency as shown in Table 12.

| **Table 26: Self cleaving peptides for use in constructs** | |
|---|---|
| **Peptide** | **Amino Acid Sequence** |
| T2A (SEQ ID NO: 1131) | EGRGSLLTCGDVEENPGP |
| P2A (SEQ ID NO: 1132) | ATNFSLLKQAGDVEENPGP |
| E2A (SEQ ID NO: 1133) | QCTNYALLKLAGDVESNPGP |
| F2A (SEQ ID NO: 1134) | VKQTLNFDLLKLAGDVESNPGP |
| T2A with GSG residues (SEQ ID NO: 1135) | GSGEGRGSLLTCGDVEENPGP |
| P2A with GSG residues (SEQ ID NO: 1136) | GSGATNFSLLKQAGDVEENPGP |
| E2A with GSG residues (SEQ ID NO: 1137) | GSGQCTNYALLKLAGDVESNPGP |
| F2A with GSG residues (SEQ ID NO: 1138) | GSGVKQTLNFDLLKLAGDVESNPGP |

**Table 5: Human guide RNA sequences**

| **Guide ID** | **Guide Sequence** | **Genomic Coordinates** | **SEQ ID NO:** |
|---|---|---|---|
| G009844 | GAGCAACCUCACUCUUGUCU | chr4:73405113-73405133 | 2 |
| G009851 | AUGCAUUUGUUUCAAAAUAU | chr4:73405000-73405020 | 3 |
| G009852 | UGCAUUUGUUUCAAAAUAUU | chr4:73404999-73405019 | 4 |
| G009857 | AUUUAUGAGAUCAACAGCAC | chr4:73404761-73404781 | 5 |
| G009858 | GAUCAACAGCACAGGUUUUG | chr4:73404753-73404773 | 6 |
| G009859 | UUAAAUAAAGCAUAGUGCAA | chr4:73404727-73404747 | 7 |
| G009860 | UAAAGCAUAGUGCAAUGGAU | chr4:73404722-73404742 | 8 |
| G009861 | UAGUGCAAUGGAUAGGUCUU | chr4:73404715-73404735 | 9 |
| G009866 | UACUAAAACUUUAUUUUACU | chr4:73404452-73404472 | 10 |
| G009867 | AAAGUUGAACAAUAGAAAAA | chr4:73404418-73404438 | 11 |
| G009868 | AAUGCAUAAUCUAAGUCAAA | chr4:73405013-73405033 | 12 |
| G009874 | UAAUAAAAUUCAAACAUCCU | chr4:73404561-73404581 | 13 |
| G012747 | GCAUCUUUAAAGAAUUAUUU | chr4:73404478-73404498 | 14 |
| G012748 | UUUGGCAUUUAUUUCUAAAA | chr4:73404496-73404516 | 15 |
| G012749 | UGUAUUUGUGAAGUCUUACA | chr4:73404529-73404549 | 16 |
| G012750 | UCCUAGGUAAAAAAAAAAAA | chr4:73404577-73404597 | 17 |
| G012751 | UAAUUUUCUUUUGCGCACUA | chr4:73404620-73404640 | 18 |
| G012752 | UGACUGAAACUUCACAGAAU | chr4:73404664-73404684 | 19 |
| G012753 | GACUGAAACUUCACAGAAUA | chr4:73404665-73404685 | 20 |
| G012754 | UUCAUUUUAGUCUGUCUUCU | chr4:73404803-73404823 | 21 |
| G012755 | AUUAUCUAAGUUUGAAUAUA | chr4:73404859-73404879 | 22 |
| G012756 | AAUUUUUAAAAUAGUAUUCU | chr4:73404897-73404917 | 23 |
| G012757 | UGAAUUAUUCUUCUGUUUAA | chr4:73404924-73404944 | 24 |
| G012758 | AUCAUCCUGAGUUUUUCUGU | chr4:73404965-73404985 | 25 |
| G012759 | UUACUAAAACUUUAUUUUAC | chr4:73404453-73404473 | 26 |
| G012760 | ACCUUUUUUUUUUUUUACCU | chr4:73404581-73404601 | 27 |
| G012761 | AGUGCAAUGGAUAGGUCUUU | chr4:73404714-73404734 | 28 |
| G012762 | UGAUUCCUACAGAAAAACUC | chr4:73404973-73404993 | 29 |
| G012763 | UGGGCAAGGGAAGAAAAAAA | chr4:73405094-73405114 | 30 |
| G012764 | CCUCACUCUUGUCUGGGCAA | chr4:73405107-73405127 | 31 |
| G012765 | ACCUCACUCUUGUCUGGGCA | chr4:73405108-73405128 | 32 |
| G012766 | UGAGCAACCUCACUCUUGUC | chr4:73405114-73405134 | 33 |

**Table 6: Mouse guide RNA sequences**

| **Guide ID** | **Guide Sequence** | **Genomic Coordinates** | **SEQ ID NO:** |
|---|---|---|---|
| G000551 | AUUUGCAUCUGAGAACCCUU | chr5:90461148-90461168 | 98 |
| G000552 | AUCGGGAACUGGCAUCUUCA | chr5:90461590-90461610 | 99 |
| G000553 | GUUACAGGAAAAUCUGAAGG | chr5:90461569-90461589 | 100 |
| G000554 | GAUCGGGAACUGGCAUCUUC | chr5:90461589-90461609 | 101 |
| G000555 | UGCAUCUGAGAACCCUUAGG | chr5:90461151-90461171 | 102 |
| G000666 | CACUCUUGUCUGUGGAAACA | chr5:90461709-90461729 | 103 |
| G000667 | AUCGUUACAGGAAAAUCUGA | chr5:90461572-90461592 | 104 |
| G000668 | GCAUCUUCAGGGAGUAGCUU | chr5:90461601-90461621 | 105 |
| G000669 | CAAUCUUUAAAUAUGUUGUG | chr5:90461674-90461694 | 106 |
| G000670 | UCACUCUUGUCUGUGGAAAC | chr5:90461710-90461730 | 107 |
| G011722 | UGCUUGUAUUUUUCUAGUAA | chr5:90461039-90461059 | 108 |
| G011723 | GUAAAUAUCUACUAAGACAA | chr5:90461425-90461445 | 109 |
| G011724 | UUUUUCUAGUAAUGGAAGCC | chr5:90461047-90461067 | 110 |
| G011725 | UUAUAUUAUUGAUAUAUUUU | chr5:90461174-90461194 | 111 |
| G011726 | GCACAGAUAUAAACACUUAA | chr5:90461480-90461500 | 112 |
| G011727 | CACAGAUAUAAACACUUAAC | chr5:90461481-90461501 | 113 |
| G011728 | GGUUUUAAAAAUAAUAAUGU | chr5:90461502-90461522 | 114 |
| G011729 | UCAGAUUUUCCUGUAACGAU | chr5:90461572-90461592 | 115 |
| G011730 | CAGAUUUUCCUGUAACGAUC | chr5:90461573-90461593 | 116 |
| G011731 | CAAUGGUAAAUAAGAAAUAA | chr5:90461408-90461428 | 117 |
| G013018 | GGAAAAUCUGAAGGUGGCAA | chr5:90461563-90461583 | 118 |
| G013019 | GGCGAUCUCACUCUUGUCUG | chr5:90461717-90461737 | 119 |

**Table 7: Cyno guide RNA sequences**

| **Guide ID** | **Guide Sequence** | **Genomic Coordinates** | **SEQ ID NO:** |
|---|---|---|---|
| G009844 | GAGCAACCUCACUCUUGUCU | chr5:61198711-61198731 | 164 |
| G009845 | AGCAACCUCACUCUUGUCUG | chr5:61198712-61198732 | 165 |
| G009846 | ACCUCACUCUUGUCUGGGGA | chr5:61198716-61198736 | 166 |
| G009847 | CCUCACUCUUGUCUGGGGAA | chr5:61198717-61198737 | 167 |
| G009848 | CUCACUCUUGUCUGGGGAAG | chr5:61198718-61198738 | 168 |
| G009849 | GGGGAAGGGGAGAAAAAAAA | chr5:61198731-61198751 | 169 |
| G009850 | GGGAAGGGGAGAAAAAAAAA | chr5:61198732-61198752 | 170 |
| G009851 | AUGCAUUUGUUUCAAAAUAU | chr5:61198825-61198845 | 171 |
| G009852 | UGCAUUUGUUUCAAAAUAUU | chr5:61198826-61198846 | 172 |
| G009853 | UGAUUCCUACAGAAAAAGUC | chr5:61198852-61198872 | 173 |
| G009854 | UACAGAAAAAGUCAGGAUAA | chr5:61198859-61198879 | 174 |
| G009855 | UUUCUUCUGCCUUUAAACAG | chr5:61198889-61198909 | 175 |
| G009856 | UUAUAGUUUUAUAUUCAAAC | chr5:61198957-61198977 | 176 |
| G009857 | AUUUAUGAGAUCAACAGCAC | chr5:61199062-61199082 | 177 |
| G009858 | GAUCAACAGCACAGGUUUUG | chr5:61199070-61199090 | 178 |
| G009859 | UUAAAUAAAGCAUAGUGCAA | chr5:61199096-61199116 | 179 |
| G009860 | UAAAGCAUAGUGCAAUGGAU | chr5:61199101-61199121 | 180 |
| G009861 | UAGUGCAAUGGAUAGGUCUU | chr5:61199108-61199128 | 181 |
| G009862 | AGUGCAAUGGAUAGGUCUUA | chr5:61199109-61199129 | 182 |
| G009863 | UUACUUUGCACUUUCCUUAG | chr5:61199186-61199206 | 183 |
| G009864 | UACUUUGCACUUUCCUUAGU | chr5:61199187-61199207 | 184 |
| G009865 | UCUGACCUUUUAUUUUACCU | chr5:61199238-61199258 | 185 |
| G009866 | UACUAAAACUUUAUUUUACU | chr5:61199367-61199387 | 186 |
| G009867 | AAAGUUGAACAAUAGAAAAA | chr5:61199401-61199421 | 187 |
| G009868 | AAUGCAUAAUCUAAGUCAAA | chr5:61198812-61198832 | 188 |
| G009869 | AUUAUCCUGACUUUUUCUGU | chr5:61198860-61198880 | 189 |
| G009870 | UGAAUUAUUCCUCUGUUUAA | chr5:61198901-61198921 | 190 |
| G009871 | UAAUUUUCUUUUGCCCACUA | chr5:61199203-61199223 | 191 |
| G009872 | AAAAGGUCAGAAUUGUUUAG | chr5:61199229-61199249 | 192 |
| G009873 | AACAUCCUAGGUAAAAUAAA | chr5:61199246-61199266 | 193 |
| G009874 | UAAUAAAAUUCAAACAUCCU | chr5:61199258-61199278 | 194 |
| G009875 | UUGUCAUGUAUUUCUAAAAU | chr5:61199322-61199342 | 195 |
| G009876 | UUUGUCAUGUAUUUCUAAAA | chr5:61199323-61199343 | 196 |

**Table 8. Human albumin sgRNA and modification patterns**

| **Guide ID** | **Full Sequence** | **SEQ ID NO:** | **Full Sequence Modified** | **SEQ ID NO:** |
|---|---|---|---|---|
| G009844 | GAGCAACCUCACUCUUGUCUGUUU U AGAGCUAGAAAUAGCAAGUUAAAA U AAGGCUAGUCCGUUAUCAACUUGA A AAAGUGGCACCGAGUCGGUGCUUU U | 34 | mG*mA*mG*CAACCUCACUCUUG UCUGU UUUAGAmGmCmUmAmGmAmAm AmUm AmGmCAAGUUAAAAUAAGGCUA GUCC GUUAUCAmAmCmUmUmGmAmA mAmAm AmGmUmGmGmCmAmCmCmGmA mGmUm CmGmGmUmGmCmU*mU*mU*mU | 66 |
| G009851 | AUGCAUUUGUUUCAAAAUAUGUUU U AGAGCUAGAAAUAGCAAGUUAAAA U AAGGCUAGUCCGUUAUCAACUUGA A AAAGUGGCACCGAGUCGGUGCUUU U | 35 | mA*mU*mG*CAUUUGUUCAAAA UAUG UUUUAGAmGmCmUmAmGmAmA mAmUm AmGmCAAGUUAAAAUAAGGCUA GUCCG UUAUCAmAmCmUmUmGmAmAm AmAmAm GmUmGmGmCmAmCmCmGmAmG mUmCm GmGmUmGmCmU*mU*mU*mU | 67 |
| G009852 | UGCAUUUGUUUCAAAAUAUUGUUU U AGAGCUAGAAAUAGCAAGUUAAAA U AAGGCUAGUCCGUUAUCAACUUGA A AAAGUGGCACCGAGUCGGUGCUUU U | 36 | mU*mG*mC*AUUUGUUUCAAAAU AUUGU UUUAGAmGmCmUmAmGmAmAm AmUmAm GmCAAGUUAAAAUAAGGCUAGU CCGUUA UCAmAmCmUmUmGmAmAmAmA mAmGmUmGmGmCmAmCmCmGm AmGmUmCmGmGmUmGmCmU*m U*mU*mU | 68 |
| G009857 | AUUUAUGAGAUCAACAGCACGUUU U AGAGCUAGAAAUAGCAAGUUAAAA U AAGGCUAGUCCGUUAUCAACUUGA A AAAGUGGCACCGAGUCGGUGCUUU U | 37 | mA*mU*mU*UAUGAGAUCAACAG CACGU UUUAGAmGmCmUmAmGmAmAm AmUmAm GmCAAGUUAAAAUAAGGCUAGU CCGUUA UCAmAmCmUmUmGmAmAmAmA mAmGm UmGmGmCmAmCmCmGmAmGmU mCmGmGmUmGmCmU*mU*mU*m U | 69 |
| G009858 | GAUCAACAGCACAGGUUUUGGUUU U AGAGCUAGAAAUAGCAAGUUAAAA U AAGGCUAGUCCGUUAUCAACUUGA A AAAGUGGCACCGAGUCGGUGCUUU U | 38 | mG*mA*mU*CAACAGCACAGGUU UUGGU UUUAGAmGmCmUmAmGmAmAm AmUmAm GmCAAGUUAAAAUAAGGCUAGU CCGUUA UCAmAmCmUmUmGmAmAmAmA mAmGm UmGmGmCmAmCmCmGmAmGmU mCmGm GmUmGmCmU*mU*mU*mU | 70 |
| G009859 | UUAAAUAAAGCAUAGUGCAAGUUU U AGAGCUAGAAAUAGCAAGUUAAAA U AAGGCUAGUCCGUUAUCAACUUGA A AAAGUGGCACCGAGUCGGUGCUUU U | 39 | mU*mU*mA*AAUAAAGCAUAGU GCAAGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 71 |
| G009860 | UAAAGCAUAGUGCAAUGGAUGUUU U AGAGCUAGAAAUAGCAAGUUAAAA U AAGGCUAGUCCGUUAUCAACUUGA A AAAGUGGCACCGAGUCGGUGCUUU U | 40 | mU*mA*mA*AGCAUAGUGCAAUG GAUGUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 72 |
| G009861 | UAGUGCAAUGGAUAGGUCUUGUUU U AGAGCUAGAAAUAGCAAGUUAAAA U AAGGCUAGUCCGUUAUCAACUUGA A AAAGUGGCACCGAGUCGGUGCUUU U | 41 | mU*mA*mG*UGCAAUGGAUAGG UCUUGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 73 |
| G009866 | UACUAAAACUUUAUUUUACUGUUU U AGAGCUAGAAAUAGCAAGUUAAAA U AAGGCUAGUCCGUUAUCAACUUGA A AAAGUGGCACCGAGUCGGUGCUUU U | 42 | mU*mA*mC*UAAAACUUUAUUUU ACUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 74 |
| G009867 | AAAGUUGAACAAUAGAAAAAGUUU U AGAGCUAGAAAUAGCAAGUUAAAA U AAGGCUAGUCCGUUAUCAACUUGA A AAAGUGGCACCGAGUCGGUGCUUU U | 43 | mA*mA*mA*GUUGAACAAUAGA AAAAGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 75 |
| G009868 | AAUGCAUAAUCUAAGUCAAAGUUU U AGAGCUAGAAAUAGCAAGUUAAAA U AAGGCUAGUCCGUUAUCAACUUGA A AAAGUGGCACCGAGUCGGUGCUUU U | 44 | mA*mA*mU*GCAUAAUCUAAGUC AAAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 76 |
| G009874 | UAAUAAAAUUCAAACAUCCUGUUU U AGAGCUAGAAAUAGCAAGUUAAAA U AAGGCUAGUCCGUUAUCAACUUGA A AAAGUGGCACCGAGUCGGUGCUUU U | 45 | mU*mA*mA*UAAAUUCAAACAU CCUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 77 |
| G012747 | GCAUCUUUAAAGAAUUAUUUGUUU U AGAGCUAGAAAUAGCAAGUUAAAA U AAGGCUAGUCCGUUAUCAACUUGA A AAAGUGGCACCGAGUCGGUGCUUU U | 46 | mG*mC*mA*UCUUUAAAGAAUUA UUUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 78 |
| G012748 | UUGGCAUUUAUUUCUAAAAGUUU U AGAGCUAGAAAUAGCAAGUUAAAA U AAGGCUAGUCCGUUAUCAACUUGA A AAAGUGGCACCGAGUCGGUGCUUU U | 47 | mU*mU*mU*GGCAUUUAUUUCUA AAAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 79 |
| G012749 | UGUAUUUGUGAAGUCUUACAGUUU U AGAGCUAGAAAUAGCAAGUUAAAA U AAGGCUAGUCCGUUAUCAACUUGA A AAAGUGGCACCGAGUCGGUGCUUU U | 48 | mU*mG*mU*AUUUGUGAAGUCU UACAGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 80 |
| G012750 | UCCUAGGUAAAAAAAAAAAAAGUUU U AGAGCUAGAAAUAGCAAGUUAAAA U AAGGCUAGUCCGUUAUCAACUUGA A AAAGUGGCACCGAGUCGGUGCUUU U | 49 | mU*mC*mC*UAGGUAAAAAAAAA AAAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 81 |
| G012751 | UAAUUUCUUUGCGCACUAGUUU U AGAGCUAGAAAUAGCAAGUUAAAA U AAGGCUAGUCCGUUAUCAACUUGA A AAAGUGGCACCGAGUCGGUGCUUU U | 50 | mU*mA*mA*UUUUCUUUUGCGCA CUAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 82 |
| G012752 | UGACUGAAACUUCACAGAAUGUUU U AGAGCUAGAAAUAGCAAGUUAAAA U AAGGCUAGUCCGUUAUCAACUUGA A AAAGUGGCACCGAGUCGGUGCUUU U | 51 | mU*mG*mA*CUGAAACUUCACAG AAUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 83 |
| G012753 | GACUGAAACUUCACAGAAUAGUUU U AGAGCUAGAAAUAGCAAGUUAAAA U AAGGCUAGUCCGUUAUCAACUUGA A AAAGUGGCACCGAGUCGGUGCUUU U | 52 | mG*mA*mC*UGAAACUUCACAGA AUAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 84 |
| G012754 | UUCAUUUUAGUCUGUCUUCUGUUU U AGAGCUAGAAAUAGCAAGUUAAAA U AAGGCUAGUCCGUUAUCAACUUGA A AAAGUGGCACCGAGUCGGUGCUUU U | 53 | mU*mU*mC*AUUUUAGUCUGUCU UCUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 85 |
| G012755 | AUUAUCUAAGUUUGAAUAUAGUUU U AGAGCUAGAAAUAGCAAGUUAAAA U AAGGCUAGUCCGUUAUCAACUUGA A AAAGUGGCACCGAGUCGGUGCUUU U | 54 | mA*mU*mU*AUCUAAGUUUGAA UAUAGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 86 |
| G012756 | AAUUUUUAAAAUAGUAUUCUGUUU U AGAGCUAGAAAUAGCAAGUUAAAA U AAGGCUAGUCCGUUAUCAACUUGA A AAAGUGGCACCGAGUCGGUGCUUU U | 55 | mA*mA*mU*UUUUAAAAUAGUA UUCUGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 87 |
| G012757 | UGAAUUAUUCUUCUGUUUAAGUUU U AGAGCUAGAAAUAGCAAGUUAAAA U AAGGCUAGUCCGUUAUCAACUUGA A AAAGUGGCACCGAGUCGGUGCUUU U | 56 | mU*mG*mA*AUUAUUCUUCUGUU UAAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 88 |
| G012758 | AUCAUCCUGAGUUUUCUGUGUUU U AGAGCUAGAAAUAGCAAGUUAAAA U AAGGCUAGUCCGUUAUCAACUUGA A AAAGUGGCACCGAGUCGGUGCUUU U | 57 | mA*mU*mC*AUCCUGAGUUUUC UGUGUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 89 |
| G012759 | UUACUAAAACUUUAUUUUACGUUU U AGAGCUAGAAAUAGCAAGUUAAAA U AAGGCUAGUCCGUUAUCAACUUGA A AAAGUGGCACCGAGUCGGUGCUUU U | 58 | mU*mU*mA*CUAAAACUUUAUUU UACGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 90 |
| G012760 | ACCUUUUUUUUUUUUACCUGUUU U AGAGCUAGAAAUAGCAAGUUAAAA U AAGGCUAGUCCGUUAUCAACUUGA A AAAGUGGCACCGAGUCGGUGCUUU U | 59 | mA*mC*mC*UUUUUUUUUUUUA CCUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 91 |
| G012761 | AGUGCAAUGGAUAGGUCUUUGUUU U AGAGCUAGAAAUAGCAAGUUAAAA U AAGGCUAGUCCGUUAUCAACUUGA A AAAGUGGCACCGAGUCGGUGCUUU U | 60 | mA*mG*mU*GCAAUGGAUAGGUC UUUGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 92 |
| G012762 | UGAUUCCUACAGAAAAACUCGUUU U AGAGCUAGAAAUAGCAAGUUAAAA U AAGGCUAGUCCGUUAUCAACUUGA A AAAGUGGCACCGAGUCGGUGCUUU U | 61 | mU*mG*mA*UUCCUACAGAAAAA CUCGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 93 |
| G012763 | UGGGCAAGGGAAGAAAAAAAGUUU U AGAGCUAGAAAUAGCAAGUUAAAA U AAGGCUAGUCCGUUAUCAACUUGA A AAAGUGGCACCGAGUCGGUGCUUU U | 62 | mU*mG*mG*GCAAGGAAGAAA AAAAGUUUUAGAmGmCmUmAm GmAmAmAmUmAmGmCAAGUUA AAAUAAGGCUAGUCCGUUAUCA mAmCmUmUmGmAmAmAmAmAm GmUmGmGmCmAmCmCmGmAmG mUmCmGmGmUmGmCmU*mU*mU *mU | 94 |
| G012764 | CCUCACUCUUGUCUGGGCAAGUUU U AGAGCUAGAAAUAGCAAGUUAAAA U AAGGCUAGUCCGUUAUCAACUUGA A AAAGUGGCACCGAGUCGGUGCUUU U | 63 | mC*mC*mU*CACUCUUGUCUGGG CAAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 95 |
| G012765 | ACCUCACUCUUGUCUGGGCAGUUU U AGAGCUAGAAAUAGCAAGUUAAAA U AAGGCUAGUCCGUUAUCAACUUGA A AAAGUGGCACCGAGUCGGUGCUUU U | 64 | mA*mC*mC*UCACUCUUGUCUGG GCAGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 96 |
| G012766 | UGAGCAACCUCACUCUUGUCGUUU U AGAGCUAGAAAUAGCAAGUUAAAA U AAGGCUAGUCCGUUAUCAACUUGA A AAAGUGGCACCGAGUCGGUGCUUU U | 65 | mU*mG*mA*GCAACCUCACUCUU GUCGUUUUAGAmGmCmUmAmG mAmAmAmUmAmGmCAAGUUAA AAUAAGGCUAGUCCGUUAUCAm AmCmUmUmGmAmAmAmAmAmG mUmGmGmCmAmCmCmGmAmGm UmCmGmGmUmGmCmU*mU*mU* mU | 97 |

**Table 9. Mouse albumin guide sgRNA and modification pattern**

| **Guide ID** | **Full Sequence** | **SEQ ID NO:** | **Full Sequence Modified** | **SEQ ID NO:** |
|---|---|---|---|---|
| G000551 | AUUUGCAUCUGAGAACCCUU GUUUUAGAGCUAGAAAUAGC AAGUUAAAAUAAGGCUAGUC CGUUAUCAACUUGAAAAAGU GGCACCGAGUCGGUGCUUUU | 120 | mA*mU*mU*UGCAUCUGAGAACCCU UGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 142 |
| G000552 | AUCGGGAACUGGCAUCUUCA GUUUUAGAGCUAGAAAUAGC AAGUUAAAAUAAGGCUAGUC CGUUAUCAACUUGAAAAAGU GGCACCGAGUCGGUGCUUUU | 121 | mA*mU*mC*GGGAACUGGCAUCUUC AGUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 143 |
| G000553 | GUUACAGGAAAAUCUGAAGG GUUUUAGAGCUAGAAAUAGC AAGUUAAAAUAAGGCUAGUC CGUUAUCAACUUGAAAAAGU GGCACCGAGUCGGUGCUUUU | 122 | mG*mU*mU*ACAGGAAAAUCUGAAG GGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 144 |
| G000554 | GAUCGGGAACUGGCAUCUUC GUUUUAGAGCUAGAAAUAGC AAGUUAAAAUAAGGCUAGUC CGUUAUCAACUUGAAAAAGU GGCACCGAGUCGGUGCUUUU | 123 | mG*mA*mU*CGGGAACUGGCAUCUU CGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC | 145 |
| | | | mCmGmAmGmUmCmGmGmUmGmCm U*mU*mU*mU | |
| G000555 | UGCAUCUGAGAACCCUUAGG GUUUUAGAGCUAGAAAUAGC AAGUUAAAAUAAGGCUAGUC CGUUAUCAACUUGAAAAAGU GGCACCGAGUCGGUGCUUUU | 124 | mU*mG*mC*AUCUGAGAACCCUUAG GGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 146 |
| G000666 | CACUCUUGUCUGUGGAAACA GUUUUAGAGCUAGAAAUAGC AAGUUAAAAUAAGGCUAGUC CGUUAUCAACUUGAAAAAGU GGCACCGAGUCGGUGCUUUU | 125 | mC*mA*mC*UCUUGUCUGUGGAAAC AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 147 |
| G000667 | AUCGUUACAGGAAAAUCUGA GUUUUAGAGCUAGAAAUAGC AAGUUAAAAUAAGGCUAGUC CGUUAUCAACUUGAAAAAGU GGCACCGAGUCGGUGCUUUU | 126 | mA*mU*mC*GUUACAGGAAAAUCUG AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 148 |
| G000668 | GCAUCUUCAGGGAGUAGCUU GUUUUAGAGCUAGAAAUAGC AAGUUAAAAUAAGGCUAGUC CGUUAUCAACUUGAAAAAGU GGCACCGAGUCGGUGCUUUU | 127 | mG*mC*mA*UCUUCAGGGAGUAGCU UGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 149 |
| G000669 | CAAUCUUUAAAUAUGUUGUG GUUUUAGAGCUAGAAAUAGC AAGUUAAAAUAAGGCUAGUC CGUUAUCAACUUGAAAAAGU GGCACCGAGUCGGUGCUUUU | 128 | mC*mA*mA*UCUUUAAAUAUGUUGU GGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 150 |
| G000670 | UCACUCUUGUCUGUGGAAAC GUUUUAGAGCUAGAAAUAGC AAGUUAAAAUAAGGCUAGUC CGUUAUCAACUUGAAAAAGU GGCACCGAGUCGGUGCUUUU | 129 | mU*mC*mA*CUCUUGUCUGUGGAAA CGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 151 |
| G011722 | UGCUUGUAUUUUUCUAGUAA GUUUUAGAGCUAGAAAUAGC AAGUUAAAAUAAGGCUAGUC CGUUAUCAACUUGAAAAAGU GGCACCGAGUCGGUGCUUUU | 130 | mU*mG*mC*UUGUAUUUUCUAGUA AGUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 152 |
| G011723 | GUAAAUAUCUACUAAGACAA GUUUUAGAGCUAGAAAUAGC AAGUUAAAAUAAGGCUAGUC CGUUAUCAACUUGAAAAAGU GGCACCGAGUCGGUGCUUUU | 131 | mG*mU*mA*AAUAUCUACUAAGACA AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 153 |
| G011724 | UUUUUCUAGUAAUGGAAGCC GUUUUAGAGCUAGAAAUAGC AAGUUAAAAUAAGGCUAGUC CGUUAUCAACUUGAAAAAGU GGCACCGAGUCGGUGCUUUU | 132 | mU*mU*mU*UUCUAGUAAUGGAAGC CGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 154 |
| G011725 | UUAUAUUAUUGAUAUAUUUU GUUUUAGAGCUAGAAAUAGC AAGUUAAAAUAAGGCUAGUC CGUUAUCAACUUGAAAAAGU GGCACCGAGUCGGUGCUUUU | 133 | mU*mU*mA*UAUUAUUGAUAUAUUU UGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 155 |
| G011726 | GCACAGAUAUAAACACUUAA GUUUUAGAGCUAGAAAUAGC AAGUUAAAAUAAGGCUAGUC CGUUAUCAACUUGAAAAAGU GGCACCGAGUCGGUGCUUUU | 134 | mG*mC*mA*CAGAUAUAAACACUUA AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 156 |
| G011727 | CACAGAUAUAAACACUUAAC GUUUUAGAGCUAGAAAUAGC AAGUUAAAAUAAGGCUAGUC CGUUAUCAACUUGAAAAAGU GGCACCGAGUCGGUGCUUUU | 135 | mC*mA*mC*AGAUAUAAACACUUAA CGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 157 |
| G011728 | GGUUUUAAAAAUAAUAAUGU GUUUUAGAGCUAGAAAUAGC AAGUUAAAAUAAGGCUAGUC CGUUAUCAACUUGAAAAAGU GGCACCGAGUCGGUGCUUUU | 136 | mG*mG*mU*UUUAAAAUAAUAAUG UGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 158 |
| G011729 | UCAGAUUUUCCUGUAACGAU GUUUUAGAGCUAGAAAUAGC AAGUUAAAAUAAGGCUAGUC CGUUAUCAACUUGAAAAAGU GGCACCGAGUCGGUGCUUUU | 137 | mU*mC*mA*GAUUUCCUGUAACGA UGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 159 |
| G011730 | CAGAUUUUCCUGUAACGAUC GUUUUAGAGCUAGAAAUAGC AAGUUAAAAUAAGGCUAGUC CGUUAUCAACUUGAAAAAGU GGCACCGAGUCGGUGCUUUU | 138 | mC*mA*mG*AUUUCCUGUAACGAU CGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | 160 |
| G011731 | CAAUGGUAAAUAAGAAAUAA GUUUUAGAGCUAGAAAUAGC AAGUUAAAAUAAGGCUAGUC CGUUAUCAACUUGAAAAAGU GGCACCGAGUCGGUGCUUUU | | mC*mA*mA*UGGUAAAUAAGAAAUA AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | |
| G013018 | GGAAAAUCUGAAGGUGGCAA GUUUUAGAGCUAGAAAUAGC AAGUUAAAAUAAGGCUAGUC CGUUAUCAACUUGAAAAAGU GGCACCGAGUCGGUGCUUUU | | mG*mG*mA*AAAUCUGAAGGUGGCA AGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | |
| G013019 | GGCGAUCUCACUCUUGUCUG GUUUUAGAGCUAGAAAUAGC AAGUUAAAAUAAGGCUAGUC CGUUAUCAACUUGAAAAAGU GGCACCGAGUCGGUGCUUUU | | mG*mG*mC*GAUCUCACUCUUGUCU GGUUUUAGAmGmCmUmAmGmAmAm AmUmAmGmCAAGUUAAAAUAAGGC UAGUCCGUUAUCAmAmCmUmUmGm AmAmAmAmAmGmUmGmGmCmAmC mCmGmAmGmUmCmGmGmUmGmCm U*mU*mU*mU | |

**Table 10: Cyno sgRNA and modification patterns**

| **Guide ID** | **Full Sequence** | **SEQ ID NO:** | **Full Sequence Modified** | **SEQ ID NO:** |
|---|---|---|---|---|
| G009844 | GAGCAACCUCACUCUU GUCU GUUUUAGAGCUAGAA AUAGC AAGUUAAAAUAAGGC UAGUC CGUUAUCAACUUGAA AAAGU GGCACCGAGUCGGUGC UUUU | 197 | mG*mA*mG*CAACCUCACUCUUGUCUGU UUUAG AmGmCmUmAmGmAmAmAmUmAmGmCA AGUUA AAAUAAGGCUAGUCCGUUAUCAmAmCm UmUm GmAmAmAmAmAmGmUmGmGmCmAmCm CmGm AmGmUmCmGmGmUmGmCmU*mU*mU*m U | 230 |
| G009845 | AGCAACCUCACUCUUG UCUG GUUUUAGAGCUAGAA AUAGC AAGUUAAAAUAAGGC UAGUC CGUUAUCAACUUGAA AAAGU GGCACCGAGUCGGUGC UUUU | 198 | mA*mG*mC*AACCUCACUCUUGUCUGGU UUUAG AmGmCmUmAmGmAmAmAmUmAmGmCA AGUUA AAAUAAGGCUAGUCCGUUAUCAmAmCm UmUm GmAmAmAmAmAmGmUmGmGmCmAmCm CmGm AmGmUmCmGmGmUmGmCmU*mU*mU*m U | 231 |
| G009846 | ACCUCACUCUUGUCUG GGGA GUUUUAGAGCUAGAA AUAGC AAGUUAAAAUAAGGC UAGUC | 199 | mA*mC*mC*UCACUCUUGUCUGGGGAGU UUU AGAmGmCmUmAmGmAmAmAmUmAmGm CAA GUUAAAAUAAGGCUAGUCCGUUAUCAm AmCm | 232 |
| | CGUUAUCAACUUGAA AAAGU GGCACCGAGUCGGUGC UUUU | | UmUmGmAmAmAmAmAmGmUmGmGmCm AmCm CmGmAmGmUmCmGmGmUmGmCmU*mU* mU*mU | |
| G009847 | CCUCACUCUUGUCUGG GGAA GUUUUAGAGCUAGAA AUAGC AAGUUAAAAUAAGGC UAGUC CGUUAUCAACUUGAA AAAGU GGCACCGAGUCGGUGC UUUU | 200 | mC*mC*mU*CACUCUUGUCUGGGGAAGU UUUA GAmGmCmUmAmGmAmAmAmUmAmGmC AAGU UAAAAUAAGGCUAGUCCGUUAUCAmAm CmUm UmGmAmAmAmAmAmGmUmGmGmCmAm CmCm GmAmGmUmCmGmGmUmGmCmU*mU*mU *mU | 233 |
| G009848 | CUCACUCUUGUCUGGG GAAG GUUUUAGAGCUAGAA AUAGC AAGUUAAAAUAAGGC UAGUC CGUUAUCAACUUGAA AAAGU GGCACCGAGUCGGUGC UUUU | 201 | mC*mU*mC*ACUCUUGUCUGGGAAGGU UUU AGAmGmCmUmAmGmAmAmAmUmAmGm CAA GUUAAAAUAAGGCUAGUCCGUUAUCAm AmCm UmUmGmAmAmAmAmAmGmUmGmGmCm AmCm CmGmAmGmUmCmGmGmUmGmCmU*mU* mU*mU | 234 |
| G009849 | GGGGAAGGGGAGAAA AAAAA GUUUUAGAGCUAGAA AUAGC AAGUUAAAAUAAGGC UAGUC CGUUAUCAACUUGAA AAAGU GGCACCGAGUCGGUGC UUUU | 202 | mG*mG*mG*GAAGGGAGAAAAAAAG UUUUAG AmGmCmUmAmGmAmAmAmUmAmGmCA AGUUAA AAUAAGGCUAGUCCGUUAUCAmAmCmU mUmGm AmAmAmAmAmGmUmGmGmCmAmCmCm GmAmGm UmCmGmGmUmGmCmU*mU*mU*mU | 235 |
| G0098 50 | GGGAAGGGGAGAAAA AAAAA GUUUUAGAGCUAGAA AUAGC AAGUUAAAAUAAGGC UAGUC CGUUAUCAACUUGAA AAAGU GGCACCGAGUCGGUGC UUUU | 203 | mG*mG*mG*AAGGGAGAAAAAAAAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 23 6 |
| G0098 51 | AUGCAUUUGUUUCAA AAUAU GUUUUAGAGCUAGAA AUAGC AAGUUAAAAUAAGGC UAGUC CGUUAUCAACUUGAA AAAGU GGCACCGAGUCGGUGC UUUU | 204 | mA*mU*mG*CAUUUGUUUCAAAUAUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 23 7 |
| G0098 52 | UGCAUUUGUUUCAAA AUAUU | 205 | mU*mG*mC*AUUUGUUUCAAAUAUUGUU UUAG | 23 8 |
| | GUUUUAGAGCUAGAA AUAGC AAGUUAAAAUAAGGC UAGUC CGUUAUCAACUUGAA AAAGU GGCACCGAGUCGGUGC UUUU | | AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | |
| G0098 53 | UGAUUCCUACAGAAA AAGUC GUUUUAGAGCUAGAA AUAGC AAGUUAAAAUAAGGC UAGUC CGUUAUCAACUUGAA AAAGU GGCACCGAGUCGGUGC UUUU | 206 | mU*mG*mA*UUCCUACAGAAAAGUCGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 23 9 |
| G0098 54 | UACAGAAAAAGUCAG GAUAA GUUUUAGAGCUAGAA AUAGC AAGUUAAAAUAAGGC UAGUC CGUUAUCAACUUGAA AAAGU GGCACCGAGUCGGUGC UUUU | 207 | mU*mA*mC*AGAAAAAGUCAGGAUAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 0 |
| G0098 55 | UUUCUUCUGCCUUUAA ACAG GUUUUAGAGCUAGAA AUAGC AAGUUAAAAUAAGGC UAGUC CGUUAUCAACUUGAA AAAGU GGCACCGAGUCGGUGC UUUU | 208 | mU*mU*mU*CUUCUGCCUUUAAACAGGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 1 |
| G0098 56 | UUAUAGUUUUAUAUU CAAAC GUUUUAGAGCUAGAA AUAGC AAGUUAAAAUAAGGC UAGUC CGUUAUCAACUUGAA AAAGU GGCACCGAGUCGGUGC UUUU | 209 | mU*mU*mA*UAGUUUAUAUUCAAACGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 2 |
| G0098 57 | AUUUAUGAGAUCAAC AGCAC GUUUUAGAGCUAGAA AUAGC AAGUUAAAAUAAGGC UAGUC CGUUAUCAACUUGAA AAAGU | 210 | mA*mU*mU*UAUGAGAUCAACAGCACGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 3 |
| | GGCACCGAGUCGGUGC UUUU | | | |
| G0098 58 | GAUCAACAGCACAGGU UUUG GUUUUAGAGCUAGAA AUAGC AAGUUAAAAUAAGGC UAGUC CGUUAUCAACUUGAA AAAGU GGCACCGAGUCGGUGC UUUU | 211 | mG*mA*mU*CAACAGCACAGGUUUUGGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 4 |
| G0098 59 | UUAAAUAAAGCAUAG UGCAA GUUUUAGAGCUAGAA AUAGC AAGUUAAAAUAAGGC UAGUC CGUUAUCAACUUGAA AAAGU GGCACCGAGUCGGUGC UUUU | 212 | mU*mU*mA*AAUAAAGCAUAGUGCAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 5 |
| G0098 60 | UAAAGCAUAGUGCAA UGGAU GUUUUAGAGCUAGAA AUAGC AAGUUAAAAUAAGGC UAGUC CGUUAUCAACUUGAA AAAGU GGCACCGAGUCGGUGC UUUU | 213 | mU*mA*mA*AGCAUAGUGCAAUGGAUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 6 |
| G0098 61 | UAGUGCAAUGGAUAG GUCUU GUUUUAGAGCUAGAA AUAGC AAGUUAAAAUAAGGC UAGUC CGUUAUCAACUUGAA AAAGU GGCACCGAGUCGGUGC UUUU | 214 | mU*mA*mG*UGCAAUGGAUAGGUCUUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 7 |
| G0098 62 | AGUGCAAUGGAUAGG UCUUA GUUUUAGAGCUAGAA AUAGC AAGUUAAAAUAAGGC UAGUC CGUUAUCAACUUGAA AAAGU GGCACCGAGUCGGUGC UUUU | 215 | mA*mG*mU*GCAAUGGAUAGGUCUUAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 24 8 |
| G0098 63 | UUACUUUGCACUUUCC UUAG GUUUUAGAGCUAGAA AUAGC | 216 | mU*mU*mA*CUUUGCACUUUCCUUAGGUU UUAG AmGmCmUmAmGmAmAmAmUmAmGmCAAG UUAA | 24 9 |
| | AAGUUAAAAUAAGGC UAGUC CGUUAUCAACUUGAA AAAGU GGCACCGAGUCGGUGC UUUU | | AAUAAGGCUAGUCCGUUAUCAmAmCmUm UmGm AmAmAmAmAmGmUmGmGmCmAmCmCmGm AmGm UmCmGmGmUmGmCmU*mU*mU*mU | |
| G0098 64 | UACUUUGCACUUUCCU UAGU GUUUUAGAGCUAGAA AUAGC AAGUUAAAAUAAGGC UAGUC CGUUAUCAACUUGAA AAAGU GGCACCGAGUCGGUGC UUUU | 217 | mU*mA*mC*UUUGCACUUUCCUUAGUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 0 |
| G0098 65 | UCUGACCUUUUAUUU UACCU GUUUUAGAGCUAGAA AUAGC AAGUUAAAAUAAGGC UAGUC CGUUAUCAACUUGAA AAAGU GGCACCGAGUCGGUGC UUUU | 218 | mU*mC*mU*GACCUUUUAUUUUACCUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 1 |
| G0098 66 | UACUAAAACUUUAUU UUACU GUUUUAGAGCUAGAA AUAGC AAGUUAAAAUAAGGC UAGUC CGUUAUCAACUUGAA AAAGU GGCACCGAGUCGGUGC UUUU | 219 | mU*mA*mC*UAAAACUUUAUUUUACUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 2 |
| G0098 67 | AAAGUUGAACAAUAG AAAAA GUUUUAGAGCUAGAA AUAGC AAGUUAAAAUAAGGC UAGUC CGUUAUCAACUUGAA AAAGU GGCACCGAGUCGGUGC UUUU | 220 | mA*mA*mA*GUUGAACAAUAGAAAAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 3 |
| G0098 68 | AAUGCAUAAUCUAAG UCAAA GUUUUAGAGCUAGAA AUAGC AAGUUAAAAUAAGGC UAGUC CGUUAUCAACUUGAA AAAGU GGCACCGAGUCGGUGC UUUU | 221 | mA*mA*mU*GCAUAAUCUAAGUCAAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 4 |
| G0098 69 | AUUAUCCUGACUUUU UCUGU GUUUUAGAGCUAGAA AUAGC AAGUUAAAAUAAGGC UAGUC CGUUAUCAACUUGAA AAAGU GGCACCGAGUCGGUGC UUUU | 222 | mA*mU*mU*AUCCUGACUUUUCUGUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 5 |
| G0098 70 | UGAAUUAUUCCUCUG UUUAA GUUUUAGAGCUAGAA AUAGC AAGUUAAAAUAAGGC UAGUC CGUUAUCAACUUGAA AAAGU GGCACCGAGUCGGUGC UUUU | 223 | mU*mG*mA*AUUAUUCCUCUGUUUAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 6 |
| G0098 71 | UAAUUUCUUUGCCC ACUA GUUUUAGAGCUAGAA AUAGC AAGUUAAAAUAAGGC UAGUC CGUUAUCAACUUGAA AAAGU GGCACCGAGUCGGUGC UUUU | 224 | mU*mA*mA*UUUUCUUUUGCCCACUAGUU UUAG AmGmCmUmAmGmAmAmAmUmAmGmCAAG UUAA AAUAAGGCUAGUCCGUUAUCAmAmCmUm Um GmAmAmAmAmAmGmUmGmGmCmAmCmCm Gm AmGmUmCmGmGmUmGmCmU*mU*mU*mU | 25 7 |
| G0098 72 | AAAAGGUCAGAAUUG UUUAG GUUUUAGAGCUAGAA AUAGC AAGUUAAAAUAAGGC UAGUC CGUUAUCAACUUGAA AAAGU GGCACCGAGUCGGUGC UUUU | 225 | mA*mA*mA*AGGUCAGAAUUGUUUAGGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 8 |
| G0098 73 | AACAUCCUAGGUAAA AUAAA GUUUUAGAGCUAGAA AUAGC AAGUUAAAAUAAGGC UAGUC CGUUAUCAACUUGAA AAAGU GGCACCGAGUCGGUGC UUUU | 226 | mA*mA*mC*AUCCUAGGUAAAAUAAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 25 9 |
| G0098 74 | UAAUAAAAUUCAAAC AUCCU GUUUUAGAGCUAGAA AUAGC AAGUUAAAAUAAGGC UAGUC | 227 | mU*mA*mA*UAAAAUUCAAACAUCCUGUU UUAG AmGmCmUmAmGmAmAmAmUmAmGmCAAG UUAA AAUAAGGCUAGUCCGUUAUCAmAmCmUm UmGm | 26 0 |
| | CGUUAUCAACUUGAA AAAGU GGCACCGAGUCGGUGC UUUU | | AmAmAmAmAmGmUmGmGmCmAmCmCmGm AmGm UmCmGmGmUmGmCmU*mU*mU*mU | |
| G0098 75 | UUGUCAUGUAUUUCU AAAAU GUUUUAGAGCUAGAA AUAGC AAGUUAAAAUAAGGC UAGUC CGUUAUCAACUUGAA AAAGU GGCACCGAGUCGGUGC UUUU | 228 | mU*mU*mG*UCAUGUAUUUCUAAAAUGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 26 1 |
| G0098 76 | UUUGUCAUGUAUUUC UAAAA GUUUUAGAGCUAGAA AUAGC AAGUUAAAAUAAGGC UAGUC CGUUAUCAACUUGAA AAAGU GGCACCGAGUCGGUGC UUUU | 229 | mU*mU*mU*GUCAUGUAUUUCUAAAAGUU UUAG AmGmCmUmAmGmAmAmAmUmAmGmCAAG UUAA AAUAAGGCUAGUCCGUUAUCAmAmCmUm UmGm AmAmAmAmAmGmUmGmGmCmAmCmCmGm AmGm UmCmGmGmUmGmCmU*mU*mU*mU | 26 2 |

**Table 11: Vector Components and Sequences**

| Plasmid ID | 5' ITR | Splice Acceptor (1^{st} orientation) | Transgene (1^{st} orientation) | Poly-A (1^{st} orientation) | Poly-A (2^{nd} orientation) | Transgene (2^{nd} orientation) | Splice Acceptor (2^{nd} orientation) | 3' ITR |
|---|---|---|---|---|---|---|---|---|
| P00147 | (SEQ ID NO: 263) | Mouse Albumin Splice Acceptor (SEQ ID NO: 264) | Human Factor IX (R338L) (SEQ ID NO: 265) | SEQ ID NO: 266 | SEQ ID NO: 267 | Human Factor IX (R338L) (SEQ ID NO: 268) | Mouse Albumin Splice Acceptor (SEQ ID NO: 269) | (SEQ ID NO: 270) |
| P00411 | (SEQ ID NO: 263) | Human Factor IX Splice Acceptor (SEQ ID NO: 271) | Human Factor IX (R338L)-HiBit (SEQ ID NO: 272) | SEQ ID NO: 266 | SEQ ID NO: 267 | Human Factor IX (R338L)-HiBit (SEQ ID NO: 273) | Human Factor IX Splice Acceptor (SEQ ID NO: 274) | (SEQ ID NO: 270) |
| P00415 | (SEQ ID NO: 263) | Mouse Albumin Splice Acceptor (SEQ ID NO: 264) | Nluc-P2A-GFP (SEQ ID NO: 275) | SEQ ID NO: 266 | SEQ ID NO: 267 | Nluc-P2A-GFP (SEQ ID NO: 276) | Mouse Albumin Splice Acceptor (SEQ ID NO: 269) | (SEQ ID NO: 270) |
| P00418 | (SEQ ID NO: 263) | Mouse Albumin Splice Acceptor (SEQ ID NO: 264) | Human Factor IX (R338L)-HiBit (SEQ ID NO: 272) | SEQ ID NO: 266 | SEQ ID NO: 267 | Human Factor IX (R338L)-HiBit (SEQ ID NO: 273) | Mouse Albumin Splice Acceptor (SEQ ID NO: 269) | (SEQ ID NO: 270) |

Subject matter of this application is also disclosed in the following paragraphs that describe and/or define embodiments of the invention:
1. A bidirectional nucleic acid construct comprising:
   a) a first segment comprising a coding sequence for an agent such as a polypeptide; and
   b) a second segment comprising a reverse complement of a coding sequence of the agent,
   wherein the construct does not comprise a promoter that drives the expression of the agent such as a polypeptide.
2. A bidirectional nucleic acid construct comprising:
   a) a first segment comprising a coding sequence for a first agent such as a first polypeptide; and
   b) a second segment comprising a reverse complement of a coding sequence of a second agent such as a a second polypeptide,
   wherein the construct does not comprise a promoter that drives the expression of the agents(s).
3. The bidirectional nucleic acid construct of embodiment 1 or 2, wherein the second segment is 3' of the first segment.
4. The bidirectional nucleic acid construct of any one of embodiments 1-3, wherein the coding sequence of the reverse complement in the second segment adopts a different codon usage from that of the first coding sequence of the first segment.
5. The bidirectional nucleic acid construct of any one of embodiments 1-4, wherein the second segment comprises a nucleotide sequence having at least about 30%, about 35%, about 40%, about 45%, about 50%, about 55%, about 60%, about 65%, about 70%, about 75%, about 80%, about 85%, about 90%, about 95%, about 97%, or about 99% complementarity to the coding sequence in the first segment.
6. The bidirectional nucleic acid construct of any one of embodiments 1-5, wherein the coding sequence of the second segment encodes the polypeptide using one more alternative codons for one or more amino acids encoded by the coding sequence in the first segment.
7. The bidirectional nucleic acid construct of any one of embodiments 1-6, wherein the second segment comprises a reverse complement of the coding sequence of the first segment, or a fragment thereof.
8. The bidirectional nucleic acid construct of embodiment 7, wherein the reverse complement is:
   a. not substantially complementary to the coding sequence of the first segment;
   b. not substantially complementary to a fragment of the coding sequence of the first segment;
   c. highly complementary to the coding sequence of the first segment;
   d. highly complementary to a fragment of the coding sequence of the first segment;
   e. at least 60% identical to the reverse complement of the coding sequence of the first segment;
   f. at least 70% identical to the reverse complement of the coding sequence of the first segment;
   f. at least 90% identical to the reverse complement of the coding sequence of the first segment;
   g. 50-80% identical to the reverse complement of the coding sequence of the first segment; and/or
   h 60-100% identical to the reverse complement of the coding sequence of the first segment.
9. The bidirectional nucleic acid construct of any one of embodiments 1-8, wherein the construct does not comprise a homology arm.
10. The bidirectional nucleic acid construct of any one of embodiments 1-9, wherein the first segment is linked to the second segment by a linker.
11. The bidirectional nucleic acid construct embodiment 10, wherein the linker is 5, 10, 20, 30, 40, 50, 60, 70, 80, 90, 100, 150, 200, 250, 300, 500, 1000, 1500, 2000 nucleotides in length.
12. The bidirectional nucleic acid construct of any one of embodiments 1-11, wherein each of the first and second segment comprises a polyadenylation sequence, such as a polyadenylation signal sequence or a polyadenylation tail sequence.
13. The bidirectional nucleic acid construct of any of one embodiments 1-12, wherein the construct comprises a splice acceptor site.
14. The bidirectional nucleic acid construct of embodiment 13, wherein the construct comprises a first splice acceptor site 5' of the first segment and a second splice acceptor site 3' of the second segment.
15. The bidirectional nucleic acid construct of any one of embodiments 1-14, wherein the construct is double-stranded, optionally double-stranded DNA.
16. The bidirectional nucleic acid construct of any one of embodiments 1-15, wherein the construct is single-stranded, optionally single-stranded DNA.
17. The bidirectional nucleic acid construct of any one of embodiments 1-16, wherein a sequence encoding the polypeptide is codon-optimized.
18. The bidirectional nucleic acid construct of any one of embodiments 1-17, wherein the construct comprises one or more of the following terminal structures: hairpin, loops, inverted terminal repeats (ITR), or toroid.
19. The bidirectional nucleic acid construct of any one of embodiments 1-18, wherein the construct comprises one, two, or three inverted terminal repeats (ITR).
20. The bidirectional nucleic acid construct of any one of embodiments 1-19, wherein the construct comprises no more than two ITRs.
21. The bidirectional nucleic acid construct of any one of embodiments 1-20, wherein the polypeptide is a secreted polypeptide.
22. The bidirectional nucleic acid construct of any one of embodiments 1-20, wherein the polypeptide y is an intracellular polypeptide.
23. The bidirectional nucleic acid construct of any one embodiments 2-22, wherein the first polypeptide and the second polypeptide are different.
24. The bidirectional nucleic acid construct of any one of embodiments 1-23, wherein the polypeptide is a variant polypeptide.
25. The bidirectional nucleic acid construct of any one of embodiments 1-24, wherein the polypeptide is a liver protein.
26. The bidirectional nucleic acid construct of any one of embodiments 1-24, wherein the polypeptide is a non-liver protein.
27. The bidirectional nucleic acid construct of any one of embodiments 1-26, wherein the construct is a homology-independent construct.
28. The bidirectional nucleic acid construct any one of embodiments 1-27, wherein the polypeptide, when expressed, comprises a heterologous signal peptide.
29. The bidirectional nucleic acid construct any one of embodiments 1-28, wherein the nucleic acid encodes a heterologous signal peptide.
30. The bidirectional nucleic acid construct any one of embodiments 1-27, wherein the nucleic acid does not encode a signal peptide.
31. The bidirectional nucleic acid construct any one of embodiments 1-27, wherein the polypeptide, when expressed, comprises its own signal peptide.
32. The bidirectional nucleic acid construct of any one of embodiments 1-31, wherein the nucleic acid encodes a heterologous peptide.
33. The bidirectional nucleic acid construct of embodiment 32, wherein the heterologous peptide is 2A.
34. A vector comprising the construct of any one of embodiments 1-33.
35. The vector of embodiment 34, wherein the vector is an adeno-associated virus (AAV) vector.
36. The vector of embodiment 34 or 35, wherein the AAV comprises a single-stranded genome (ssAAV) or a self-complementary genome (scAAV).
37. The vector of embodiment 34 or 35, wherein the AAV vector is selected from the group consisting of AAV1, AAV2, AAV3, AAV3B, AAV4, AAV5, AAV6, AAV6.2, AAV7, AAVrh.64R1, AAVhu.37, AAVrh.8, AAVrh.32.33, AAV8, AAV9, AAV-DJ, AAV2/8, AAVrh10, AAVLK03, AV10, AAV11, AAV12, rh10, and hybrids thereof.
38. A viral vector comprising a self-complementary (or double-stranded) nucleic acid construct that comprises a nucleotide sequence encoding a polypeptide, wherein the vector does not comprise a promoter that drives the expression of the polypeptide.
39. The vector of embodiment 38, wherein the vector does not comprise a homology arm.
40. A lipid nanoparticle comprising the construct of any one of embodiments 1-33.
41. A host cell comprising the construct of any one of embodiments 1-33.
42. The host cell of embodiment 41, wherein the host cell is a liver cell, a muscle cell, or a neuronal cell.
43. The host cell of embodiment 41, wherein the host cell is a non-dividing cell type.
44. The host cell of any one of embodiments 41-43, wherein the host cell expresses the polypeptide encoded by the construct.
45. The host cell of any one of embodiments 41-44, wherein the host cell is a hepatocyte.
46. A method of modifying a target locus comprising providing a cell with a construct according to any one of embodiments 1-33, a vector according to any one of embodiments 34-39, or an LNP of embodiment 40.
47. A method of introducing a construct into a cell, comprising providing the cell with a construct according to any one of embodiments 1-33, a vector according to any one of embodiments 34-39, or an LNP of embodiment 40.
48. A method of expressing a polypeptide in a cell, comprising providing the cell with a construct of any one of embodiments 1-33, a vector according to any one of embodiments 34-39, or an LNP of embodiment 40.
49. A method of increasing expression of a polypeptide in a cell, comprising providing the cell with a construct of any one of embodiments 1-33, a vector according to any one of embodiments 34-39, or an LNP of embodiment 40.
50. The method of any one of embodiments 46-49, wherein the construct, vector, or LNP is provided in vivo.
51. The method of any one of embodiments 46-49, wherein the construct, vector, or LNP is provided in vitro.
52. An ex vivo method of modifying a target locus comprising providing a cell with a construct according to any one of embodiments 1-33, a vector according to any one of embodiments 34-39, or an LNP of embodiment 40.
53. The method of any of embodiments 46-52, wherein the cell is a non-dividing cell type.
54. The method of any of embodiments 46-53, further comprising providing the cell with a nuclease, wherein the nuclease is capable of introducing a site-specific ssDNA or dsDNA break.
55. The method of any one of embodiments 46-54, comprising providing the cell with an RNA-guided DNA-binding agent and a guide RNA (gRNA).
56. The method of embodiment 55, wherein the gRNA is a single gRNA (sgRNA).
57. The method of embodiment 55 or 56, wherein the construct, RNA-guided DNA-binding agent, and the gRNA are provided to the cell simultaneously.
58. The method of embodiment 55 or 56, wherein the construct, RNA-guided DNA-binding agent, and the gRNA are provided to the cell sequentially, in any order.
59. The method of embodiment 58, wherein the construct is provided to the cell prior to providing the gRNA and RNA-guided DNA-binding agent.
60. The method of embodiment 58, wherein the RNA-guided DNA-binding agent, or RNA-guided DNA-binding agent and gRNA in combination, is provided to the cell prior to providing the construct.
61. The method of any one of embodiments 54-60, wherein the construct is provided in a vector.
62. The method of any one of embodiments 54-60, wherein the construct is provided in a lipid nanoparticle.
63. The method of any one of embodiments 54-62, wherein the RNA-guided DNA-binding agent is provided as a nucleic acid encoding an RNA-guided DNA-binding agent.
64. The method of any one of embodiments 54-62, wherein the RNA-guided DNA-binding agent is provided as a Cas enzyme or an mRNA encoding an RNA-guided DNA-binding agent.
65. The method of any one of embodiments 54-62, wherein the RNA-guided DNA-binding agent is a Cas nuclease, such as a Cas9 nuclease.
66. The method of embodiment 65, wherein the Cas nuclease is a class 2 Cas nuclease, or a variant thereof.
67. The method of any one of embodiments 54-66, wherein the Cas nuclease is a S. *pyogenes* Cas9, or a variant thereof.
68. The method of any one of embodiments 54-67, wherein the Cas nuclease is a nickase.
69. The method of any one of embodiments 54-68, wherein the locus is an albumin locus.
70. The method of any one of embodiments 54-69, wherein the cell is a non-dividing cell type.
71. The method of any one of embodiments 54-70, wherein the cell is a liver cell, a neuronal cell, or a muscle cell.
72. A cell made by the method of any one of embodiments 46-71.
73. The cell of embodiment 72, wherein the cell is a host cell.
74. The host cell of embodiment 73, wherein the host cell is a liver cell, a muscle cell, or a neuronal cell.
75. The cell of any one of embodiments 72-74, wherein the host cell is a non-dividing cell type.
76. The cell of any one of embodiments 72-75, wherein the host cell expresses the polypeptide encoded by the construct.
77. The cell of any one of embodiments 72-76, wherein the host cell is a hepatocyte.
78. The bidirectional nucleic acid construct of any one of embodiments 1-33, wherein the agent is a therapeutic agent.
79. The bidirectional nucleic acid construct of any one of embodiments 1-33, wherein the agent is any one or more of a functional RNA, an mRNA, or a polypeptide.
80. The bidirectional nucleic acid construct of any one of embodiments 1-33, wherein the agent is a polypeptide.
81. The bidirectional nucleic acid construct of any one of embodiments 1-33, wherein the agent is a therapeutic polypeptide.
   Human albumin intron 1: (SEQ ID NO: 1) 
GTAAGAAATCCATTTTTCTATTGTTCAACTTTTATTCTATTTTCCCAGTAAAATAA
AGTTTTAGTAAACTCTGCATCTTTAAAGAATTATTTTGGCATTTATTTCTAAAATG
GCATAGTATTTTGTATTTGTGAAGTCTTACAAGGTTATCTTATTAATAAAATTCAA
ACATCCTAGGTAAAAAAAAAAAAAGGTCAGAATTGTTTAGTGACTGTAATTTTCT
TTTGCGCACTAAGGAAAGTGCAAAGTAACTTAGAGTGACTGAAACTTCACAGAA
TAGGGTTGAAGATTGAATTCATAACTATCCCAAAGACCTATCCATTGCACTATGC
TTTATTTAAAAACCACAAAACCTGTGCTGTTGATCTCATAAATAGAACTTGTATTT
ATATTTATTTTCATTTTAGTCTGTCTTCTTGGTTGCTGTTGATAGACACTAAAAGA
GTATTAGATATTATCTAAGTTTGAATATAAGGCTATAAATATTTAATAATTTTTAA
AATAGTATTCTTGGTAATTGAATTATTCTTCTGTTTAAAGGCAGAAGAAATAATT
GAACATCATCCTGAGTTTTTCTGTAGGAATCAGAGCCCAATATTTTGAAACAAAT
GCATAATCTAAGTCAAATGGAAAGAAATATAAAAAGTAACATTATTACTTCTTGT
TTTCTTCAGTATTTAACAATCCTTTTTTTTCTTCCCTTGCCCAG
   5' ITR Sequence (SEQ ID NO: 263): 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCT
   Mouse Albumin Splice Acceptor (1^{st} orientation) (SEQ ID NO: 264): 
TAGGTCAGTGAAGAGAAGAACAAAAAGCAGCATATTACAGTTAGTTGTCTTCAT
CAATCTTTAAATATGTTGTGTGGTTTTTCTCTCCCTGTTTCCACAG
   Human Factor IX (R338L), 1^{st} Orientation (SEQ ID NO: 265): 
TTTCTTGATCATGAAAACGCCAACAAAATTCTGAATCGGCCAAAGAGGTATAATT
CAGGTAAATTGGAAGAGTTTGTTCAAGGGAACCTTGAGAGAGAATGTATGGAAG
AAAAGTGTAGTTTTGAAGAAGCACGAGAAGTTTTTGAAAACACTGAAAGAACAA
CTGAATTTTGGAAGCAGTATGTTGATGGAGATCAGTGTGAGTCCAATCCATGTTT
AAATGGCGGCAGTTGCAAGGATGACATTAATTCCTATGAATGTTGGTGTCCCTTT
GGATTTGAAGGAAAGAACTGTGAATTAGATGTAACATGTAACATTAAGAATGGC
AGATGCGAGCAGTTTTGTAAAAATAGTGCTGATAACAAGGTGGTTTGCTCCTGTA
CTGAGGGATATCGACTTGCAGAAAACCAGAAGTCCTGTGAACCAGCAGTGCCAT
TTCCATGTGGAAGAGTTTCTGTTTCACAAACTTCTAAGCTCACCCGTGCTGAGAC
TGTTTTTCCTGATGTGGACTATGTAAATTCTACTGAAGCTGAAACCATTTTGGATA
ACATCACTCAAAGCACCCAATCATTTAATGACTTCACTCGGGTTGTTGGTGGAGA
AGATGCCAAACCAGGTCAATTCCCTTGGCAGGTTGTTTTGAATGGTAAAGTTGAT
GCATTCTGTGGAGGCTCTATCGTTAATGAAAAATGGATTGTAACTGCTGCCCACT
GTGTTGAAACTGGTGTTAAAATTACAGTTGTCGCAGGTGAACATAATATTGAGGA
GACAGAACATACAGAGCAAAAGCGAAATGTGATTCGAATTATTCCTCACCACAA
CTACAATGCAGCTATTAATAAGTACAACCATGACATTGCCCTTCTGGAACTGGAC
GAACCCTTAGTGCTAAACAGCTACGTTACACCTATTTGCATTGCTGACAAGGAAT
ACACGAACATCTTCCTCAAATTTGGATCTGGCTATGTAAGTGGCTGGGGAAGAGT
CTTCCACAAAGGGAGATCAGCTTTAGTTCTTCAGTACCTTAGAGTTCCACTTGTT
GACCGAGCCACATGTCTTCTATCTACAAAGTTCACCATCTATAACAACATGTTCT
GTGCTGGCTTCCATGAAGGAGGTAGAGATTCATGTCAAGGAGATAGTGGGGGAC
CCCATGTTACTGAAGTGGAAGGGACCAGTTTCTTAACTGGAATTATTAGCTGGGG
TGAAGAGTGTGCAATGAAAGGCAAATATGGAATATATACCAAGGTATCCCGGTA
TGTCAACTGGATTAAGGAAAAAACAAAGCTCACTTAA
   Poly-A (1^{st} orientation) (SEQ ID NO: 266): 
CCTCGACTGTGCCTTCTAGTTGCCAGCCATCTGTTGTTTGCCCCTCCCCCGTGCCT
TCCTTGACCCTGGAAGGTGCCACTCCCACTGTCCTTTCCTAATAAAATGAGGAAA
TTGCATCGCATTGTCTGAGTAGGTGTCATTCTATTCTGGGGGGTGGGGTGGGGCA
GGACAGCAAGGGGGAGGATTGGGAAGACAATAGCAGGCATGCTGGGGATGCGG
TGGGCTCTATGGCTTCTGAGGCGGAAAGAACCAGCTGGGGCTCTAGGGGGTATC
CCC
   Poly-A (2^{nd} orientation) (SEQ ID NO: 267): 
AAAAAACCTCCCACACCTCCCCCTGAACCTGAAACATAAAATGAATGCAATTGTT
GTTGTTAACTTGTTTATTGCAGCTTATAATGGTTACAAATAAAGCAATAGCATCA
CAAATTTCACAAATAAAGCATTTTTTTCACTGCATTCTAGTTGTGGTTTGTCCAAA
CTCATCAATGTATCTTATCATGTCTG
   Human Factor IX (R338L), 2^{nd} Orientation (SEQ ID NO: 268): 
TTAGGTGAGCTTAGTCTTTTCTTTTATCCAATTCACGTAGCGAGAGACCTTCGTAT
AGATGCCATATTTCCCCTTCATCGCACATTCCTCCCCCCAACTTATTATCCCGGTC
AAGAAACTTGTTCCTTCGACTTCAGTGACGTGTGGTCCACCTGAATCACCTTGGC
ATGAGTCGCGACCGCCCTCGTGAAACCCAGCACAAAACATGTTATTGTAAATCGT
AAATTTCGTGGACAGAAGACAGGTCGCTCTATCGACCAACGGGACGCGCAAATA
TTGCAGAACGAGGGCTGATCGACCTTTGTGGAAGACCCGCCCCCACCCACTCAC
ATATCCGCTCCCAAATTTCAAGAAGATATTTGTATATTCTTTATCGGCTATACAAA
TCGGGGTAACATAGGAGTTAAGTACGAGTGGCTCGTCCAGCTCCAGGAGGGCTA
TATCATGGTTGTACTTGTTTATAGCGGCATTATAATTGTGATGGGGTATGATCCTG
ATAACATTCCTTTTCTGTTCAGTATGCTCAGTTTCTTCAATGTTGTGTTCGCCAGC
CACGACCGTAATCTTAACCCCCGTCTCGACACAGTGTGCGGCCGTTACAATCCAC
TTTTCATTGACTATGGAGCCCCCACAAAACGCGTCGACTTTTCCGTTGAGCACCA
CCTGCCATGGAAATTGGCCAGGTTTAGCGTCCTCGCCCCCGACAACCCTAGTAAA
GTCATTAAATGACTGTGTGGATTGTGTTATATTATCAAGAATCGTTTCGGCTTCAG
TAGAGTTAACGTAGTCCACATCGGGAAAAACTGTCTCGGCCCTTGTCAACTTTGA
TGTCTGGGACACACTTACCCGACCGCACGGGAAGGGCACCGCCGGTTCACAGCT
CTTTTGATTCTCAGCGAGCCGGTAGCCCTCAGTGCAACTACACACAACTTTGTTG
TCGGCGGAATTTTTACAGAATTGCTCGCATCGTCCATTTTTAATGTTGCAGGTGAC
GTCCAACTCGCAGTTTTTTCCTTCAAAACCAAAAGGGCACCAACACTCGTAGGAA
TTTATATCGTCTTTACAACTCCCCCCATTCAGACATGGATTAGATTCGCATTGGTC
CCCATCGACATATTGCTTCCAGAACTCAGTGGTCCGTTCTGTATTCTCAAACACCT
CGCGCGCTTCTTCAAAACTGCATTTTTCCTCCATACACTCTCGCTCCAAGTTCCCT
TGCACGAATTCTTCAAGCTTTCCTGAGTTATACCTTTTAGGCCGGTTAAGTATCTT
ATTCGCGTTTTCGTGGTCCAGAAA
   Mouse Albumin Splice Acceptor (2^{nd} orientation) (SEQ ID NO: 269): 
CTGTGGAAACAGGGAGAGAAAAACCACACAACATATTTAAAGATTGATGAAGAC
AACTAACTGTAATATGCTGCTTTTTGTTCTTCTCTTCACTGACCTA
   3' ITR Sequence (2^{nd} orientation) (SEQ ID NO: 270): 
AGGAACCCCTAGTGATGGAGTTGGCCACTCCCTCTCTGCGCGCTCGCTCGCTCAC
TGAGGCCGCCCGGGCAAAGCCCGGGCGTCGGGCGACCTTTGGTCGCCCGGCCTC
AGTGAGCGAGCGAGCGCGCAGAGAGGGAGTGGCCAA
   Human Factor IX Splice Acceptor (1^{st} Orientation) (SEQ ID NO: 271): 
GATTATTTGGATTAAAAACAAAGACTTTCTTAAGAGATGTAAAATTTTCATGATG
TTTTCTTTTTTGCTAAAACTAAAGAATTATTCTTTTACATTTCAG
   Human Factor IX (R338L)-HiBit (1^{st} Orientation) (SEQ ID NO: 272): 
TTTCTTGATCATGAAAACGCCAACAAAATTCTGAATCGGCCAAAGAGGTATAATT
CAGGTAAATTGGAAGAGTTTGTTCAAGGGAACCTTGAGAGAGAATGTATGGAAG
AAAAGTGTAGTTTTGAAGAAGCACGAGAAGTTTTTGAAAACACTGAAAGAACAA
CTGAATTTTGGAAGCAGTATGTTGATGGAGATCAGTGTGAGTCCAATCCATGTTT
AAATGGCGGCAGTTGCAAGGATGACATTAATTCCTATGAATGTTGGTGTCCCTTT
GGATTTGAAGGAAAGAACTGTGAATTAGATGTAACATGTAACATTAAGAATGGC
AGATGCGAGCAGTTTTGTAAAAATAGTGCTGATAACAAGGTGGTTTGCTCCTGTA
CTGAGGGATATCGACTTGCAGAAAACCAGAAGTCCTGTGAACCAGCAGTGCCAT
TTCCATGTGGAAGAGTTTCTGTTTCACAAACTTCTAAGCTCACCCGTGCTGAGAC
TGTTTTTCCTGATGTGGACTATGTAAATTCTACTGAAGCTGAAACCATTTTGGATA
 
ACATCACTCAAAGCACCCAATCATTTAATGACTTCACTCGGGTTGTTGGTGGAGA
AGATGCCAAACCAGGTCAATTCCCTTGGCAGGTTGTTTTGAATGGTAAAGTTGAT
GCATTCTGTGGAGGCTCTATCGTTAATGAAAAATGGATTGTAACTGCTGCCCACT
GTGTTGAAACTGGTGTTAAAATTACAGTTGTCGCAGGTGAACATAATATTGAGGA
GACAGAACATACAGAGCAAAAGCGAAATGTGATTCGAATTATTCCTCACCACAA
CTACAATGCAGCTATTAATAAGTACAACCATGACATTGCCCTTCTGGAACTGGAC
GAACCCTTAGTGCTAAACAGCTACGTTACACCTATTTGCATTGCTGACAAGGAAT
ACACGAACATCTTCCTCAAATTTGGATCTGGCTATGTAAGTGGCTGGGGAAGAGT
CTTCCACAAAGGGAGATCAGCTTTAGTTCTTCAGTACCTTAGAGTTCCACTTGTT
GACCGAGCCACATGTCTTCTATCTACAAAGTTCACCATCTATAACAACATGTTCT
GTGCTGGCTTCCATGAAGGAGGTAGAGATTCATGTCAAGGAGATAGTGGGGGAC
CCCATGTTACTGAAGTGGAAGGGACCAGTTTCTTAACTGGAATTATTAGCTGGGG
TGAAGAGTGTGCAATGAAAGGCAAATATGGAATATATACCAAGGTCTCCCGGTA
TGTCAACTGGATTAAGGAAAAAACAAAGCTCACTGTCAGCGGATGGAGACTGTT
CAAGAAGATCAGCTAA
   Human Factor IX (R338L)-HiBit (2^{nd} Orientation) (SEQ ID NO: 273): 
TTAGGAAATCTTCTTAAACAGCCGCCAGCCGCTCACGGTGAGCTTAGTCTTTTCT
TTTATCCAATTCACGTAGCGAGAGACCTTCGTATAGATGCCATATTTCCCCTTCAT
CGCACATTCCTCCCCCCAACTTATTATCCCGGTCAAGAAACTTGTTCCTTCGACTT
CAGTGACGTGTGGTCCACCTGAATCACCTTGGCATGAGTCGCGACCGCCCTCGTG
AAACCCAGCACAAAACATGTTATTGTAAATCGTAAATTTCGTGGACAGAAGACA
GGTCGCTCTATCGACCAACGGGACGCGCAAATATTGCAGAACGAGGGCTGATCG
ACCTTTGTGGAAGACCCGCCCCCACCCACTCACATATCCGCTCCCAAATTTCAAG
AAGATATTTGTATATTCTTTATCGGCTATACAAATCGGGGTAACATAGGAGTTAA
GTACGAGTGGCTCGTCCAGCTCCAGGAGGGCTATATCATGGTTGTACTTGTTTAT
AGCGGCATTATAATTGTGATGGGGTATGATCCTGATAACATTCCTTTTCTGTTCAG
TATGCTCAGTTTCTTCAATGTTGTGTTCGCCAGCCACGACCGTAATCTTAACCCCC
GTCTCGACACAGTGTGCGGCCGTTACAATCCACTTTTCATTGACTATGGAGCCCC
CACAAAACGCGTCGACTTTTCCGTTGAGCACCACCTGCCATGGAAATTGGCCAGG
TTTAGCGTCCTCGCCCCCGACAACCCTAGTAAAGTCATTAAATGACTGTGTGGAT
TGTGTTATATTATCAAGAATCGTTTCGGCTTCAGTAGAGTTAACGTAGTCCACAT
CGGGAAAAACTGTCTCGGCCCTTGTCAACTTTGATGTCTGGGACACACTTACCCG
ACCGCACGGGAAGGGCACCGCCGGTTCACAGCTCTTTTGATTCTCAGCGAGCCG
GTAGCCCTCAGTGCAACTACACACAACTTTGTTGTCGGCGGAATTTTTACAGAAT
TGCTCGCATCGTCCATTTTTAATGTTGCAGGTGACGTCCAACTCGCAGTTTTTTCC
TTCAAAACCAAAAGGGCACCAACACTCGTAGGAATTTATATCGTCTTTACAACTC
CCCCCATTCAGACATGGATTAGATTCGCATTGGTCCCCATCGACATATTGCTTCC
AGAACTCAGTGGTCCGTTCTGTATTCTCAAACACCTCGCGCGCTTCTTCAAAACT
GCATTTTTCCTCCATACACTCTCGCTCCAAGTTCCCTTGCACGAATTCTTCAAGCT
TTCCTGAGTTATACCTTTTAGGCCGGTTAAGTATCTTATTCGCGTTTTCGTGGTCC
AGAAA
   Human Factor IX Splice Acceptor (2^{nd} Orientation) (SEQ ID NO: 274): 
CTGAAATGTAAAAGAATAATTCTTTAGTTTTAGCAAAAAAGAAAACATCATGAA
AATTTTACATCTCTTAAGAAAGTCTTTGTTTTTAATCCAAATAATC
   Nluc-P2A-GFP (1^{st} Orientation) (SEQ ID NO: 275): 
TTTCTTGATCATGAAAACGCCAACAAAATTCTGAATCGGCCAAAGAGGTATAATT
CAGGTAAATTGGAAGAGTTTGTTCAAGGGAACCTTGAGAGAGAATGTATGGAAG
 
AAAAGTGTAGTTTTGAAGAAGCAGTATTCACTTTGGAGGACTTTGTCGGTGACTG
GAGGCAAACCGCTGGTTATAATCTCGACCAAGTACTGGAACAGGGCGGGGTAAG
TTCCCTCTTTCAGAATTTGGGTGTAAGCGTCACACCAATCCAGCGGATTGTGTTG
TCTGGAGAGAACGGACTCAAAATTGACATCCATGTTATCATTCCATATGAAGGTC
TCAGTGGAGACCAAATGGGGCAGATCGAGAAGATTTTCAAGGTAGTTTACCCAG
TCGACGATCACCACTTCAAAGTCATTCTCCACTATGGCACACTTGTTATCGACGG
AGTAACTCCTAATATGATTGATTACTTTGGTCGCCCGTATGAGGGCATCGCAGTG
TTTGATGGCAAAAAGATCACCGTAACAGGAACGTTGTGGAATGGGAACAAGATA
ATCGACGAGAGATTGATAAATCCAGACGGGTCACTCCTGTTCAGGGTTACAATTA
ACGGCGTCACAGGATGGAGACTCTGTGAACGAATACTGGCCACAAATTTTTCACT
CCTGAAGCAGGCCGGAGACGTGGAGGAAAACCCAGGGCCCGTGAGCAAGGGCG
AGGAGCTGTTCACCGGGGTGGTGCCCATCCTGGTCGAGCTGGACGGCGACGTAA
ACGGCCACAAGTTCAGCGTGTCCGGCGAGGGCGAGGGCGATGCCACCTACGGCA
AGCTGACCCTGAAGTTCATCTGCACCACCGGCAAGCTGCCCGTGCCCTGGCCCAC
CCTCGTGACCACCCTGACCTACGGCGTGCAGTGCTTCAGCCGCTACCCCGACCAC
ATGAAGCAGCACGACTTCTTCAAGTCCGCCATGCCCGAAGGCTACGTCCAGGAG
CGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAG
GAGGACGGCAACATCCTGGGGCACAAGCTGGAGTACAACTACAACAGCCACAAC
GTCTATATCATGGCCGACAAGCAGAAGAACGGCATCAAGGTGAACTTCAAGATC
CGCCACAACATCGAGGACGGCAGCGTGCAGCTCGCCGACCACTACCAGCAGAAC
ACCCCCATCGGCGACGGCCCCGTGCTGCTGCCCGACAACCACTACCTGAGCACCC
AGTCCGCCCTGAGCAAAGACCCCAACGAGAAGCGCGATCACATGGTCCTGCTGG
AGTTCGTGACCGCCGCCGGGATCACTCTCGGCATGGACGAGCTGTACAAGGGAG
GAGGAAGCCCGAAGAAGAAGAGAAAGGTCTAA
   Nluc-P2A-GFP (2^{nd} Orientation) (SEQ ID NO: 276): 
TTACACCTTCCTCTTCTTGGGGCTGCCGCCGCCCTTGTACAGCTCGTCCATGC
CCAGGGTGATGCCGGCGGTCACGAACTCCAGCAGCACCATGTGGTCCCTCTT
CTCGTTGGGGTCCTTGCTCAGGGCGCTCTGGGTGCTCAGGTAGTGGTTGTCGGGC
AGCAGCACGGGGCCGTCGCCGATGGGGGTGTTCTGCTGGTAGTGGTCGGCCAGC
TGCACGCTGCCGTCCTCGATGTTGTGCCTGATCTTGAAGTTCACCTTGATGCCGTT
CTTCTGCTTGTCGGCCATGATGTACACGTTGTGGCTGTTGTAGTTGTACTCCAGCT
TGTGGCCCAGGATGTTGCCGTCCTCCTTGAAGTCGATGCCCTTCAGCTCGATCCT
GTTCACCAGGGTGTCGCCCTCGAACTTCACCTCGGCCCTGGTCTTGTAGTTGCCG
TCGTCCTTGAAGAAGATGGTCCTCTCCTGCACGTAGCCCTCGGGCATGGCGCTCT
TGAAGAAGTCGTGCTGCTTCATGTGGTCGGGGTACCTGCTGAAGCACTGCACGCC
GTAGGTCAGGGTGGTCACCAGGGTGGGCCAGGGCACGGGCAGCTTGCCGGTGGT
GCAGATGAACTTCAGGGTCAGCTTGCCGTAGGTGGCGTCGCCCTCGCCCTCGCCG
CTCACGCTGAACTTGTGGCCGTTCACGTCGCCGTCCAGCTCCACCAGGATGGGCA
CCACGCCGGTGAACAGCTCCTCGCCCTTGCTCACGGGGCCGGGGTTCTCCTCCAC
GTCGCCGGCCTGCTTCAGCAGGCTGAAGTTGGTGGCCAGGATCCTCTCGCACAGC
CTCCAGCCGGTCACGCCGTTGATGGTCACCCTGAACAGCAGGCTGCCGTCGGGGT
TGATCAGCCTCTCGTCGATGATCTTGTTGCCGTTCCACAGGGTGCCGGTCACGGT
GATCTTCTTGCCGTCGAACACGGCGATGCCCTCGTAGGGCCTGCCGAAGTAGTCG
ATCATGTTGGGGGTCACGCCGTCGATCACCAGGGTGCCGTAGTGCAGGATCACCT
TGAAGTGGTGGTCGTCCACGGGGTACACCACCTTGAAAATCTTCTCGATCTGGCC
CATCTGGTCGCCGCTCAGGCCCTCGTAGGGGATGATCACGTGGATGTCGATCTTC
AGGCCGTTCTCGCCGCTCAGCACGATCCTCTGGATGGGGGTCACGCTCACGCCCA
GGTTCTGGAACAGGCTGCTCACGCCGCCCTGCTCCAGCACCTGGTCCAGGTTGTA
 
GCCGGCGGTCTGCCTCCAGTCGCCCACGAAGTCCTCCAGGGTGAACACGGCCTCC
TCGAAGCTGCACTTCTCCTCCATGCACTCCCTCTCCAGGTTGCCCTGCACGAACTC
CTCCAGCTTGCCGCTGTTGTACCTCTTGGGCCTGTTCAGGATCTTGTTGGCGTTCT
CGTGGTCCAGGAA
   P00147 full sequence (from ITR to ITR): (SEQ ID NO: 277) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTCTTAGGTCAGTG
AAGAGAAGAACAAAAAGCAGCATATTACAGTTAGTTGTCTTCATCAATCTTTAAA
TATGTTGTGTGGTTTTTCTCTCCCTGTTTCCACAGTTTTTCTTGATCATGAAAACGC
CAACAAAATTCTGAATCGGCCAAAGAGGTATAATTCAGGTAAATTGGAAGAGTT
TGTTCAAGGGAACCTTGAGAGAGAATGTATGGAAGAAAAGTGTAGTTTTGAAGA
AGCACGAGAAGTTTTTGAAAACACTGAAAGAACAACTGAATTTTGGAAGCAGTA
TGTTGATGGAGATCAGTGTGAGTCCAATCCATGTTTAAATGGCGGCAGTTGCAAG
GATGACATTAATTCCTATGAATGTTGGTGTCCCTTTGGATTTGAAGGAAAGAACT
GTGAATTAGATGTAACATGTAACATTAAGAATGGCAGATGCGAGCAGTTTTGTA
AAAATAGTGCTGATAACAAGGTGGTTTGCTCCTGTACTGAGGGATATCGACTTGC
AGAAAACCAGAAGTCCTGTGAACCAGCAGTGCCATTTCCATGTGGAAGAGTTTC
TGTTTCACAAACTTCTAAGCTCACCCGTGCTGAGACTGTTTTTCCTGATGTGGACT
ATGTAAATTCTACTGAAGCTGAAACCATTTTGGATAACATCACTCAAAGCACCCA
ATCATTTAATGACTTCACTCGGGTTGTTGGTGGAGAAGATGCCAAACCAGGTCAA
TTCCCTTGGCAGGTTGTTTTGAATGGTAAAGTTGATGCATTCTGTGGAGGCTCTAT
CGTTAATGAAAAATGGATTGTAACTGCTGCCCACTGTGTTGAAACTGGTGTTAAA
ATTACAGTTGTCGCAGGTGAACATAATATTGAGGAGACAGAACATACAGAGCAA
AAGCGAAATGTGATTCGAATTATTCCTCACCACAACTACAATGCAGCTATTAATA
AGTACAACCATGACATTGCCCTTCTGGAACTGGACGAACCCTTAGTGCTAAACAG
CTACGTTACACCTATTTGCATTGCTGACAAGGAATACACGAACATCTTCCTCAAA
TTTGGATCTGGCTATGTAAGTGGCTGGGGAAGAGTCTTCCACAAAGGGAGATCA
GCTTTAGTTCTTCAGTACCTTAGAGTTCCACTTGTTGACCGAGCCACATGTCTTCT
ATCTACAAAGTTCACCATCTATAACAACATGTTCTGTGCTGGCTTCCATGAAGGA
GGTAGAGATTCATGTCAAGGAGATAGTGGGGGACCCCATGTTACTGAAGTGGAA
GGGACCAGTTTCTTAACTGGAATTATTAGCTGGGGTGAAGAGTGTGCAATGAAA
GGCAAATATGGAATATATACCAAGGTATCCCGGTATGTCAACTGGATTAAGGAA
AAAACAAAGCTCACTTAACCTCGACTGTGCCTTCTAGTTGCCAGCCATCTGTTGT
TTGCCCCTCCCCCGTGCCTTCCTTGACCCTGGAAGGTGCCACTCCCACTGTCCTTT
CCTAATAAAATGAGGAAATTGCATCGCATTGTCTGAGTAGGTGTCATTCTATTCT
GGGGGGTGGGGTGGGGCAGGACAGCAAGGGGGAGGATTGGGAAGACAATAGCA
GGCATGCTGGGGATGCGGTGGGCTCTATGGCTTCTGAGGCGGAAAGAACCAGCT
GGGGCTCTAGGGGGTATCCCCAAAAAACCTCCCACACCTCCCCCTGAACCTGAA
ACATAAAATGAATGCAATTGTTGTTGTTAACTTGTTTATTGCAGCTTATAATGGTT
ACAAATAAAGCAATAGCATCACAAATTTCACAAATAAAGCATTTTTTTCACTGCA
TTCTAGTTGTGGTTTGTCCAAACTCATCAATGTATCTTATCATGTCTGTTAGGTGA
GCTTAGTCTTTTCTTTTATCCAATTCACGTAGCGAGAGACCTTCGTATAGATGCCA
TATTTCCCCTTCATCGCACATTCCTCCCCCCAACTTATTATCCCGGTCAAGAAACT
TGTTCCTTCGACTTCAGTGACGTGTGGTCCACCTGAATCACCTTGGCATGAGTCG
CGACCGCCCTCGTGAAACCCAGCACAAAACATGTTATTGTAAATCGTAAATTTCG
TGGACAGAAGACAGGTCGCTCTATCGACCAACGGGACGCGCAAATATTGCAGAA
CGAGGGCTGATCGACCTTTGTGGAAGACCCGCCCCCACCCACTCACATATCCGCT
 
CCCAAATTTCAAGAAGATATTTGTATATTCTTTATCGGCTATACAAATCGGGGTA
ACATAGGAGTTAAGTACGAGTGGCTCGTCCAGCTCCAGGAGGGCTATATCATGG
TTGTACTTGTTTATAGCGGCATTATAATTGTGATGGGGTATGATCCTGATAACATT
CCTTTTCTGTTCAGTATGCTCAGTTTCTTCAATGTTGTGTTCGCCAGCCACGACCG
TAATCTTAACCCCCGTCTCGACACAGTGTGCGGCCGTTACAATCCACTTTTCATTG
ACTATGGAGCCCCCACAAAACGCGTCGACTTTTCCGTTGAGCACCACCTGCCATG
GAAATTGGCCAGGTTTAGCGTCCTCGCCCCCGACAACCCTAGTAAAGTCATTAAA
TGACTGTGTGGATTGTGTTATATTATCAAGAATCGTTTCGGCTTCAGTAGAGTTA
ACGTAGTCCACATCGGGAAAAACTGTCTCGGCCCTTGTCAACTTTGATGTCTGGG
ACACACTTACCCGACCGCACGGGAAGGGCACCGCCGGTTCACAGCTCTTTTGATT
CTCAGCGAGCCGGTAGCCCTCAGTGCAACTACACACAACTTTGTTGTCGGCGGAA
TTTTTACAGAATTGCTCGCATCGTCCATTTTTAATGTTGCAGGTGACGTCCAACTC
GCAGTTTTTTCCTTCAAAACCAAAAGGGCACCAACACTCGTAGGAATTTATATCG
TCTTTACAACTCCCCCCATTCAGACATGGATTAGATTCGCATTGGTCCCCATCGA
CATATTGCTTCCAGAACTCAGTGGTCCGTTCTGTATTCTCAAACACCTCGCGCGCT
TCTTCAAAACTGCATTTTTCCTCCATACACTCTCGCTCCAAGTTCCCTTGCACGAA
TTCTTCAAGCTTTCCTGAGTTATACCTTTTAGGCCGGTTAAGTATCTTATTCGCGT
TTTCGTGGTCCAGAAAAACTGTGGAAACAGGGAGAGAAAAACCACACAACATAT
TTAAAGATTGATGAAGACAACTAACTGTAATATGCTGCTTTTTGTTCTTCTCTTCA
CTGACCTAAGAGATCTAGGAACCCCTAGTGATGGAGTTGGCCACTCCCTCTCTGC
GCGCTCGCTCGCTCACTGAGGCCGCCCGGGCAAAGCCCGGGCGTCGGGCGACCT
TTGGTCGCCCGGCCTCAGTGAGCGAGCGAGCGCGCAGAGAGGGAGTGGCCAA
   P00411 full sequence (form ITR to ITR): (SEQ ID NO: 278) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTCTGATTATTTGGA
TTAAAAACAAAGACTTTCTTAAGAGATGTAAAATTTTCATGATGTTTTCTTTTTTG
CTAAAACTAAAGAATTATTCTTTTACATTTCAGTTTTTCTTGATCATGAAAACGCC
AACAAAATTCTGAATCGGCCAAAGAGGTATAATTCAGGTAAATTGGAAGAGTTT
GTTCAAGGGAACCTTGAGAGAGAATGTATGGAAGAAAAGTGTAGTTTTGAAGAA
GCACGAGAAGTTTTTGAAAACACTGAAAGAACAACTGAATTTTGGAAGCAGTAT
GTTGATGGAGATCAGTGTGAGTCCAATCCATGTTTAAATGGCGGCAGTTGCAAGG
ATGACATTAATTCCTATGAATGTTGGTGTCCCTTTGGATTTGAAGGAAAGAACTG
TGAATTAGATGTAACATGTAACATTAAGAATGGCAGATGCGAGCAGTTTTGTAA
AAATAGTGCTGATAACAAGGTGGTTTGCTCCTGTACTGAGGGATATCGACTTGCA
GAAAACCAGAAGTCCTGTGAACCAGCAGTGCCATTTCCATGTGGAAGAGTTTCT
GTTTCACAAACTTCTAAGCTCACCCGTGCTGAGACTGTTTTTCCTGATGTGGACTA
TGTAAATTCTACTGAAGCTGAAACCATTTTGGATAACATCACTCAAAGCACCCAA
TCATTTAATGACTTCACTCGGGTTGTTGGTGGAGAAGATGCCAAACCAGGTCAAT
TCCCTTGGCAGGTTGTTTTGAATGGTAAAGTTGATGCATTCTGTGGAGGCTCTATC
GTTAATGAAAAATGGATTGTAACTGCTGCCCACTGTGTTGAAACTGGTGTTAAAA
TTACAGTTGTCGCAGGTGAACATAATATTGAGGAGACAGAACATACAGAGCAAA
AGCGAAATGTGATTCGAATTATTCCTCACCACAACTACAATGCAGCTATTAATAA
GTACAACCATGACATTGCCCTTCTGGAACTGGACGAACCCTTAGTGCTAAACAGC
TACGTTACACCTATTTGCATTGCTGACAAGGAATACACGAACATCTTCCTCAAAT
TTGGATCTGGCTATGTAAGTGGCTGGGGAAGAGTCTTCCACAAAGGGAGATCAG
CTTTAGTTCTTCAGTACCTTAGAGTTCCACTTGTTGACCGAGCCACATGTCTTCTA
TCTACAAAGTTCACCATCTATAACAACATGTTCTGTGCTGGCTTCCATGAAGGAG
GTAGAGATTCATGTCAAGGAGATAGTGGGGGACCCCATGTTACTGAAGTGGAAG
 
GGACCAGTTTCTTAACTGGAATTATTAGCTGGGGTGAAGAGTGTGCAATGAAAG
GCAAATATGGAATATATACCAAGGTCTCCCGGTATGTCAACTGGATTAAGGAAA
AAACAAAGCTCACTGTCAGCGGATGGAGACTGTTCAAGAAGATCAGCTAACCTC
GACTGTGCCTTCTAGTTGCCAGCCATCTGTTGTTTGCCCCTCCCCCGTGCCTTCCT
TGACCCTGGAAGGTGCCACTCCCACTGTCCTTTCCTAATAAAATGAGGAAATTGC
ATCGCATTGTCTGAGTAGGTGTCATTCTATTCTGGGGGGTGGGGTGGGGCAGGAC
AGCAAGGGGGAGGATTGGGAAGACAATAGCAGGCATGCTGGGGATGCGGTGGG
CTCTATGGCTTCTGAGGCGGAAAGAACCAGCTGGGGCTCTAGGGGGTATCCCCA
AAAAACCTCCCACACCTCCCCCTGAACCTGAAACATAAAATGAATGCAATTGTTG
TTGTTAACTTGTTTATTGCAGCTTATAATGGTTACAAATAAAGCAATAGCATCAC
AAATTTCACAAATAAAGCATTTTTTTCACTGCATTCTAGTTGTGGTTTGTCCAAAC
TCATCAATGTATCTTATCATGTCTGTTAGGAAATCTTCTTAAACAGCCGCCAGCC
GCTCACGGTGAGCTTAGTCTTTTCTTTTATCCAATTCACGTAGCGAGAGACCTTCG
TATAGATGCCATATTTCCCCTTCATCGCACATTCCTCCCCCCAACTTATTATCCCG
GTCAAGAAACTTGTTCCTTCGACTTCAGTGACGTGTGGTCCACCTGAATCACCTT
GGCATGAGTCGCGACCGCCCTCGTGAAACCCAGCACAAAACATGTTATTGTAAA
TCGTAAATTTCGTGGACAGAAGACAGGTCGCTCTATCGACCAACGGGACGCGCA
AATATTGCAGAACGAGGGCTGATCGACCTTTGTGGAAGACCCGCCCCCACCCAC
TCACATATCCGCTCCCAAATTTCAAGAAGATATTTGTATATTCTTTATCGGCTATA
CAAATCGGGGTAACATAGGAGTTAAGTACGAGTGGCTCGTCCAGCTCCAGGAGG
GCTATATCATGGTTGTACTTGTTTATAGCGGCATTATAATTGTGATGGGGTATGAT
CCTGATAACATTCCTTTTCTGTTCAGTATGCTCAGTTTCTTCAATGTTGTGTTCGCC
AGCCACGACCGTAATCTTAACCCCCGTCTCGACACAGTGTGCGGCCGTTACAATC
CACTTTTCATTGACTATGGAGCCCCCACAAAACGCGTCGACTTTTCCGTTGAGCA
CCACCTGCCATGGAAATTGGCCAGGTTTAGCGTCCTCGCCCCCGACAACCCTAGT
AAAGTCATTAAATGACTGTGTGGATTGTGTTATATTATCAAGAATCGTTTCGGCT
TCAGTAGAGTTAACGTAGTCCACATCGGGAAAAACTGTCTCGGCCCTTGTCAACT
TTGATGTCTGGGACACACTTACCCGACCGCACGGGAAGGGCACCGCCGGTTCAC
AGCTCTTTTGATTCTCAGCGAGCCGGTAGCCCTCAGTGCAACTACACACAACTTT
GTTGTCGGCGGAATTTTTACAGAATTGCTCGCATCGTCCATTTTTAATGTTGCAGG
TGACGTCCAACTCGCAGTTTTTTCCTTCAAAACCAAAAGGGCACCAACACTCGTA
GGAATTTATATCGTCTTTACAACTCCCCCCATTCAGACATGGATTAGATTCGCATT
GGTCCCCATCGACATATTGCTTCCAGAACTCAGTGGTCCGTTCTGTATTCTCAAA
CACCTCGCGCGCTTCTTCAAAACTGCATTTTTCCTCCATACACTCTCGCTCCAAGT
TCCCTTGCACGAATTCTTCAAGCTTTCCTGAGTTATACCTTTTAGGCCGGTTAAGT
ATCTTATTCGCGTTTTCGTGGTCCAGAAAAACTGAAATGTAAAAGAATAATTCTT
TAGTTTTAGCAAAAAAGAAAACATCATGAAAATTTTACATCTCTTAAGAAAGTCT
TTGTTTTTAATCCAAATAATCAGAGATCTAGGAACCCCTAGTGATGGAGTTGGCC
ACTCCCTCTCTGCGCGCTCGCTCGCTCACTGAGGCCGCCCGGGCAAAGCCCGGGC
GTCGGGCGACCTTTGGTCGCCCGGCCTCAGTGAGCGAGCGAGCGCGCAGAGAGG
GAGTGGCCAA
   P00415 full sequence (from ITR to ITR): (SEQ ID NO: 279) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTCTTAGGTCAGTG
AAGAGAAGAACAAAAAGCAGCATATTACAGTTAGTTGTCTTCATCAATCTTTAAA
TATGTTGTGTGGTTTTTCTCTCCCTGTTTCCACAGTTTTTCTTGATCATGAAAACGC
CAACAAAATTCTGAATCGGCCAAAGAGGTATAATTCAGGTAAATTGGAAGAGTT
TGTTCAAGGGAACCTTGAGAGAGAATGTATGGAAGAAAAGTGTAGTTTTGAAGA
 
AGCAGTATTCACTTTGGAGGACTTTGTCGGTGACTGGAGGCAAACCGCTGGTTAT
AATCTCGACCAAGTACTGGAACAGGGCGGGGTAAGTTCCCTCTTTCAGAATTTGG
GTGTAAGCGTCACACCAATCCAGCGGATTGTGTTGTCTGGAGAGAACGGACTCA
AAATTGACATCCATGTTATCATTCCATATGAAGGTCTCAGTGGAGACCAAATGGG
GCAGATCGAGAAGATTTTCAAGGTAGTTTACCCAGTCGACGATCACCACTTCAAA
GTCATTCTCCACTATGGCACACTTGTTATCGACGGAGTAACTCCTAATATGATTG
ATTACTTTGGTCGCCCGTATGAGGGCATCGCAGTGTTTGATGGCAAAAAGATCAC
CGTAACAGGAACGTTGTGGAATGGGAACAAGATAATCGACGAGAGATTGATAAA
TCCAGACGGGTCACTCCTGTTCAGGGTTACAATTAACGGCGTCACAGGATGGAG
ACTCTGTGAACGAATACTGGCCACAAATTTTTCACTCCTGAAGCAGGCCGGAGAC
GTGGAGGAAAACCCAGGGCCCGTGAGCAAGGGCGAGGAGCTGTTCACCGGGGT
GGTGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTCAT
CTGCACCACCGGCAAGCTGCCCGTGCCCTGGCCCACCCTCGTGACCACCCTGACC
TACGGCGTGCAGTGCTTCAGCCGCTACCCCGACCACATGAAGCAGCACGACTTCT
TCAAGTCCGCCATGCCCGAAGGCTACGTCCAGGAGCGCACCATCTTCTTCAAGGA
CGACGGCAACTACAAGACCCGCGCCGAGGTGAAGTTCGAGGGCGACACCCTGGT
GAACCGCATCGAGCTGAAGGGCATCGACTTCAAGGAGGACGGCAACATCCTGGG
GCACAAGCTGGAGTACAACTACAACAGCCACAACGTCTATATCATGGCCGACAA
GCAGAAGAACGGCATCAAGGTGAACTTCAAGATCCGCCACAACATCGAGGACGG
CAGCGTGCAGCTCGCCGACCACTACCAGCAGAACACCCCCATCGGCGACGGCCC
CGTGCTGCTGCCCGACAACCACTACCTGAGCACCCAGTCCGCCCTGAGCAAAGA
CCCCAACGAGAAGCGCGATCACATGGTCCTGCTGGAGTTCGTGACCGCCGCCGG
GATCACTCTCGGCATGGACGAGCTGTACAAGGGAGGAGGAAGCCCGAAGAAGA
AGAGAAAGGTCTAACCTCGACTGTGCCTTCTAGTTGCCAGCCATCTGTTGTTTGC
CCCTCCCCCGTGCCTTCCTTGACCCTGGAAGGTGCCACTCCCACTGTCCTTTCCTA
ATAAAATGAGGAAATTGCATCGCATTGTCTGAGTAGGTGTCATTCTATTCTGGGG
GGTGGGGTGGGGCAGGACAGCAAGGGGGAGGATTGGGAAGACAATAGCAGGCA
TGCTGGGGATGCGGTGGGCTCTATGGCTTCTGAGGCGGAAAGAACCAGCTGGGG
CTCTAGGGGGTATCCCCAAAAAACCTCCCACACCTCCCCCTGAACCTGAAACATA
AAATGAATGCAATTGTTGTTGTTAACTTGTTTATTGCAGCTTATAATGGTTACAAA
TAAAGCAATAGCATCACAAATTTCACAAATAAAGCATTTTTTTCACTGCATTCTA
GTTGTGGTTTGTCCAAACTCATCAATGTATCTTATCATGTCTGTTACACCTTCCTC
TTCTTCTTGGGGCTGCCGCCGCCCTTGTACAGCTCGTCCATGCCCAGGGTGATGC
CGGCGGCGGTCACGAACTCCAGCAGCACCATGTGGTCCCTCTTCTCGTTGGGGTC
CTTGCTCAGGGCGCTCTGGGTGCTCAGGTAGTGGTTGTCGGGCAGCAGCACGGG
GCCGTCGCCGATGGGGGTGTTCTGCTGGTAGTGGTCGGCCAGCTGCACGCTGCCG
TCCTCGATGTTGTGCCTGATCTTGAAGTTCACCTTGATGCCGTTCTTCTGCTTGTC
GGCCATGATGTACACGTTGTGGCTGTTGTAGTTGTACTCCAGCTTGTGGCCCAGG
ATGTTGCCGTCCTCCTTGAAGTCGATGCCCTTCAGCTCGATCCTGTTCACCAGGGT
GTCGCCCTCGAACTTCACCTCGGCCCTGGTCTTGTAGTTGCCGTCGTCCTTGAAG
AAGATGGTCCTCTCCTGCACGTAGCCCTCGGGCATGGCGCTCTTGAAGAAGTCGT
GCTGCTTCATGTGGTCGGGGTACCTGCTGAAGCACTGCACGCCGTAGGTCAGGGT
GGTCACCAGGGTGGGCCAGGGCACGGGCAGCTTGCCGGTGGTGCAGATGAACTT
CAGGGTCAGCTTGCCGTAGGTGGCGTCGCCCTCGCCCTCGCCGCTCACGCTGAAC
TTGTGGCCGTTCACGTCGCCGTCCAGCTCCACCAGGATGGGCACCACGCCGGTGA
ACAGCTCCTCGCCCTTGCTCACGGGGCCGGGGTTCTCCTCCACGTCGCCGGCCTG
CTTCAGCAGGCTGAAGTTGGTGGCCAGGATCCTCTCGCACAGCCTCCAGCCGGTC
ACGCCGTTGATGGTCACCCTGAACAGCAGGCTGCCGTCGGGGTTGATCAGCCTCT
CGTCGATGATCTTGTTGCCGTTCCACAGGGTGCCGGTCACGGTGATCTTCTTGCC
 
GTCGAACACGGCGATGCCCTCGTAGGGCCTGCCGAAGTAGTCGATCATGTTGGG
GGTCACGCCGTCGATCACCAGGGTGCCGTAGTGCAGGATCACCTTGAAGTGGTG
GTCGTCCACGGGGTACACCACCTTGAAAATCTTCTCGATCTGGCCCATCTGGTCG
CCGCTCAGGCCCTCGTAGGGGATGATCACGTGGATGTCGATCTTCAGGCCGTTCT
CGCCGCTCAGCACGATCCTCTGGATGGGGGTCACGCTCACGCCCAGGTTCTGGAA
CAGGCTGCTCACGCCGCCCTGCTCCAGCACCTGGTCCAGGTTGTAGCCGGCGGTC
TGCCTCCAGTCGCCCACGAAGTCCTCCAGGGTGAACACGGCCTCCTCGAAGCTGC
ACTTCTCCTCCATGCACTCCCTCTCCAGGTTGCCCTGCACGAACTCCTCCAGCTTG
CCGCTGTTGTACCTCTTGGGCCTGTTCAGGATCTTGTTGGCGTTCTCGTGGTCCAG
GAA
   P00418 full sequence (from ITR to ITR): (SEQ ID NO: 280) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTCTTAGGTCAGTG
AAGAGAAGAACAAAAAGCAGCATATTACAGTTAGTTGTCTTCATCAATCTTTAAA
TATGTTGTGTGGTTTTTCTCTCCCTGTTTCCACAGTTTTTCTTGATCATGAAAACGC
CAACAAAATTCTGAATCGGCCAAAGAGGTATAATTCAGGTAAATTGGAAGAGTT
TGTTCAAGGGAACCTTGAGAGAGAATGTATGGAAGAAAAGTGTAGTTTTGAAGA
AGCACGAGAAGTTTTTGAAAACACTGAAAGAACAACTGAATTTTGGAAGCAGTA
TGTTGATGGAGATCAGTGTGAGTCCAATCCATGTTTAAATGGCGGCAGTTGCAAG
GATGACATTAATTCCTATGAATGTTGGTGTCCCTTTGGATTTGAAGGAAAGAACT
GTGAATTAGATGTAACATGTAACATTAAGAATGGCAGATGCGAGCAGTTTTGTA
AAAATAGTGCTGATAACAAGGTGGTTTGCTCCTGTACTGAGGGATATCGACTTGC
AGAAAACCAGAAGTCCTGTGAACCAGCAGTGCCATTTCCATGTGGAAGAGTTTC
TGTTTCACAAACTTCTAAGCTCACCCGTGCTGAGACTGTTTTTCCTGATGTGGACT
ATGTAAATTCTACTGAAGCTGAAACCATTTTGGATAACATCACTCAAAGCACCCA
ATCATTTAATGACTTCACTCGGGTTGTTGGTGGAGAAGATGCCAAACCAGGTCAA
TTCCCTTGGCAGGTTGTTTTGAATGGTAAAGTTGATGCATTCTGTGGAGGCTCTAT
CGTTAATGAAAAATGGATTGTAACTGCTGCCCACTGTGTTGAAACTGGTGTTAAA
ATTACAGTTGTCGCAGGTGAACATAATATTGAGGAGACAGAACATACAGAGCAA
AAGCGAAATGTGATTCGAATTATTCCTCACCACAACTACAATGCAGCTATTAATA
AGTACAACCATGACATTGCCCTTCTGGAACTGGACGAACCCTTAGTGCTAAACAG
CTACGTTACACCTATTTGCATTGCTGACAAGGAATACACGAACATCTTCCTCAAA
TTTGGATCTGGCTATGTAAGTGGCTGGGGAAGAGTCTTCCACAAAGGGAGATCA
GCTTTAGTTCTTCAGTACCTTAGAGTTCCACTTGTTGACCGAGCCACATGTCTTCT
ATCTACAAAGTTCACCATCTATAACAACATGTTCTGTGCTGGCTTCCATGAAGGA
GGTAGAGATTCATGTCAAGGAGATAGTGGGGGACCCCATGTTACTGAAGTGGAA
GGGACCAGTTTCTTAACTGGAATTATTAGCTGGGGTGAAGAGTGTGCAATGAAA
GGCAAATATGGAATATATACCAAGGTCTCCCGGTATGTCAACTGGATTAAGGAA
AAAACAAAGCTCACTGTCAGCGGATGGAGACTGTTCAAGAAGATCAGCTAACCT
CGACTGTGCCTTCTAGTTGCCAGCCATCTGTTGTTTGCCCCTCCCCCGTGCCTTCC
TTGACCCTGGAAGGTGCCACTCCCACTGTCCTTTCCTAATAAAATGAGGAAATTG
CATCGCATTGTCTGAGTAGGTGTCATTCTATTCTGGGGGGTGGGGTGGGGCAGGA
CAGCAAGGGGGAGGATTGGGAAGACAATAGCAGGCATGCTGGGGATGCGGTGG
GCTCTATGGCTTCTGAGGCGGAAAGAACCAGCTGGGGCTCTAGGGGGTATCCCC
AAAAAACCTCCCACACCTCCCCCTGAACCTGAAACATAAAATGAATGCAATTGTT
GTTGTTAACTTGTTTATTGCAGCTTATAATGGTTACAAATAAAGCAATAGCATCA
CAAATTTCACAAATAAAGCATTTTTTTCACTGCATTCTAGTTGTGGTTTGTCCAAA
CTCATCAATGTATCTTATCATGTCTGTTAGGAAATCTTCTTAAACAGCCGCCAGC
 
CGCTCACGGTGAGCTTAGTCTTTTCTTTTATCCAATTCACGTAGCGAGAGACCTTC
GTATAGATGCCATATTTCCCCTTCATCGCACATTCCTCCCCCCAACTTATTATCCC
GGTCAAGAAACTTGTTCCTTCGACTTCAGTGACGTGTGGTCCACCTGAATCACCT
TGGCATGAGTCGCGACCGCCCTCGTGAAACCCAGCACAAAACATGTTATTGTAA
ATCGTAAATTTCGTGGACAGAAGACAGGTCGCTCTATCGACCAACGGGACGCGC
AAATATTGCAGAACGAGGGCTGATCGACCTTTGTGGAAGACCCGCCCCCACCCA
CTCACATATCCGCTCCCAAATTTCAAGAAGATATTTGTATATTCTTTATCGGCTAT
ACAAATCGGGGTAACATAGGAGTTAAGTACGAGTGGCTCGTCCAGCTCCAGGAG
GGCTATATCATGGTTGTACTTGTTTATAGCGGCATTATAATTGTGATGGGGTATG
ATCCTGATAACATTCCTTTTCTGTTCAGTATGCTCAGTTTCTTCAATGTTGTGTTCG
CCAGCCACGACCGTAATCTTAACCCCCGTCTCGACACAGTGTGCGGCCGTTACAA
TCCACTTTTCATTGACTATGGAGCCCCCACAAAACGCGTCGACTTTTCCGTTGAG
CACCACCTGCCATGGAAATTGGCCAGGTTTAGCGTCCTCGCCCCCGACAACCCTA
GTAAAGTCATTAAATGACTGTGTGGATTGTGTTATATTATCAAGAATCGTTTCGG
CTTCAGTAGAGTTAACGTAGTCCACATCGGGAAAAACTGTCTCGGCCCTTGTCAA
CTTTGATGTCTGGGACACACTTACCCGACCGCACGGGAAGGGCACCGCCGGTTC
ACAGCTCTTTTGATTCTCAGCGAGCCGGTAGCCCTCAGTGCAACTACACACAACT
TTGTTGTCGGCGGAATTTTTACAGAATTGCTCGCATCGTCCATTTTTAATGTTGCA
GGTGACGTCCAACTCGCAGTTTTTTCCTTCAAAACCAAAAGGGCACCAACACTCG
TAGGAATTTATATCGTCTTTACAACTCCCCCCATTCAGACATGGATTAGATTCGC
ATTGGTCCCCATCGACATATTGCTTCCAGAACTCAGTGGTCCGTTCTGTATTCTCA
AACACCTCGCGCGCTTCTTCAAAACTGCATTTTTCCTCCATACACTCTCGCTCCAA
GTTCCCTTGCACGAATTCTTCAAGCTTTCCTGAGTTATACCTTTTAGGCCGGTTAA
GTATCTTATTCGCGTTTTCGTGGTCCAGAAAAACTGTGGAAACAGGGAGAGAAA
AACCACACAACATATTTAAAGATTGATGAAGACAACTAACTGTAATATGCTGCTT
TTTGTTCTTCTCTTCACTGACCTAAGAGATCTAGGAACCCCTAGTGATGGAGTTG
GCCACTCCCTCTCTGCGCGCTCGCTCGCTCACTGAGGCCGCCCGGGCAAAGCCCG
GGCGTCGGGCGACCTTTGGTCGCCCGGCCTCAGTGAGCGAGCGAGCGCGCAGAG
AGGGAGTGGCCAA
   P00123 full sequence (from ITR to ITR): (SEQ ID NO: 281) 
GGCCACTCCCTCTCTGCGCGCTCGCTCGCTCACTGAGGCCGGGCGACCAAAGGTC
GCCCGACGCCCGGGCTTTGCCCGGGCGGCCTCAGTGAGCGAGCGAGCGCGCAGA
GAGGGAGTGGCCAACTCCATCACTAGGGGTTCCTGGAGGGGTGGAGTCGTGATA
GGTCAGTGAAGAGAAGAACAAAAAGCAGCATATTACAGTTAGTTGTCTTCATCA
ATCTTTAAATATGTTGTGTGGTTTTTCTCTCCCTGTTTCCACAGTTTTTCTTGATCA
TGAAAACGCCAACAAAATTCTGAATCGGCCAAAGAGGTATAATTCAGGTAAATT
GGAAGAGTTTGTTCAAGGGAACCTTGAGAGAGAATGTATGGAAGAAAAGTGTAG
TTTTGAAGAAGCACGAGAAGTTTTTGAAAACACTGAAAGAACAACTGAATTTTG
GAAGCAGTATGTTGATGGAGATCAGTGTGAGTCCAATCCATGTTTAAATGGCGGC
AGTTGCAAGGATGACATTAATTCCTATGAATGTTGGTGTCCCTTTGGATTTGAAG
GAAAGAACTGTGAATTAGATGTAACATGTAACATTAAGAATGGCAGATGCGAGC
AGTTTTGTAAAAATAGTGCTGATAACAAGGTGGTTTGCTCCTGTACTGAGGGATA
TCGACTTGCAGAAAACCAGAAGTCCTGTGAACCAGCAGTGCCATTTCCATGTGG
AAGAGTTTCTGTTTCACAAACTTCTAAGCTCACCCGTGCTGAGACTGTTTTTCCTG
ATGTGGACTATGTAAATTCTACTGAAGCTGAAACCATTTTGGATAACATCACTCA
AAGCACCCAATCATTTAATGACTTCACTCGGGTTGTTGGTGGAGAAGATGCCAAA
CCAGGTCAATTCCCTTGGCAGGTTGTTTTGAATGGTAAAGTTGATGCATTCTGTG
GAGGCTCTATCGTTAATGAAAAATGGATTGTAACTGCTGCCCACTGTGTTGAAAC
TGGTGTTAAAATTACAGTTGTCGCAGGTGAACATAATATTGAGGAGACAGAACA
 
TACAGAGCAAAAGCGAAATGTGATTCGAATTATTCCTCACCACAACTACAATGC
AGCTATTAATAAGTACAACCATGACATTGCCCTTCTGGAACTGGACGAACCCTTA
GTGCTAAACAGCTACGTTACACCTATTTGCATTGCTGACAAGGAATACACGAACA
TCTTCCTCAAATTTGGATCTGGCTATGTAAGTGGCTGGGGAAGAGTCTTCCACAA
AGGGAGATCAGCTTTAGTTCTTCAGTACCTTAGAGTTCCACTTGTTGACCGAGCC
ACATGTCTTCTATCTACAAAGTTCACCATCTATAACAACATGTTCTGTGCTGGCTT
CCATGAAGGAGGTAGAGATTCATGTCAAGGAGATAGTGGGGGACCCCATGTTAC
TGAAGTGGAAGGGACCAGTTTCTTAACTGGAATTATTAGCTGGGGTGAAGAGTG
TGCAATGAAAGGCAAATATGGAATATATACCAAGGTATCCCGGTATGTCAACTG
GATTAAGGAAAAAACAAAGCTCACTTAACCTCGACTGTGCCTTCTAGTTGCCAGC
CATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTC
ATTCTATTCTGGGGGGTGGGGTGGGGCAGGACAGCAAGGGGGAGGATTGGGAAG
ACAATAGCAGGCATGCTGGGGATGCGGTGGGCTCTATGGCTTCTGAGGCGGAAA
GAACCAGCTGGGGCTCTAGGGGGTATCCCCACTAGTCCACTCCCTCTCTGCGCGC
TCGCTCGCTCACTGAGGCCGGGCGACCAAAGGTCGCCCGACGCCCGGGCTTTGC
CCGGGCGGCCTCAGTGAGCGAGCGAGCGCGCAGAGAGGGA
   P00204 full sequence (from ITR to ITR): (SEQ ID NO: 282) 
GGCCACTCCCTCTCTGCGCGCTCGCTCGCTCACTGAGGCCGGGCGACCAAAGGTC
GCCCGACGCCCGGGCTTTGCCCGGGCGGCCTCAGTGAGCGAGCGAGCGCGCAGA
GAGGGAGTGGCCAACTCCATCACTAGGGGTTCCTGGAGGGGTGGAGTCGTGACC
TAGGTCGTCTCCGGCTCTGCTTTTTCCAGGGGTGTGTTTCGCCGAGAAGCACGTA
AGAGTTTTATGTTTTTTCATCTCTGCTTGTATTTTTCTAGTAATGGAAGCCTGGTA
TTTTAAAATAGTTAAATTTTCCTTTAGTGCTGATTTCTAGATTATTATTACTGTTGT
TGTTGTTATTATTGTCATTATTTGCATCTGAGAACTAGGTCAGTGAAGAGAAGAA
CAAAAAGCAGCATATTACAGTTAGTTGTCTTCATCAATCTTTAAATATGTTGTGT
GGTTTTTCTCTCCCTGTTTCCACAGTTTTTCTTGATCATGAAAACGCCAACAAAAT
TCTGAATCGGCCAAAGAGGTATAATTCAGGTAAATTGGAAGAGTTTGTTCAAGG
GAACCTTGAGAGAGAATGTATGGAAGAAAAGTGTAGTTTTGAAGAAGCACGAGA
AGTTTTTGAAAACACTGAAAGAACAACTGAATTTTGGAAGCAGTATGTTGATGG
AGATCAGTGTGAGTCCAATCCATGTTTAAATGGCGGCAGTTGCAAGGATGACATT
AATTCCTATGAATGTTGGTGTCCCTTTGGATTTGAAGGAAAGAACTGTGAATTAG
ATGTAACATGTAACATTAAGAATGGCAGATGCGAGCAGTTTTGTAAAAATAGTG
CTGATAACAAGGTGGTTTGCTCCTGTACTGAGGGATATCGACTTGCAGAAAACCA
GAAGTCCTGTGAACCAGCAGTGCCATTTCCATGTGGAAGAGTTTCTGTTTCACAA
ACTTCTAAGCTCACCCGTGCTGAGACTGTTTTTCCTGATGTGGACTATGTAAATTC
TACTGAAGCTGAAACCATTTTGGATAACATCACTCAAAGCACCCAATCATTTAAT
GACTTCACTCGGGTTGTTGGTGGAGAAGATGCCAAACCAGGTCAATTCCCTTGGC
AGGTTGTTTTGAATGGTAAAGTTGATGCATTCTGTGGAGGCTCTATCGTTAATGA
AAAATGGATTGTAACTGCTGCCCACTGTGTTGAAACTGGTGTTAAAATTACAGTT
GTCGCAGGTGAACATAATATTGAGGAGACAGAACATACAGAGCAAAAGCGAAA
TGTGATTCGAATTATTCCTCACCACAACTACAATGCAGCTATTAATAAGTACAAC
CATGACATTGCCCTTCTGGAACTGGACGAACCCTTAGTGCTAAACAGCTACGTTA
CACCTATTTGCATTGCTGACAAGGAATACACGAACATCTTCCTCAAATTTGGATC
TGGCTATGTAAGTGGCTGGGGAAGAGTCTTCCACAAAGGGAGATCAGCTTTAGTT
CTTCAGTACCTTAGAGTTCCACTTGTTGACCGAGCCACATGTCTTCTATCTACAAA
GTTCACCATCTATAACAACATGTTCTGTGCTGGCTTCCATGAAGGAGGTAGAGAT
TCATGTCAAGGAGATAGTGGGGGACCCCATGTTACTGAAGTGGAAGGGACCAGT
TTCTTAACTGGAATTATTAGCTGGGGTGAAGAGTGTGCAATGAAAGGCAAATAT
 
GGAATATATACCAAGGTATCCCGGTATGTCAACTGGATTAAGGAAAAAACAAAG
CTCACTTAACCTCGACTGTGCCTTCTAGTTGCCAGCCATCTGTTGTTTGCCCCTCC
CCCGTGCCTTCCTTGACCCTGGAAGGTGCCACTCCCACTGTCCTTTCCTAATAAA
ATGAGGAAATTGCATCGCATTGTCTGAGTAGGTGTCATTCTATTCTGGGGGGTGG
GGTGGGGCAGGACAGCAAGGGGGAGGATTGGGAAGACAATAGCAGGCATGCTG
GGGATGCGGTGGGCTCTATGGCTTCTGAGGCGGAAAGAACCAGCTGGGGCTCTA
GGGGGTATCCCCCTTAGGTGGTTATATTATTGATATATTTTTGGTATCTTTGATGA
CAATAATGGGGGATTTTGAAAGCTTAGCTTTAAATTTCTTTTAATTAAAAAAAAA
TGCTAGGCAGAATGACTCAAATTACGTTGGATACAGTTGAATTTATTACGGTCTC
ATAGGGCCTGCCTGCTCGACCATGCTATACTAAAAATTAAAAGTGTACTAGTCCA
CTCCCTCTCTGCGCGCTCGCTCGCTCACTGAGGCCGGGCGACCAAAGGTCGCCCG
ACGCCCGGGCTTTGCCCGGGCGGCCTCAGTGAGCGAGCGAGCGCGCAGAGAGGG
A
   P00353 full sequence (from ITR to ITR): (SEQ ID NO: 283) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTGATTTTGAAAGCT
TAGCTTTAAATTTCTTTTAATTAAAAAAAAATGCTAGGCAGAATGACTCAAATTA
CGTTGGATACAGTTGAATTTATTACGGTCTCATAGGGCCTGCCTGCTCGACCATG
CTATACTAAAAATTAAAAGTGTGTGTTACTAATTTTATAAATGGAGTTTCCATTTA
TATTTACCTTTATTTCTTATTTACCATTGTCTTAGTAGATATTTACAAACATGACA
GAAACACTAAATCTTGAGTTTGAATGCACAGATATAAACACTTAACGGGTTTTAA
AAATAATAATGTTGGTGAAAAAATATAACTTTGAGTGTAGCAGAGAGGAACCAT
TGCCACCTTCAGATTTTCCTGTAACGATCGGGAACTGGCATCTTCAGGGAGTAGC
TTAGGTCAGTGAAGAGAAGAACAAAAAGCAGCATATTACAGTTAGTTGTCTTCA
TCAATCTTTAAATATGTTGTGTGGTTTTTCTCTCCCTGTTTCCACAGTTTTTCTTGA
TCATGAAAACGCCAACAAAATTCTGAATCGGCCAAAGAGGTTTCTTGATCATGA
AAACGCCAACAAAATTCTGAATCGGCCAAAGAGGTATAATTCAGGTAAATTGGA
AGAGTTTGTTCAAGGGAACCTTGAGAGAGAATGTATGGAAGAAAAGTGTAGTTT
TGAAGAAGCACGAGAAGTTTTTGAAAACACTGAAAGAACAACTGAATTTTGGAA
GCAGTATGTTGATGGAGATCAGTGTGAGTCCAATCCATGTTTAAATGGCGGCAGT
TGCAAGGATGACATTAATTCCTATGAATGTTGGTGTCCCTTTGGATTTGAAGGAA
AGAACTGTGAATTAGATGTAACATGTAACATTAAGAATGGCAGATGCGAGCAGT
TTTGTAAAAATAGTGCTGATAACAAGGTGGTTTGCTCCTGTACTGAGGGATATCG
ACTTGCAGAAAACCAGAAGTCCTGTGAACCAGCAGTGCCATTTCCATGTGGAAG
AGTTTCTGTTTCACAAACTTCTAAGCTCACCCGTGCTGAGACTGTTTTTCCTGATG
TGGACTATGTAAATTCTACTGAAGCTGAAACCATTTTGGATAACATCACTCAAAG
CACCCAATCATTTAATGACTTCACTCGGGTTGTTGGTGGAGAAGATGCCAAACCA
GGTCAATTCCCTTGGCAGGTTGTTTTGAATGGTAAAGTTGATGCATTCTGTGGAG
GCTCTATCGTTAATGAAAAATGGATTGTAACTGCTGCCCACTGTGTTGAAACTGG
TGTTAAAATTACAGTTGTCGCAGGTGAACATAATATTGAGGAGACAGAACATAC
AGAGCAAAAGCGAAATGTGATTCGAATTATTCCTCACCACAACTACAATGCAGC
TATTAATAAGTACAACCATGACATTGCCCTTCTGGAACTGGACGAACCCTTAGTG
CTAAACAGCTACGTTACACCTATTTGCATTGCTGACAAGGAATACACGAACATCT
TCCTCAAATTTGGATCTGGCTATGTAAGTGGCTGGGGAAGAGTCTTCCACAAAGG
GAGATCAGCTTTAGTTCTTCAGTACCTTAGAGTTCCACTTGTTGACCGAGCCACA
TGTCTTCTATCTACAAAGTTCACCATCTATAACAACATGTTCTGTGCTGGCTTCCA
TGAAGGAGGTAGAGATTCATGTCAAGGAGATAGTGGGGGACCCCATGTTACTGA
AGTGGAAGGGACCAGTTTCTTAACTGGAATTATTAGCTGGGGTGAAGAGTGTGC
 
AATGAAAGGCAAATATGGAATATATACCAAGGTATCCCGGTATGTCAACTGGAT
TAAGGAAAAAACAAAGCTCACTTAACCTCGACTGTGCCTTCTAGTTGCCAGCCAT
CTGTTGTTTGCCCCTCCCCCGTGCCTTCCTTGACCCTGGAAGGTGCCACTCCCACT
GTCCTTTCCTAATAAAATGAGGAAATTGCATCGCATTGTCTGAGTAGGTGTCATT
CTATTCTGGGGGGTGGGGTGGGGCAGGACAGCAAGGGGGAGGATTGGGAAGAC
AATAGCAGGCATGCTGGGGATGCGGTGGGCTCTATGGCTTCTGAGGCGGAAAGA
ACCAGCTGGGGCTCTAGGGGGTATCCCCGTGAGATCGCCCATCGGTATAATGATT
TGGGAGAACAACATTTCAAAGGCCTGTAAGTTATAATGCTGAAAGCCCACTTAA
TATTTCTGGTAGTATTAGTTAAAGTTTTAAAACACCTTTTTCCACCTTGAGTGTGA
GAATTGTAGAGCAGTGCTGTCCAGTAGAAATGTGTGCATTGACAGAAAGACTGT
GGATCTGTGCTGAGCAATGTGGCAGCCAGAGATCACAAGGCTATCAAGCACTTT
GCACATGGCAAGTGTAACTGAGAAGCACACATTCAAATAATAGTTAATTTTAATT
GAATGTATCTAGCCATGTGTGGCTAGTAGCTCCTTTCCTGGAGAGAGAATCTGGA
GCCCACATCTAACTTGTTAAGTCTGGAATCTTATTTTTTATTTCTGGAAAGGTCTA
TGAACTATAGTTTTGGGGGCAGCTCACTTACTAACTTTTAATGCAATAAGAATCT
CATGGTATCTTGAGAACATTATTTTGTCTCTTTGTAGATCTAGGAACCCCTAGTGA
TGGAGTTGGCCACTCCCTCTCTGCGCGCTCGCTCGCTCACTGAGGCCGCCCGGGC
AAAGCCCGGGCGTCGGGCGACCTTTGGTCGCCCGGCCTCAGTGAGCGAGCGAGC
GCGCAGAGAGGGAGTGGCCAA
   P00354 full sequence (from ITR to ITR): (SEQ ID NO: 284) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTTAGCCTCTGGCA
AAATGAAGTGGGTAACCTTTCTCCTCCTCCTCTTCGTCTCCGGCTCTGCTTTTTCC
AGGGGTGTGTTTCGCCGAGAAGCACGTAAGAGTTTTATGTTTTTTCATCTCTGCTT
GTATTTTTCTAGTAATGGAAGCCTGGTATTTTAAAATAGTTAAATTTTCCTTTAGT
GCTGATTTCTAGATTATTATTACTGTTGTTGTTATTATTGTCATTATTTGCATC
TGAGAACCCTTAGGTGGTTATATTATTGATATATTTTTGGTATCTTTGATGACAAT
AATGGGGGATTTTGAAAGCTTAGCTTTAAATTTCTTTTAATTAAAAAAAAATGCT
AGGCAGAATGACTCAAATTACGTTGGATACAGTTGAATTTATTACGGTCTCATAG
GGCCTGCCTGCTCGACCATGCTATACTAAAAATTAAAAGTGTGTGTTACTAATTT
TATAAATGGAGTTTCCATTTATATTTACCTTTATTTCTTATTTACCATTGTCTTAGT
AGATATTTACAAACATGACAGAAACACTAAATCTTGAGTTTGAATGCACAGATAT
AAACACTTAACGGGTTTTAAAAATAATAATGTTGGTGAAAAAATATAACTTTGAG
TGTAGCAGAGAGGAACCATTGCCACCTTCAGATTTTCCTGTAACGATCGGGAACT
GGCATCTTCAGGGAGTAGCTTAGGTCAGTGAAGAGAAGAACAAAAAGCAGCATA
TTACAGTTAGTTGTCTTCATCAATCTTTAAATATGTTGTGTGGTTTTTCTCTCCCTG
TTTCCACAGTTTTTCTTGATCATGAAAACGCCAACAAAATTCTGAATCGGCCAAA
GAGGTATAATTCAGGTAAATTGGAAGAGTTTGTTCAAGGGAACCTTGAGAGAGA
ATGTATGGAAGAAAAGTGTAGTTTTGAAGAAGCACGAGAAGTTTTTGAAAACAC
TGAAAGAACAACTGAATTTTGGAAGCAGTATGTTGATGGAGATCAGTGTGAGTC
CAATCCATGTTTAAATGGCGGCAGTTGCAAGGATGACATTAATTCCTATGAATGT
TGGTGTCCCTTTGGATTTGAAGGAAAGAACTGTGAATTAGATGTAACATGTAACA
TTAAGAATGGCAGATGCGAGCAGTTTTGTAAAAATAGTGCTGATAACAAGGTGG
TTTGCTCCTGTACTGAGGGATATCGACTTGCAGAAAACCAGAAGTCCTGTGAACC
AGCAGTGCCATTTCCATGTGGAAGAGTTTCTGTTTCACAAACTTCTAAGCTCACC
CGTGCTGAGACTGTTTTTCCTGATGTGGACTATGTAAATTCTACTGAAGCTGAAA
CCATTTTGGATAACATCACTCAAAGCACCCAATCATTTAATGACTTCACTCGGGT
TGTTGGTGGAGAAGATGCCAAACCAGGTCAATTCCCTTGGCAGGTTGTTTTGAAT
 
GGTAAAGTTGATGCATTCTGTGGAGGCTCTATCGTTAATGAAAAATGGATTGTAA
CTGCTGCCCACTGTGTTGAAACTGGTGTTAAAATTACAGTTGTCGCAGGTGAACA
TAATATTGAGGAGACAGAACATACAGAGCAAAAGCGAAATGTGATTCGAATTAT
TCCTCACCACAACTACAATGCAGCTATTAATAAGTACAACCATGACATTGCCCTT
CTGGAACTGGACGAACCCTTAGTGCTAAACAGCTACGTTACACCTATTTGCATTG
CTGACAAGGAATACACGAACATCTTCCTCAAATTTGGATCTGGCTATGTAAGTGG
CTGGGGAAGAGTCTTCCACAAAGGGAGATCAGCTTTAGTTCTTCAGTACCTTAGA
GTTCCACTTGTTGACCGAGCCACATGTCTTCTATCTACAAAGTTCACCATCTATAA
CAACATGTTCTGTGCTGGCTTCCATGAAGGAGGTAGAGATTCATGTCAAGGAGAT
AGTGGGGGACCCCATGTTACTGAAGTGGAAGGGACCAGTTTCTTAACTGGAATT
ATTAGCTGGGGTGAAGAGTGTGCAATGAAAGGCAAATATGGAATATATACCAAG
GTATCCCGGTATGTCAACTGGATTAAGGAAAAAACAAAGCTCACTTAACCTCGA
CTGTGCCTTCTAGTTGCCAGCCATCTGTTGTTTGCCCCTCCCCCGTGCCTTCCTTG
ACCCTGGAAGGTGCCACTCCCACTGTCCTTTCCTAATAAAATGAGGAAATTGCAT
CGCATTGTCTGAGTAGGTGTCATTCTATTCTGGGGGGTGGGGTGGGGCAGGACAG
CAAGGGGGAGGATTGGGAAGACAATAGCAGGCATGCTGGGGATGCGGTGGGCT
CTATGGCTTCTGAGGCGGAAAGAACCAGCTGGGGCTCTAGGGGGTATCCCCGTG
AGATCGCCCATCGGTATAATGATTTGGGAGAACAACATTTCAAAGGCCTGTAAG
TTATAATGCTGAAAGCCCACTTAATATTTCTGGTAGTATTAGTTAAAGTTTTAAA
ACACCTTTTTCCACCTTGAGTGTGAGAATTGTAGAGCAGTGCTGTCCAGTAGAAA
TGTGTGCATTGACAGAAAGACTGTGGATCTGTGCTGAGCAATGTGGCAGCCAGA
GATCACAAGGCTATCAAGCACTTTGCACATGGCAAGTGTAACTGAGAAGCACAC
ATTCAAATAATAGTTAATTTTAATTGAATGTATCTAGCCATGTGTGGCTAGTAGC
TCCTTTCCTGGAGAGAGAATCTGGAGCCCACATCTAACTTGTTAAGTCTGGAATC
TTATTTTTTATTTCTGGAAAGGTCTATGAACTATAGTTTTGGGGGCAGCTCACTTA
CTAACTTTTAATGCAATAAGAATCTCATGGTATCTTGAGAACATTATTTTGTCTCT
TTGTAGTACTGAAACCTTATACATGTGAAGTAAGGGGTCTATACTTAAGTCACAT
CTCCAACCTTAGTAATGTTTTAATGTAGTAAAAAAATGAGTAATTAATTTATTTTT
AGAAGGTCAATAGTATCATGTATTCCAAATAACAGAGGTATATGGTTAGAAAAG
AAACAATTCAAAGGACTTATATAATATCTAGCCTTGACAATGAATAAATTTAGAG
AGTAGTTTGCCTGTTTGCCTCATGTTCATAAATCTATTGACACATATGTGCATCTG
CACTTCAGCATGGTAGAAGTCCATATTCAGATCTAGGAACCCCTAGTGATGGAGT
TGGCCACTCCCTCTCTGCGCGCTCGCTCGCTCACTGAGGCCGCCCGGGCAAAGCC
CGGGCGTCGGGCGACCTTTGGTCGCCCGGCCTCAGTGAGCGAGCGAGCGCGCAG
AGAGGGAGTGGCCAA
   P00350: The 300/600bp HA F9 construct (for G551) (SEQ ID NO: 285) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTAAGTATATTAGA
GCGAGTCTTTCTGCACACAGATCACCTTTCCTATCAACCCCACTAGCCTCTGGCA
AAATGAAGTGGGTAACCTTTCTCCTCCTCCTCTTCGTCTCCGGCTCTGCTTTTTCC
AGGGGTGTGTTTCGCCGAGAAGCACGTAAGAGTTTTATGTTTTTTCATCTCTGCTT
GTATTTTTCTAGTAATGGAAGCCTGGTATTTTAAAATAGTTAAATTTTCCTTTAGT
GCTGATTTCTAGATTATTATTACTGTTGTTGTTGTTATTATTGTCATTATTTGCATC
TGAGAACCTTTTTCTTGATCATGAAAACGCCAACAAAATTCTGAATCGGCCAAAG
AGGTATAATTCAGGTAAATTGGAAGAGTTTGTTCAAGGGAACCTTGAGAGAGAA
TGTATGGAAGAAAAGTGTAGTTTTGAAGAAGCACGAGAAGTTTTTGAAAACACT
GAAAGAACAACTGAATTTTGGAAGCAGTATGTTGATGGAGATCAGTGTGAGTCC
AATCCATGTTTAAATGGCGGCAGTTGCAAGGATGACATTAATTCCTATGAATGTT
 
GGTGTCCCTTTGGATTTGAAGGAAAGAACTGTGAATTAGATGTAACATGTAACAT
TAAGAATGGCAGATGCGAGCAGTTTTGTAAAAATAGTGCTGATAACAAGGTGGT
TTGCTCCTGTACTGAGGGATATCGACTTGCAGAAAACCAGAAGTCCTGTGAACCA
GCAGTGCCATTTCCATGTGGAAGAGTTTCTGTTTCACAAACTTCTAAGCTCACCC
GTGCTGAGACTGTTTTTCCTGATGTGGACTATGTAAATTCTACTGAAGCTGAAAC
CATTTTGGATAACATCACTCAAAGCACCCAATCATTTAATGACTTCACTCGGGTT
GTTGGTGGAGAAGATGCCAAACCAGGTCAATTCCCTTGGCAGGTTGTTTTGAATG
GTAAAGTTGATGCATTCTGTGGAGGCTCTATCGTTAATGAAAAATGGATTGTAAC
TGCTGCCCACTGTGTTGAAACTGGTGTTAAAATTACAGTTGTCGCAGGTGAACAT
AATATTGAGGAGACAGAACATACAGAGCAAAAGCGAAATGTGATTCGAATTATT
CCTCACCACAACTACAATGCAGCTATTAATAAGTACAACCATGACATTGCCCTTC
TGGAACTGGACGAACCCTTAGTGCTAAACAGCTACGTTACACCTATTTGCATTGC
TGACAAGGAATACACGAACATCTTCCTCAAATTTGGATCTGGCTATGTAAGTGGC
TGGGGAAGAGTCTTCCACAAAGGGAGATCAGCTTTAGTTCTTCAGTACCTTAGAG
TTCCACTTGTTGACCGAGCCACATGTCTTCTATCTACAAAGTTCACCATCTATAAC
AACATGTTCTGTGCTGGCTTCCATGAAGGAGGTAGAGATTCATGTCAAGGAGATA
GTGGGGGACCCCATGTTACTGAAGTGGAAGGGACCAGTTTCTTAACTGGAATTAT
TAGCTGGGGTGAAGAGTGTGCAATGAAAGGCAAATATGGAATATATACCAAGGT
ATCCCGGTATGTCAACTGGATTAAGGAAAAAACAAAGCTCACTTAACCTCGACT
GTGCCTTCTAGTTGCCAGCCATCTGTTGTTTGCCCCTCCCCCGTGCCTTCCTTGAC
CCTGGAAGGTGCCACTCCCACTGTCCTTTCCTAATAAAATGAGGAAATTGCATCG
CATTGTCTGAGTAGGTGTCATTCTATTCTGGGGGGTGGGGTGGGGCAGGACAGCA
AGGGGGAGGATTGGGAAGACAATAGCAGGCATGCTGGGGATGCGGTGGGCTCT
ATGGCTTCTGAGGCGGAAAGAACCAGCTGGGGCTCTAGGGGGTATCCCCCTTAG
GTGGTTATATTATTGATATATTTTTGGTATCTTTGATGACAATAATGGGGGATTTT
GAAAGCTTAGCTTTAAATTTCTTTTAATTAAAAAAAAATGCTAGGCAGAATGACT
CAAATTACGTTGGATACAGTTGAATTTATTACGGTCTCATAGGGCCTGCCTGCTC
GACCATGCTATACTAAAAATTAAAAGTGTGTGTTACTAATTTTATAAATGGAGTT
TCCATTTATATTTACCTTTATTTCTTATTTACCATTGTCTTAGTAGATATTTACAAA
CATGACAGAAACACTAAAGATCTAGGAACCCCTAGTGATGGAGTTGGCCACTCC
CTCTCTGCGCGCTCGCTCGCTCACTGAGGCCGCCCGGGCAAAGCCCGGGCGTCGG
GCGACCTTTGGTCGCCCGGCCTCAGTGAGCGAGCGAGCGCGCAGAGAGGGAGTG
GCCAA
   P00356: The 300/2000bp HA F9 construct (for G551) (SEQ ID NO: 286) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTAAGTATATTAGA
GCGAGTCTTTCTGCACACAGATCACCTTTCCTATCAACCCCACTAGCCTCTGGCA
AAATGAAGTGGGTAACCTTTCTCCTCCTCCTCTTCGTCTCCGGCTCTGCTTTTTCC
AGGGGTGTGTTTCGCCGAGAAGCACGTAAGAGTTTTATGTTTTTTCATCTCTGCTT
GTATTTTTCTAGTAATGGAAGCCTGGTATTTTAAAATAGTTAAATTTTCCTTTAGT
GCTGATTTCTAGATTATTATTACTGTTGTTGTTGTTATTATTGTCATTATTTGCATC
TGAGAACCTTTTTCTTGATCATGAAAACGCCAACAAAATTCTGAATCGGCCAAAG
AGGTATAATTCAGGTAAATTGGAAGAGTTTGTTCAAGGGAACCTTGAGAGAGAA
TGTATGGAAGAAAAGTGTAGTTTTGAAGAAGCACGAGAAGTTTTTGAAAACACT
GAAAGAACAACTGAATTTTGGAAGCAGTATGTTGATGGAGATCAGTGTGAGTCC
AATCCATGTTTAAATGGCGGCAGTTGCAAGGATGACATTAATTCCTATGAATGTT
GGTGTCCCTTTGGATTTGAAGGAAAGAACTGTGAATTAGATGTAACATGTAACAT
TAAGAATGGCAGATGCGAGCAGTTTTGTAAAAATAGTGCTGATAACAAGGTGGT
 
TTGCTCCTGTACTGAGGGATATCGACTTGCAGAAAACCAGAAGTCCTGTGAACCA
GCAGTGCCATTTCCATGTGGAAGAGTTTCTGTTTCACAAACTTCTAAGCTCACCC
GTGCTGAGACTGTTTTTCCTGATGTGGACTATGTAAATTCTACTGAAGCTGAAAC
CATTTTGGATAACATCACTCAAAGCACCCAATCATTTAATGACTTCACTCGGGTT
GTTGGTGGAGAAGATGCCAAACCAGGTCAATTCCCTTGGCAGGTTGTTTTGAATG
GTAAAGTTGATGCATTCTGTGGAGGCTCTATCGTTAATGAAAAATGGATTGTAAC
TGCTGCCCACTGTGTTGAAACTGGTGTTAAAATTACAGTTGTCGCAGGTGAACAT
AATATTGAGGAGACAGAACATACAGAGCAAAAGCGAAATGTGATTCGAATTATT
CCTCACCACAACTACAATGCAGCTATTAATAAGTACAACCATGACATTGCCCTTC
TGGAACTGGACGAACCCTTAGTGCTAAACAGCTACGTTACACCTATTTGCATTGC
TGACAAGGAATACACGAACATCTTCCTCAAATTTGGATCTGGCTATGTAAGTGGC
TGGGGAAGAGTCTTCCACAAAGGGAGATCAGCTTTAGTTCTTCAGTACCTTAGAG
TTCCACTTGTTGACCGAGCCACATGTCTTCTATCTACAAAGTTCACCATCTATAAC
AACATGTTCTGTGCTGGCTTCCATGAAGGAGGTAGAGATTCATGTCAAGGAGATA
GTGGGGGACCCCATGTTACTGAAGTGGAAGGGACCAGTTTCTTAACTGGAATTAT
TAGCTGGGGTGAAGAGTGTGCAATGAAAGGCAAATATGGAATATATACCAAGGT
ATCCCGGTATGTCAACTGGATTAAGGAAAAAACAAAGCTCACTTAACCTCGACT
GTGCCTTCTAGTTGCCAGCCATCTGTTGTTTGCCCCTCCCCCGTGCCTTCCTTGAC
CCTGGAAGGTGCCACTCCCACTGTCCTTTCCTAATAAAATGAGGAAATTGCATCG
CATTGTCTGAGTAGGTGTCATTCTATTCTGGGGGGTGGGGTGGGGCAGGACAGCA
AGGGGGAGGATTGGGAAGACAATAGCAGGCATGCTGGGGATGCGGTGGGCTCT
ATGGCTTCTGAGGCGGAAAGAACCAGCTGGGGCTCTAGGGGGTATCCCCCTTAG
GTGGTTATATTATTGATATATTTTTGGTATCTTTGATGACAATAATGGGGGATTTT
GAAAGCTTAGCTTTAAATTTCTTTTAATTAAAAAAAAATGCTAGGCAGAATGACT
CAAATTACGTTGGATACAGTTGAATTTATTACGGTCTCATAGGGCCTGCCTGCTC
GACCATGCTATACTAAAAATTAAAAGTGTGTGTTACTAATTTTATAAATGGAGTT
TCCATTTATATTTACCTTTATTTCTTATTTACCATTGTCTTAGTAGATATTTACAAA
CATGACAGAAACACTAAATCTTGAGTTTGAATGCACAGATATAAACACTTAACG
GGTTTTAAAAATAATAATGTTGGTGAAAAAATATAACTTTGAGTGTAGCAGAGA
GGAACCATTGCCACCTTCAGATTTTCCTGTAACGATCGGGAACTGGCATCTTCAG
GGAGTAGCTTAGGTCAGTGAAGAGAAGAACAAAAAGCAGCATATTACAGTTAGT
TGTCTTCATCAATCTTTAAATATGTTGTGTGGTTTTTCTCTCCCTGTTTCCACAGAC
AAGAGTGAGATCGCCCATCGGTATAATGATTTGGGAGAACAACATTTCAAAGGC
CTGTAAGTTATAATGCTGAAAGCCCACTTAATATTTCTGGTAGTATTAGTTAAAG
TTTTAAAACACCTTTTTCCACCTTGAGTGTGAGAATTGTAGAGCAGTGCTGTCCA
GTAGAAATGTGTGCATTGACAGAAAGACTGTGGATCTGTGCTGAGCAATGTGGC
AGCCAGAGATCACAAGGCTATCAAGCACTTTGCACATGGCAAGTGTAACTGAGA
AGCACACATTCAAATAATAGTTAATTTTAATTGAATGTATCTAGCCATGTGTGGC
TAGTAGCTCCTTTCCTGGAGAGAGAATCTGGAGCCCACATCTAACTTGTTAAGTC
TGGAATCTTATTTTTTATTTCTGGAAAGGTCTATGAACTATAGTTTTGGGGGCAGC
TCACTTACTAACTTTTAATGCAATAAGATCCATGGTATCTTGAGAACATTATTTTG
TCTCTTTGTAGTACTGAAACCTTATACATGTGAAGTAAGGGGTCTATACTTAAGT
CACATCTCCAACCTTAGTAATGTTTTAATGTAGTAAAAAAATGAGTAATTAATTT
ATTTTTAGAAGGTCAATAGTATCATGTATTCCAAATAACAGAGGTATATGGTTAG
AAAAGAAACAATTCAAAGGACTTATATAATATCTAGCCTTGACAATGAATAAAT
TTAGAGAGTAGTTTGCCTGTTTGCCTCATGTTCATAAATCTATTGACACATATGTG
CATCTGCACTTCAGCATGGTAGAAGTCCATATTCCTTTGCTTGGAAAGGCAGGTG
TTCCCATTACGCCTCAGAGAATAGCTGACGGGAAGAGGCTTTCTAGATAGTTGTA
TGAAAGATATACAAAATCTCGCAGGTATACACAGGCATGATTTGCTGGTTGGGA
GAGCCACTTGCCTCATACTGAGGTTTTTGTGTCTGCTTTTCAGAGTCCTGATTGCC
 
TTTTCCCAGTATCTCCAGAAATGCTCATACGATGAGCATGCCAAATTAGTGCAGG
AAGTAACAGACTTTGCAAAGACGTGTGTTGCCGATGAGTCTGCCGCCAACTGTG
ACAAATCCCTTGTGAGTACCTTCTGATTTTGTGGATCTACTTTCCTGCTTTCTGGA
ACTCTGTTTCAAAGCCAATCATGACTCCATCACTTAAGGCCCCGGGAACACTGTG
GCAGAGGGCAGCAGAGAGATTGATAAAGCCAGGGTGATGGGAATTTTCTGTGGG
ACTCCATTTCATAGTAATTGCAGAAGCTACAATACACTCAAAAAGTCTCACCACA
TGACTGCCCAAATGGGAGCTTGACAGTGACAGTGACAGTAGATATGCCAAAGTG
GATGAGGGAAAGACCACAAGAGCTAAACCCTGTAAAAAGAACTGTAGGCAACT
AAGGAATGCAGAGAGAAAGATCTAGGAACCCCTAGTGATGGAGTTGGCCACTCC
CTCTCTGCGCGCTCGCTCGCTCACTGAGGCCGCCCGGGCAAAGCCCGGGCGTCGG
GCGACCTTTGGTCGCCCGGCCTCAGTGAGCGAGCGAGCGCGCAGAGAGGGAGTG
GCCAA
   P00362: The 300/1500bp HA F9 construct (for G551) (SEQ ID NO: 287) 
TTGGCCACTCCCTCTCTGCGCGCTCGCTCGCTCACTGAGGCCGGGCGACCAAAGG
TCGCCCGACGCCCGGGCTTTGCCCGGGCGGCCTCAGTGAGCGAGCGAGCGCGCA
GAGAGGGAGTGGCCAACTCCATCACTAGGGGTTCCTAGATCTAAGTATATTAGA
GCGAGTCTTTCTGCACACAGATCACCTTTCCTATCAACCCCACTAGCCTCTGGCA
AAATGAAGTGGGTAACCTTTCTCCTCCTCCTCTTCGTCTCCGGCTCTGCTTTTTCC
AGGGGTGTGTTTCGCCGAGAAGCACGTAAGAGTTTTATGTTTTTTCATCTCTGCTT
GTATTTTTCTAGTAATGGAAGCCTGGTATTTTAAAATAGTTAAATTTTCCTTTAGT
GCTGATTTCTAGATTATTATTACTGTTGTTGTTGTTATTATTGTCATTATTTGCATC
TGAGAACCTTTTTCTTGATCATGAAAACGCCAACAAAATTCTGAATCGGCCAAAG
AGGTATAATTCAGGTAAATTGGAAGAGTTTGTTCAAGGGAACCTTGAGAGAGAA
TGTATGGAAGAAAAGTGTAGTTTTGAAGAAGCACGAGAAGTTTTTGAAAACACT
GAAAGAACAACTGAATTTTGGAAGCAGTATGTTGATGGAGATCAGTGTGAGTCC
AATCCATGTTTAAATGGCGGCAGTTGCAAGGATGACATTAATTCCTATGAATGTT
GGTGTCCCTTTGGATTTGAAGGAAAGAACTGTGAATTAGATGTAACATGTAACAT
TAAGAATGGCAGATGCGAGCAGTTTTGTAAAAATAGTGCTGATAACAAGGTGGT
TTGCTCCTGTACTGAGGGATATCGACTTGCAGAAAACCAGAAGTCCTGTGAACCA
GCAGTGCCATTTCCATGTGGAAGAGTTTCTGTTTCACAAACTTCTAAGCTCACCC
GTGCTGAGACTGTTTTTCCTGATGTGGACTATGTAAATTCTACTGAAGCTGAAAC
CATTTTGGATAACATCACTCAAAGCACCCAATCATTTAATGACTTCACTCGGGTT
GTTGGTGGAGAAGATGCCAAACCAGGTCAATTCCCTTGGCAGGTTGTTTTGAATG
GTAAAGTTGATGCATTCTGTGGAGGCTCTATCGTTAATGAAAAATGGATTGTAAC
TGCTGCCCACTGTGTTGAAACTGGTGTTAAAATTACAGTTGTCGCAGGTGAACAT
AATATTGAGGAGACAGAACATACAGAGCAAAAGCGAAATGTGATTCGAATTATT
CCTCACCACAACTACAATGCAGCTATTAATAAGTACAACCATGACATTGCCCTTC
TGGAACTGGACGAACCCTTAGTGCTAAACAGCTACGTTACACCTATTTGCATTGC
TGACAAGGAATACACGAACATCTTCCTCAAATTTGGATCTGGCTATGTAAGTGGC
TGGGGAAGAGTCTTCCACAAAGGGAGATCAGCTTTAGTTCTTCAGTACCTTAGAG
TTCCACTTGTTGACCGAGCCACATGTCTTCTATCTACAAAGTTCACCATCTATAAC
AACATGTTCTGTGCTGGCTTCCATGAAGGAGGTAGAGATTCATGTCAAGGAGATA
GTGGGGGACCCCATGTTACTGAAGTGGAAGGGACCAGTTTCTTAACTGGAATTAT
TAGCTGGGGTGAAGAGTGTGCAATGAAAGGCAAATATGGAATATATACCAAGGT
ATCCCGGTATGTCAACTGGATTAAGGAAAAAACAAAGCTCACTTAACCTCGACT
GTGCCTTCTAGTTGCCAGCCATCTGTTGTTTGCCCCTCCCCCGTGCCTTCCTTGAC
CCTGGAAGGTGCCACTCCCACTGTCCTTTCCTAATAAAATGAGGAAATTGCATCG
CATTGTCTGAGTAGGTGTCATTCTATTCTGGGGGGTGGGGTGGGGCAGGACAGCA
AGGGGGAGGATTGGGAAGACAATAGCAGGCATGCTGGGGATGCGGTGGGCTCT
 
ATGGCTTCTGAGGCGGAAAGAACCAGCTGGGGCTCTAGGGGGTATCCCCCTTAG
GTGGTTATATTATTGATATATTTTTGGTATCTTTGATGACAATAATGGGGGATTTT
GAAAGCTTAGCTTTAAATTTCTTTTAATTAAAAAAAAATGCTAGGCAGAATGACT
CAAATTACGTTGGATACAGTTGAATTTATTACGGTCTCATAGGGCCTGCCTGCTC
GACCATGCTATACTAAAAATTAAAAGTGTGTGTTACTAATTTTATAAATGGAGTT
TCCATTTATATTTACCTTTATTTCTTATTTACCATTGTCTTAGTAGATATTTACAAA
CATGACAGAAACACTAAATCTTGAGTTTGAATGCACAGATATAAACACTTAACG
GGTTTTAAAAATAATAATGTTGGTGAAAAAATATAACTTTGAGTGTAGCAGAGA
GGAACCATTGCCACCTTCAGATTTTCCTGTAACGATCGGGAACTGGCATCTTCAG
GGAGTAGCTTAGGTCAGTGAAGAGAAGAACAAAAAGCAGCATATTACAGTTAGT
TGTCTTCATCAATCTTTAAATATGTTGTGTGGTTTTTCTCTCCCTGTTTCCACAGAC
AAGAGTGAGATCGCCCATCGGTATAATGATTTGGGAGAACAACATTTCAAAGGC
CTGTAAGTTATAATGCTGAAAGCCCACTTAATATTTCTGGTAGTATTAGTTAAAG
TTTTAAAACACCTTTTTCCACCTTGAGTGTGAGAATTGTAGAGCAGTGCTGTCCA
GTAGAAATGTGTGCATTGACAGAAAGACTGTGGATCTGTGCTGAGCAATGTGGC
AGCCAGAGATCACAAGGCTATCAAGCACTTTGCACATGGCAAGTGTAACTGAGA
AGCACACATTCAAATAATAGTTAATTTTAATTGAATGTATCTAGCCATGTGTGGC
TAGTAGCTCCTTTCCTGGAGAGAGAATCTGGAGCCCACATCTAACTTGTTAAGTC
TGGAATCTTATTTTTTATTTCTGGAAAGGTCTATGAACTATAGTTTTGGGGGCAGC
TCACTTACTAACTTTTAATGCAATAAGATCCATGGTATCTTGAGAACATTATTTTG
TCTCTTTGTAGTACTGAAACCTTATACATGTGAAGTAAGGGGTCTATACTTAAGT
CACATCTCCAACCTTAGTAATGTTTTAATGTAGTAAAAAAATGAGTAATTAATTT
ATTTTTAGAAGGTCAATAGTATCATGTATTCCAAATAACAGAGGTATATGGTTAG
AAAAGAAACAATTCAAAGGACTTATATAATATCTAGCCTTGACAATGAATAAAT
TTAGAGAGTAGTTTGCCTGTTTGCCTCATGTTCATAAATCTATTGACACATATGTG
CATCTGCACTTCAGCATGGTAGAAGTCCATATTCCTTTGCTTGGAAAGGCAGGTG
TTCCCATTACGCCTCAGAGAATAGCTGACGGGAAGAGGCTTTCTAGATAGTTGTA
TGAAAGATATACAAAATCTCGCAGGTATACACAGGCATGATTTGCTGGTTGGGA
GAGCCACTTAGATCTAGGAACCCCTAGTGATGGAGTTGGCCACTCCCTCTCTGCG
CGCTCGCTCGCTCACTGAGGCCGCCCGGGCAAAGCCCGGGCGTCGGGCGACCTT
TGGTCGCCCGGCCTCAGTGAGCGAGCGAGCGCGCAGAGAGGGAGTGGCCAA
   Cas9 ORF (SEQ ID NO: 703) 
ATGGATAAGAAGTACTCAATCGGGCTGGATATCGGAACTAATTCCGTGGGTTGG
GCAGTGATCACGGATGAATACAAAGTGCCGTCCAAGAAGTTCAAGGTCCTGGGG
AACACCGATAGACACAGCATCAAGAAAAATCTCATCGGAGCCCTGCTGTTTGAC
TCCGGCGAAACCGCAGAAGCGACCCGGCTCAAACGTACCGCGAGGCGACGCTAC
ACCCGGCGGAAGAATCGCATCTGCTATCTGCAAGAGATCTTTTCGAACGAAATG
GCAAAGGTCGACGACAGCTTCTTCCACCGCCTGGAAGAATCTTTCCTGGTGGAGG
AGGACAAGAAGCATGAACGGCATCCTATCTTTGGAAACATCGTCGACGAAGTGG
CGTACCACGAAAAGTACCCGACCATCTACCATCTGCGGAAGAAGTTGGTTGACT
CAACTGACAAGGCCGACCTCAGATTGATCTACTTGGCCCTCGCCCATATGATCAA
ATTCCGCGGACACTTCCTGATCGAAGGCGATCTGAACCCTGATAACTCCGACGTG
GATAAGCTTTTCATTCAACTGGTGCAGACCTACAACCAACTGTTCGAAGAAAACC
CAATCAATGCTAGCGGCGTCGATGCCAAGGCCATCCTGTCCGCCCGGCTGTCGAA
GTCGCGGCGCCTCGAAAACCTGATCGCACAGCTGCCGGGAGAGAAAAAGAACG
GACTTTTCGGCAACTTGATCGCTCTCTCACTGGGACTCACTCCCAATTTCAAGTCC
AATTTTGACCTGGCCGAGGACGCGAAGCTGCAACTCTCAAAGGACACCTACGAC
GACGACTTGGACAATTTGCTGGCACAAATTGGCGATCAGTACGCGGATCTGTTCC
 
TTGCCGCTAAGAACCTTTCGGACGCAATCTTGCTGTCCGATATCCTGCGCGTGAA
CACCGAAATAACCAAAGCGCCGCTTAGCGCCTCGATGATTAAGCGGTACGACGA
GCATCACCAGGATCTCACGCTGCTCAAAGCGCTCGTGAGACAGCAACTGCCTGA
AAAGTACAAGGAGATCTTCTTCGACCAGTCCAAGAATGGGTACGCAGGGTACAT
CGATGGAGGCGCTAGCCAGGAAGAGTTCTATAAGTTCATCAAGCCAATCCTGGA
AAAGATGGACGGAACCGAAGAACTGCTGGTCAAGCTGAACAGGGAGGATCTGCT
CCGGAAACAGAGAACCTTTGACAACGGATCCATTCCCCACCAGATCCATCTGGG
TGAGCTGCACGCCATCTTGCGGCGCCAGGAGGACTTTTACCCATTCCTCAAGGAC
AACCGGGAAAAGATCGAGAAAATTCTGACGTTCCGCATCCCGTATTACGTGGGC
CCACTGGCGCGCGGCAATTCGCGCTTCGCGTGGATGACTAGAAAATCAGAGGAA
ACCATCACTCCTTGGAATTTCGAGGAAGTTGTGGATAAGGGAGCTTCGGCACAA
AGCTTCATCGAACGAATGACCAACTTCGACAAGAATCTCCCAAACGAGAAGGTG
CTTCCTAAGCACAGCCTCCTTTACGAATACTTCACTGTCTACAACGAACTGACTA
AAGTGAAATACGTTACTGAAGGAATGAGGAAGCCGGCCTTTCTGTCCGGAGAAC
AGAAGAAAGCAATTGTCGATCTGCTGTTCAAGACCAACCGCAAGGTGACCGTCA
AGCAGCTTAAAGAGGACTACTTCAAGAAGATCGAGTGTTTCGACTCAGTGGAAA
TCAGCGGGGTGGAGGACAGATTCAACGCTTCGCTGGGAACCTATCATGATCTCCT
GAAGATCATCAAGGACAAGGACTTCCTTGACAACGAGGAGAACGAGGACATCCT
GGAAGATATCGTCCTGACCTTGACCCTTTTCGAGGATCGCGAGATGATCGAGGA
GAGGCTTAAGACCTACGCTCATCTCTTCGACGATAAGGTCATGAAACAACTCAA
GCGCCGCCGGTACACTGGTTGGGGCCGCCTCTCCCGCAAGCTGATCAACGGTATT
CGCGATAAACAGAGCGGTAAAACTATCCTGGATTTCCTCAAATCGGATGGCTTCG
CTAATCGTAACTTCATGCAATTGATCCACGACGACAGCCTGACCTTTAAGGAGGA
CATCCAAAAAGCACAAGTGTCCGGACAGGGAGACTCACTCCATGAACACATCGC
GAATCTGGCCGGTTCGCCGGCGATTAAGAAGGGAATTCTGCAAACTGTGAAGGT
GGTCGACGAGCTGGTGAAGGTCATGGGACGGCACAAACCGGAGAATATCGTGAT
TGAAATGGCCCGAGAAAACCAGACTACCCAGAAGGGCCAGAAAAACTCCCGCG
AAAGGATGAAGCGGATCGAAGAAGGAATCAAGGAGCTGGGCAGCCAGATCCTG
AAAGAGCACCCGGTGGAAAACACGCAGCTGCAGAACGAGAAGCTCTACCTGTAC
TATTTGCAAAATGGACGGGACATGTACGTGGACCAAGAGCTGGACATCAATCGG
TTGTCTGATTACGACGTGGACCACATCGTTCCACAGTCCTTTCTGAAGGATGACT
CGATCGATAACAAGGTGTTGACTCGCAGCGACAAGAACAGAGGGAAGTCAGATA
ATGTGCCATCGGAGGAGGTCGTGAAGAAGATGAAGAATTACTGGCGGCAGCTCC
TGAATGCGAAGCTGATTACCCAGAGAAAGTTTGACAATCTCACTAAAGCCGAGC
GCGGCGGACTCTCAGAGCTGGATAAGGCTGGATTCATCAAACGGCAGCTGGTCG
AGACTCGGCAGATTACCAAGCACGTGGCGCAGATCTTGGACTCCCGCATGAACA
CTAAATACGACGAGAACGATAAGCTCATCCGGGAAGTGAAGGTGATTACCCTGA
AAAGCAAACTTGTGTCGGACTTTCGGAAGGACTTTCAGTTTTACAAAGTGAGAGA
AATCAACAACTACCATCACGCGCATGACGCATACCTCAACGCTGTGGTCGGTACC
GCCCTGATCAAAAAGTACCCTAAACTTGAATCGGAGTTTGTGTACGGAGACTAC
AAGGTCTACGACGTGAGGAAGATGATAGCCAAGTCCGAACAGGAAATCGGGAA
AGCAACTGCGAAATACTTCTTTTACTCAAACATCATGAACTTTTTCAAGACTGAA
ATTACGCTGGCCAATGGAGAAATCAGGAAGAGGCCACTGATCGAAACTAACGGA
GAAACGGGCGAAATCGTGTGGGACAAGGGCAGGGACTTCGCAACTGTTCGCAAA
GTGCTCTCTATGCCGCAAGTCAATATTGTGAAGAAAACCGAAGTGCAAACCGGC
GGATTTTCAAAGGAATCGATCCTCCCAAAGAGAAATAGCGACAAGCTCATTGCA
CGCAAGAAAGACTGGGACCCGAAGAAGTACGGAGGATTCGATTCGCCGACTGTC
GCATACTCCGTCCTCGTGGTGGCCAAGGTGGAGAAGGGAAAGAGCAAAAAGCTC
AAATCCGTCAAAGAGCTGCTGGGGATTACCATCATGGAACGATCCTCGTTCGAG
AAGAACCCGATTGATTTCCTCGAGGCGAAGGGTTACAAGGAGGTGAAGAAGGAT
 
CTGATCATCAAACTCCCCAAGTACTCACTGTTCGAACTGGAAAATGGTCGGAAGC
GCATGCTGGCTTCGGCCGGAGAACTCCAAAAAGGAAATGAGCTGGCCTTGCCTA
GCAAGTACGTCAACTTCCTCTATCTTGCTTCGCACTACGAAAAACTCAAAGGGTC
ACCGGAAGATAACGAACAGAAGCAGCTTTTCGTGGAGCAGCACAAGCATTATCT
GGATGAAATCATCGAACAAATCTCCGAGTTTTCAAAGCGCGTGATCCTCGCCGAC
GCCAACCTCGACAAAGTCCTGTCGGCCTACAATAAGCATAGAGATAAGCCGATC
AGAGAACAGGCCGAGAACATTATCCACTTGTTCACCCTGACTAACCTGGGAGCC
CCAGCCGCCTTCAAGTACTTCGATACTACTATCGATCGCAAAAGATACACGTCCA
CCAAGGAAGTTCTGGACGCGACCCTGATCCACCAAAGCATCACTGGACTCTACG
AAACTAGGATCGATCTGTCGCAGCTGGGTGGCGAT
   U-dep Cas9 ORF (SEQ ID NO: 704) 
ATGGACAAGAAGTACAGCATCGGACTGGACATCGGAACAAACAGCGTCGGATG
GGCAGTCATCACAGACGAATACAAGGTCCCGAGCAAGAAGTTCAAGGTCCTGGG
AAACACAGACAGACACAGCATCAAGAAGAACCTGATCGGAGCACTGCTGTTCGA
CAGCGGAGAAACAGCAGAAGCAACAAGACTGAAGAGAACAGCAAGAAGAAGAT
ACACAAGAAGAAAGAACAGAATCTGCTACCTGCAGGAAATCTTCAGCAACGAAA
TGGCAAAGGTCGACGACAGCTTCTTCCACAGACTGGAAGAAAGCTTCCTGGTCG
AAGAAGACAAGAAGCACGAAAGACACCCGATCTTCGGAAACATCGTCGACGAA
GTCGCATACCACGAAAAGTACCCGACAATCTACCACCTGAGAAAGAAGCTGGTC
GACAGCACAGACAAGGCAGACCTGAGACTGATCTACCTGGCACTGGCACACATG
ATCAAGTTCAGAGGACACTTCCTGATCGAAGGAGACCTGAACCCGGACAACAGC
GACGTCGACAAGCTGTTCATCCAGCTGGTCCAGACATACAACCAGCTGTTCGAA
GAAAACCCGATCAACGCAAGCGGAGTCGACGCAAAGGCAATCCTGAGCGCAAG
ACTGAGCAAGAGCAGAAGACTGGAAAACCTGATCGCACAGCTGCCGGGAGAAA
AGAAGAACGGACTGTTCGGAAACCTGATCGCACTGAGCCTGGGACTGACACCGA
ACTTCAAGAGCAACTTCGACCTGGCAGAAGACGCAAAGCTGCAGCTGAGCAAGG
ACACATACGACGACGACCTGGACAACCTGCTGGCACAGATCGGAGACCAGTACG
CAGACCTGTTCCTGGCAGCAAAGAACCTGAGCGACGCAATCCTGCTGAGCGACA
TCCTGAGAGTCAACACAGAAATCACAAAGGCACCGCTGAGCGCAAGCATGATCA
AGAGATACGACGAACACCACCAGGACCTGACACTGCTGAAGGCACTGGTCAGAC
AGCAGCTGCCGGAAAAGTACAAGGAAATCTTCTTCGACCAGAGCAAGAACGGAT
ACGCAGGATACATCGACGGAGGAGCAAGCCAGGAAGAATTCTACAAGTTCATCA
AGCCGATCCTGGAAAAGATGGACGGAACAGAAGAACTGCTGGTCAAGCTGAAC
AGAGAAGACCTGCTGAGAAAGCAGAGAACATTCGACAACGGAAGCATCCCGCA
CCAGATCCACCTGGGAGAACTGCACGCAATCCTGAGAAGACAGGAAGACTTCTA
CCCGTTCCTGAAGGACAACAGAGAAAAGATCGAAAAGATCCTGACATTCAGAAT
CCCGTACTACGTCGGACCGCTGGCAAGAGGAAACAGCAGATTCGCATGGATGAC
AAGAAAGAGCGAAGAAACAATCACACCGTGGAACTTCGAAGAAGTCGTCGACA
AGGGAGCAAGCGCACAGAGCTTCATCGAAAGAATGACAAACTTCGACAAGAAC
CTGCCGAACGAAAAGGTCCTGCCGAAGCACAGCCTGCTGTACGAATACTTCACA
GTCTACAACGAACTGACAAAGGTCAAGTACGTCACAGAAGGAATGAGAAAGCC
GGCATTCCTGAGCGGAGAACAGAAGAAGGCAATCGTCGACCTGCTGTTCAAGAC
AAACAGAAAGGTCACAGTCAAGCAGCTGAAGGAAGACTACTTCAAGAAGATCG
AATGCTTCGACAGCGTCGAAATCAGCGGAGTCGAAGACAGATTCAACGCAAGCC
TGGGAACATACCACGACCTGCTGAAGATCATCAAGGACAAGGACTTCCTGGACA
ACGAAGAAAACGAAGACATCCTGGAAGACATCGTCCTGACACTGACACTGTTCG
AAGACAGAGAAATGATCGAAGAAAGACTGAAGACATACGCACACCTGTTCGAC
GACAAGGTCATGAAGCAGCTGAAGAGAAGAAGATACACAGGATGGGGAAGACT
GAGCAGAAAGCTGATCAACGGAATCAGAGACAAGCAGAGCGGAAAGACAATCC
 
TGGACTTCCTGAAGAGCGACGGATTCGCAAACAGAAACTTCATGCAGCTGATCC
ACGACGACAGCCTGACATTCAAGGAAGACATCCAGAAGGCACAGGTCAGCGGA
CAGGGAGACAGCCTGCACGAACACATCGCAAACCTGGCAGGAAGCCCGGCAATC
AAGAAGGGAATCCTGCAGACAGTCAAGGTCGTCGACGAACTGGTCAAGGTCATG
GGAAGACACAAGCCGGAAAACATCGTCATCGAAATGGCAAGAGAAAACCAGAC
AACACAGAAGGGACAGAAGAACAGCAGAGAAAGAATGAAGAGAATCGAAGAA
GGAATCAAGGAACTGGGAAGCCAGATCCTGAAGGAACACCCGGTCGAAAACAC
ACAGCTGCAGAACGAAAAGCTGTACCTGTACTACCTGCAGAACGGAAGAGACAT
GTACGTCGACCAGGAACTGGACATCAACAGACTGAGCGACTACGACGTCGACCA
CATCGTCCCGCAGAGCTTCCTGAAGGACGACAGCATCGACAACAAGGTCCTGAC
AAGAAGCGACAAGAACAGAGGAAAGAGCGACAACGTCCCGAGCGAAGAAGTCG
TCAAGAAGATGAAGAACTACTGGAGACAGCTGCTGAACGCAAAGCTGATCACAC
AGAGAAAGTTCGACAACCTGACAAAGGCAGAGAGAGGAGGACTGAGCGAACTG
GACAAGGCAGGATTCATCAAGAGACAGCTGGTCGAAACAAGACAGATCACAAA
GCACGTCGCACAGATCCTGGACAGCAGAATGAACACAAAGTACGACGAAAACG
ACAAGCTGATCAGAGAAGTCAAGGTCATCACACTGAAGAGCAAGCTGGTCAGCG
ACTTCAGAAAGGACTTCCAGTTCTACAAGGTCAGAGAAATCAACAACTACCACC
ACGCACACGACGCATACCTGAACGCAGTCGTCGGAACAGCACTGATCAAGAAGT
ACCCGAAGCTGGAAAGCGAATTCGTCTACGGAGACTACAAGGTCTACGACGTCA
GAAAGATGATCGCAAAGAGCGAACAGGAAATCGGAAAGGCAACAGCAAAGTAC
TTCTTCTACAGCAACATCATGAACTTCTTCAAGACAGAAATCACACTGGCAAACG
GAGAAATCAGAAAGAGACCGCTGATCGAAACAAACGGAGAAACAGGAGAAATC
GTCTGGGACAAGGGAAGAGACTTCGCAACAGTCAGAAAGGTCCTGAGCATGCCG
CAGGTCAACATCGTCAAGAAGACAGAAGTCCAGACAGGAGGATTCAGCAAGGA
AAGCATCCTGCCGAAGAGAAACAGCGACAAGCTGATCGCAAGAAAGAAGGACT
GGGACCCGAAGAAGTACGGAGGATTCGACAGCCCGACAGTCGCATACAGCGTCC
TGGTCGTCGCAAAGGTCGAAAAGGGAAAGAGCAAGAAGCTGAAGAGCGTCAAG
GAACTGCTGGGAATCACAATCATGGAAAGAAGCAGCTTCGAAAAGAACCCGATC
GACTTCCTGGAAGCAAAGGGATACAAGGAAGTCAAGAAGGACCTGATCATCAAG
CTGCCGAAGTACAGCCTGTTCGAACTGGAAAACGGAAGAAAGAGAATGCTGGCA
AGCGCAGGAGAACTGCAGAAGGGAAACGAACTGGCACTGCCGAGCAAGTACGT
CAACTTCCTGTACCTGGCAAGCCACTACGAAAAGCTGAAGGGAAGCCCGGAAGA
CAACGAACAGAAGCAGCTGTTCGTCGAACAGCACAAGCACTACCTGGACGAAAT
CATCGAACAGATCAGCGAATTCAGCAAGAGAGTCATCCTGGCAGACGCAAACCT
GGACAAGGTCCTGAGCGCATACAACAAGCACAGAGACAAGCCGATCAGAGAAC
AGGCAGAAAACATCATCCACCTGTTCACACTGACAAACCTGGGAGCACCGGCAG
CATTCAAGTACTTCGACACAACAATCGACAGAAAGAGATACACAAGCACAAAGG
AAGTCCTGGACGCAACACTGATCCACCAGAGCATCACAGGACTGTACGAAACAA
GAATCGACCTGAGCCAGCTGGGAGGAGACGGAGGAGGAAGCCCGAAGAAGAAG
AGAAAGGTCTAG
   mRNA comprising U dep Cas9 (SEQ ID NO: 705)

GGGUCCCGCAGUCGGCGUCCAGCGGCUCUGCUUGUUCGUGUGUGUGUCGUUGC
AGGCCUUAUUCGGAUCCGCCACCAUGGACAAGAAGUACAGCAUCGGACUGGAC
AUCGGAACAAACAGCGUCGGAUGGGCAGUCAUCACAGACGAAUACAAGGUCCC
GAGCAAGAAGUUCAAGGUCCUGGGAAACACAGACAGACACAGCAUCAAGAAG
AACCUGAUCGGAGCACUGCUGUUCGACAGCGGAGAAACAGCAGAAGCAACAAG
ACUGAAGAGAACAGCAAGAAGAAGAUACACAAGAAGAAAGAACAGAAUCUGC
UACCUGCAGGAAAUCUUCAGCAACGAAAUGGCAAAGGUCGACGACAGCUUCUU
CCACAGACUGGAAGAAAGCUUCCUGGUCGAAGAAGACAAGAAGCACGAAAGA

CACCCGAUCUUCGGAAACAUCGUCGACGAAGUCGCAUACCACGAAAAGUACCC
GACAAUCUACCACCUGAGAAAGAAGCUGGUCGACAGCACAGACAAGGCAGACC
UGAGACUGAUCUACCUGGCACUGGCACACAUGAUCAAGUUCAGAGGACACUUC
CUGAUCGAAGGAGACCUGAACCCGGACAACAGCGACGUCGACAAGCUGUUCAU
CCAGCUGGUCCAGACAUACAACCAGCUGUUCGAAGAAAACCCGAUCAACGCAA
GCGGAGUCGACGCAAAGGCAAUCCUGAGCGCAAGACUGAGCAAGAGCAGAAG
ACUGGAAAACCUGAUCGCACAGCUGCCGGGAGAAAAGAAGAACGGACUGUUC
GGAAACCUGAUCGCACUGAGCCUGGGACUGACACCGAACUUCAAGAGCAACUU
CGACCUGGCAGAAGACGCAAAGCUGCAGCUGAGCAAGGACACAUACGACGACG
ACCUGGACAACCUGCUGGCACAGAUCGGAGACCAGUACGCAGACCUGUUCCUG
GCAGCAAAGAACCUGAGCGACGCAAUCCUGCUGAGCGACAUCCUGAGAGUCAA
CACAGAAAUCACAAAGGCACCGCUGAGCGCAAGCAUGAUCAAGAGAUACGACG
AACACCACCAGGACCUGACACUGCUGAAGGCACUGGUCAGACAGCAGCUGCCG
GAAAAGUACAAGGAAAUCUUCUUCGACCAGAGCAAGAACGGAUACGCAGGAU
ACAUCGACGGAGGAGCAAGCCAGGAAGAAUUCUACAAGUUCAUCAAGCCGAUC
CUGGAAAAGAUGGACGGAACAGAAGAACUGCUGGUCAAGCUGAACAGAGAAG
ACCUGCUGAGAAAGCAGAGAACAUUCGACAACGGAAGCAUCCCGCACCAGAUC
CACCUGGGAGAACUGCACGCAAUCCUGAGAAGACAGGAAGACUUCUACCCGUU
CCUGAAGGACAACAGAGAAAAGAUCGAAAAGAUCCUGACAUUCAGAAUCCCG
UACUACGUCGGACCGCUGGCAAGAGGAAACAGCAGAUUCGCAUGGAUGACAA
GAAAGAGCGAAGAAACAAUCACACCGUGGAACUUCGAAGAAGUCGUCGACAA
GGGAGCAAGCGCACAGAGCUUCAUCGAAAGAAUGACAAACUUCGACAAGAACC
UGCCGAACGAAAAGGUCCUGCCGAAGCACAGCCUGCUGUACGAAUACUUCACA
GUCUACAACGAACUGACAAAGGUCAAGUACGUCACAGAAGGAAUGAGAAAGC
CGGCAUUCCUGAGCGGAGAACAGAAGAAGGCAAUCGUCGACCUGCUGUUCAAG
ACAAACAGAAAGGUCACAGUCAAGCAGCUGAAGGAAGACUACUUCAAGAAGA
UCGAAUGCUUCGACAGCGUCGAAAUCAGCGGAGUCGAAGACAGAUUCAACGCA
AGCCUGGGAACAUACCACGACCUGCUGAAGAUCAUCAAGGACAAGGACUUCCU
GGACAACGAAGAAAACGAAGACAUCCUGGAAGACAUCGUCCUGACACUGACAC
UGUUCGAAGACAGAGAAAUGAUCGAAGAAAGACUGAAGACAUACGCACACCU
GUUCGACGACAAGGUCAUGAAGCAGCUGAAGAGAAGAAGAUACACAGGAUGG
GGAAGACUGAGCAGAAAGCUGAUCAACGGAAUCAGAGACAAGCAGAGCGGAA
AGACAAUCCUGGACUUCCUGAAGAGCGACGGAUUCGCAAACAGAAACUUCAUG
CAGCUGAUCCACGACGACAGCCUGACAUUCAAGGAAGACAUCCAGAAGGCACA
GGUCAGCGGACAGGGAGACAGCCUGCACGAACACAUCGCAAACCUGGCAGGAA
GCCCGGCAAUCAAGAAGGGAAUCCUGCAGACAGUCAAGGUCGUCGACGAACUG
GUCAAGGUCAUGGGAAGACACAAGCCGGAAAACAUCGUCAUCGAAAUGGCAA
GAGAAAACCAGACAACACAGAAGGGACAGAAGAACAGCAGAGAAAGAAUGAA
GAGAAUCGAAGAAGGAAUCAAGGAACUGGGAAGCCAGAUCCUGAAGGAACAC
CCGGUCGAAAACACACAGCUGCAGAACGAAAAGCUGUACCUGUACUACCUGCA
GAACGGAAGAGACAUGUACGUCGACCAGGAACUGGACAUCAACAGACUGAGC
GACUACGACGUCGACCACAUCGUCCCGCAGAGCUUCCUGAAGGACGACAGCAU
CGACAACAAGGUCCUGACAAGAAGCGACAAGAACAGAGGAAAGAGCGACAAC
GUCCCGAGCGAAGAAGUCGUCAAGAAGAUGAAGAACUACUGGAGACAGCUGC
UGAACGCAAAGCUGAUCACACAGAGAAAGUUCGACAACCUGACAAAGGCAGA
GAGAGGAGGACUGAGCGAACUGGACAAGGCAGGAUUCAUCAAGAGACAGCUG
GUCGAAACAAGACAGAUCACAAAGCACGUCGCACAGAUCCUGGACAGCAGAAU
GAACACAAAGUACGACGAAAACGACAAGCUGAUCAGAGAAGUCAAGGUCAUC
ACACUGAAGAGCAAGCUGGUCAGCGACUUCAGAAAGGACUUCCAGUUCUACAA
GGUCAGAGAAAUCAACAACUACCACCACGCACACGACGCAUACCUGAACGCAG

UCGUCGGAACAGCACUGAUCAAGAAGUACCCGAAGCUGGAAAGCGAAUUCGUC
UACGGAGACUACAAGGUCUACGACGUCAGAAAGAUGAUCGCAAAGAGCGAAC
AGGAAAUCGGAAAGGCAACAGCAAAGUACUUCUUCUACAGCAACAUCAUGAA
CUUCUUCAAGACAGAAAUCACACUGGCAAACGGAGAAAUCAGAAAGAGACCGC
UGAUCGAAACAAACGGAGAAACAGGAGAAAUCGUCUGGGACAAGGGAAGAGA
CUUCGCAACAGUCAGAAAGGUCCUGAGCAUGCCGCAGGUCAACAUCGUCAAGA
AGACAGAAGUCCAGACAGGAGGAUUCAGCAAGGAAAGCAUCCUGCCGAAGAG
AAACAGCGACAAGCUGAUCGCAAGAAAGAAGGACUGGGACCCGAAGAAGUAC
GGAGGAUUCGACAGCCCGACAGUCGCAUACAGCGUCCUGGUCGUCGCAAAGGU
CGAAAAGGGAAAGAGCAAGAAGCUGAAGAGCGUCAAGGAACUGCUGGGAAUC
ACAAUCAUGGAAAGAAGCAGCUUCGAAAAGAACCCGAUCGACUUCCUGGAAGC
AAAGGGAUACAAGGAAGUCAAGAAGGACCUGAUCAUCAAGCUGCCGAAGUAC
AGCCUGUUCGAACUGGAAAACGGAAGAAAGAGAAUGCUGGCAAGCGCAGGAG
AACUGCAGAAGGGAAACGAACUGGCACUGCCGAGCAAGUACGUCAACUUCCUG
UACCUGGCAAGCCACUACGAAAAGCUGAAGGGAAGCCCGGAAGACAACGAACA
GAAGCAGCUGUUCGUCGAACAGCACAAGCACUACCUGGACGAAAUCAUCGAAC
AGAUCAGCGAAUUCAGCAAGAGAGUCAUCCUGGCAGACGCAAACCUGGACAAG
GUCCUGAGCGCAUACAACAAGCACAGAGACAAGCCGAUCAGAGAACAGGCAGA
AAACAUCAUCCACCUGUUCACACUGACAAACCUGGGAGCACCGGCAGCAUUCA
AGUACUUCGACACAACAAUCGACAGAAAGAGAUACACAAGCACAAAGGAAGU
CCUGGACGCAACACUGAUCCACCAGAGCAUCACAGGACUGUACGAAACAAGAA
UCGACCUGAGCCAGCUGGGAGGAGACGGAGGAGGAAGCCCGAAGAAGAAGAG
AAAGGUCUAGCUAGCCAUCACAUUUAAAAGCAUCUCAGCCUACCAUGAGAAUA
AGAGAAAGAAAAUGAAGAUCAAUAGCUUAUUCAUCUCUUUUUCUUUUUCGUU
GGUGUAAAGCCAACACCCUGUCUAAAAAACAUAAAUUUCUUUAAUCAUUUUG
CCUCUUUUCUCUGUGCUUCAAUUAAUAAAAAAUGGAAAGAACCUCGAGAAAA
AAAAAAAAAAAAAAAAAAAAAAAAAAAAAAAAAAAAAAAAAAAAAAAAAAAAA
AAAAAAAAAAAAAAAAAAAAAAAAAAAAAAAAAAAA

## Claims

1. A bidirectional nucleic acid construct comprising:
a) a first segment comprising a first coding sequence for a polypeptide; and
b) a second segment comprising a reverse complement of a second coding sequence for the polypeptide, wherein the second coding sequence adopts a different codon usage from that of the first coding sequence,
wherein the bidirectional nucleic acid construct does not comprise a promoter that drives the expression of the first or second coding sequence; wherein the construct does not comprise a homology arm, and wherein the construct does not comprise a first and second rare-cutting endonuclease target site.

2. The bidirectional nucleic acid construct of claim 1, wherein the first coding sequence and the second coding sequence each encode a therapeutic protein.

3. A bidirectional nucleic acid construct comprising:
a) a first segment comprising a first sequence encoding a therapeutic protein; and
b) a second segment comprising a reverse complement of a second sequence encoding the therapeutic protein, wherein the second coding sequence adopts a different codon usage from that of the first coding sequence,
wherein the bidirectional nucleic acid construct does not comprise a promoter that drives the expression of the first or second coding sequence; wherein the construct does not comprise a homology arm.

4. The bidirectional nucleic acid construct of any one of claims 1-3, wherein the first segment is linked to the second segment by a linker; optionally wherein the linker is 5, 10, 20, 30, 40, 50, 60, 70, 80, 90, 100, 150, 200, 250, 300, 500, 1000, 1500, 2000 nucleotides in length.

5. The bidirectional nucleic acid construct of any one of claims 1-4, wherein each of the first segment and second segment comprises a polyadenylation sequence, such as a polyadenylation signal sequence or a polyadenylation tail sequence.

6. The bidirectional nucleic acid construct of any of one claims 1-5, wherein the construct comprises a splice acceptor site; optionally wherein the construct comprises a first splice acceptor site 5' of the first segment and a second splice acceptor site 3' of the second segment.

7. The bidirectional nucleic acid construct of any one of claims 1-6, wherein the construct comprises one, two, or three inverted terminal repeats (ITR); optionally wherein the construct comprises no more than two ITRs.

8. The bidirectional nucleic acid construct of any one of claims 1-7, wherein the polypeptide or therapeutic protein, when expressed, comprises a heterologous signal peptide.

9. A vector comprising the construct of any one of claims 1-8; optionally wherein the vector is a viral vector; optionally wherein the viral vector is an adeno-associated virus (AAV) vector; optionally wherein the AAV comprises a single-stranded genome (ssAAV) or a self-complementary genome (scAAV) and/or wherein the AAV vector is selected from the group consisting of AAV1, AAV2, AAV3, AAV3B, AAV4, AAV5, AAV6, AAV6.2, AAV7, AAVrh.64R1, AAVhu.37, AAVrh.8, AAVrh.32.33, AAV8, AAV9, AAV-DJ, AAV2/8, AAVrh10, AAVLK03_{,} AV10, AAV11, AAV12, rh10, and hybrids thereof.

10. A lipid nanoparticle comprising the construct of any one of claims 1-8.

11. A host cell comprising the construct of any one of claims 1-8, optionally wherein the host cell is a liver cell, a muscle cell or a neuronal cell; optionally wherein the host cell is a hepatocyte.

12. An *in vitro* method of modifying a target locus comprising providing a cell with a construct according to any one of claims 1-8, a vector according to claim 9, or an LNP of claim 10.

13. A construct according to any one of claims 1-8, a vector according to claim 9, or an LNP according to claim 10 for use in a method of expressing the polypeptide or therapeutic protein in a subject, the method comprising administering to the subject the construct, vector, or LNP such that a target locus in a cell in the subject is modified and the cell expresses the polypeptide or therapeutic protein from the target locus.

14. The method according to claim 12 or the construct, vector or LNP for the use according to claim 13, wherein the cell is a liver cell, a neuronal cell, or a muscle cell.

15. The method according to claim 12 or the construct, vector or LNP for the use according to claim 13, the method further comprising providing the cell with an RNA-guided DNA-binding agent and a guide RNA (gRNA); optionally wherein:
the gRNA is a single gRNA (sgRNA);
the construct, RNA-guided DNA-binding agent, and the gRNA are provided to the cell simultaneously or sequentially in any order; and/or
the RNA-guided DNA-binding agent is provided as a Cas nuclease or an mRNA encoding a Cas nuclease; optionally wherein the Cas nuclease is a Cas9 nuclease; optionally wherein the Cas9 nuclease is a *S. pyogenes* Cas9, or a variant thereof.
